(19) Europäisches Patentamt
European Patent Office
Office européen des brevets

(11) Numéro de publication : **0 428 437 B1**

(12) # FASCICULE DE BREVET EUROPEEN

(45) Date de publication du fascicule du brevet : **17.08.94 Bulletin 94/33**

(51) Int. Cl.[5] : **C07D 413/04,** A61K 31/435

(21) Numéro de dépôt : **90403145.7**

(22) Date de dépôt : **07.11.90**

(54) **Dérivés du 1,2-benzisoxazole, leurs procédés de préparation et les compositions pharmaceutiques qui les contiennent.**

(30) Priorité : **07.11.89 FR 8914571**

(43) Date de publication de la demande : **22.05.91 Bulletin 91/21**

(45) Mention de la délivrance du brevet : **17.08.94 Bulletin 94/33**

(84) Etats contractants désignés : **AT BE CH DE DK ES FR GB GR IT LI LU NL SE**

(56) Documents cités :
**EP-A- 0 080 104**
**EP-A- 0 196 132**
**US-A- 4 352 811**
**JOURNAL OF MEDICINAL CHEMISTRY, vol. 28, no. 6, juin 1985, pages 761-769,American Chemical Society, Washington, DC, US; J.T. STRUPCZEWSKI et al.:"Synthesis and neuroleptic activity of 3-(1-subsituted-4-piperidinyl)-1,2-benzisoxazoles"**

(73) Titulaire : **ADIR ET COMPAGNIE**
**1 rue Carle Hébert**
**F-92415 Courbevoie Cédex (FR)**

(72) Inventeur : **Peglion, Jean Louis**
**5 allée des Bégonias**
**F-78110 Le Vesinet (FR)**
Inventeur : **Colpaert, Francis**
**36bis boulevard Carnot**
**F-78110 Le Vesinet (FR)**

(74) Mandataire : **Reverbori, Marcelle**
**Adir et Compagnie,**
**1, rue Carle Hébert**
**F-92415 Courbevoie Cédex (FR)**

EP 0 428 437 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés du 1,2-benzisoxazole, leurs procédés de préparation et les compositions pharmaceutiques qui les contiennent.

De nombreux dérivés du 3-pipéridyl 1,2-benzisoxazole sont connus dans la littérature. Les demandes EP 196132, EP 314098 ainsi que le brevet US 4352811 décrivent des dérivés substitués en position 1 du noyau pipéridine avec des hétérocycles comportant 2 ou 3 hétéroatomes ou avec des dérivés du noyau phényle. Ces composés sont doués de propriétés antipsychotiques. Des dérivés du 3-pipéridyl 1,2-benzisoxazole ayant des propriétés analgésiques ou neuroleptiques sont aussi connus (Brevets US 4469869 et US 4355037 ; Demande de Brevet EP 080104 ; J. Med. Chem (1985) 28 p.761-769).

Les composés de la présente invention sont des dérivés du 3-pipéridyl et du 3-pyrrolidinyl 1,2-benzisoxazole et se distinguent des composés déjà connus par leurs substituants en position 1 du noyau pipéridine et pyrrolidine et par leurs propriétés pharmacologiques. En effet, les différents essais pharmacologiques ont démontré que les dérivés de l'invention sont des antagonistes de la dopamine et de la sérotonine et possèdent une activité antipsychotique comparable à celle de l'halopéridol, substance de référence pour l'évaluation des antipsychotiques mais, aux doses actives, n'induisent pas d'effets secondaires et notamment d'effets extrapyramidaux. Or, il est connu que les antipsychotiques peuvent provoquer des effets secondaires très importants et ce facteur peut limiter leur utilisation. Les composés de l'invention constituent donc une nouvelle classe d'antipsychotiques et trouvent leur application comme agents sédatifs, anxiolytiques, antiagressifs et analgésiques. Ils sont également utiles pour le traitement de la schizophrénie, et celui de la dépression.

La présente invention a plus particulièrement pour objet les dérivés du 1,2-benzisoxazole, de formule générale I :

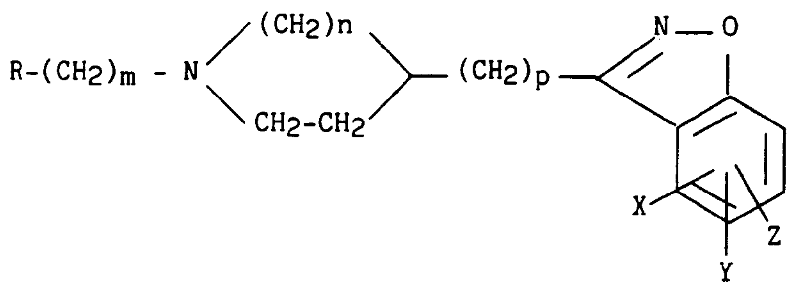

(I)

dans laquelle

- m représente un nombre entier de 0 à 5,
- n représente un nombre entier de 1 à 2,
- p est égal à 0, 1, ou 2,
- X, Y et Z identiques ou différents représentent chacun un atome d'hydrogène, un atome d'halogène, un radical alkyle linéaire ou ramifié de 1 à 6 atomes de carbone, un radical trifluorométhyle, un radical alcoxy de 1 à 6 atomes de carbone, un radical alkylthio de 1 à 6 atomes de carbone ou un radical hydroxy,
- R représente un radical benzofuran-2-yle ou 2,3-dihydro benzofuran-2-yle (chacun pouvant être substitué sur le cycle benzénique par un ou plusieurs atomes d'halogène, par des radicaux alcoxy de 1 à 6 atomes de carbone, par des radicaux alkylthio de 1 à 6 atomes de carbone ou par des radicaux alkyle linéaires ou ramifiés de 1 à 6 atomes de carbone), un radical 2,3,6,7-tétrahydro benzo[1,2,-b:4,5-b']difuran-2-yl, un radical 4-oxo 4H-chromèn-2-yl (éventuellement substitué sur le cycle benzénique par un ou plusieurs atomes d'halogène, par des radicaux alkyle linéaires ou ramifiés de 1 à 6 atomes de carbone ou par des radicaux alcoxy de 1 à 6 atomes de carbone), un radical benzocyclobutényle de formule A ou un radical indanyle de formule B :

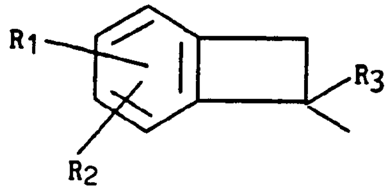

(A)

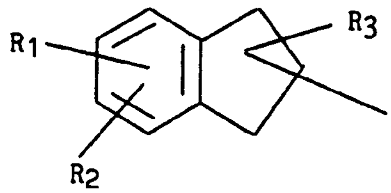

(B)

dans lesquelles

$R_1$ et $R_2$ identiques ou différents représentent chacun un atome d'hydrogène, un atome d'halogène, un radical trifluorométhyle, un radical alkyle linéaire ou ramifié de 1 à 6 atomes de carbone, un radical alcoxy de 1 à 6 atomes de carbone, un radical hydroxy, un radical hydroxyalkyle de 1 à 6 atomes de carbone, un radical alkylthio de 1 à 6 atomes de carbone, ou forment ensemble un radical méthylènedioxy ou un radical éthylènedioxy,

et $R_3$ représente un atome d'hydrogène ou un radical alkyle linéaire ou ramifié de 1 à 6 atomes de carbone,

ou un radical de formule C :

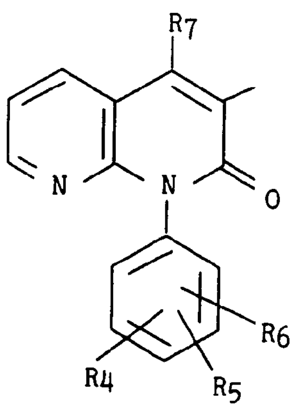

(C)

dans laquelle

$R_4$, $R_5$ et $R_6$ identiques ou différents représentent chacun un atome d'hydrogène, un atome d'halogène, un radical trifluorométhyle, un radical hydroxy, un radical alkyle linéaire ou ramifié de 1 à 6 atomes de carbone, un radical alcoxy de 1 à 6 atomes de carbone, ou un radical alkylthio de 1 à 6 atomes de carbone,

et $R_7$ représente un atome d'hydrogène ou un radical hydroxy,

leurs isomères optiques et leurs sels d'addition avec un acide organique ou minéral pharmaceutiquement acceptable.

La présente invention a également pour objet un procédé de préparation des composés de formule générale I, caractérisé en ce que l'on fait réagir :

ou

un composé de formule II :

$$R - (CH_2)_m - W \qquad (II)$$

dans laquelle R et m ont la même signification que pour la formule I et W représente un atome d'halogène, un radical tosyloxy, ou un radical mésyloxy,

<u>soit</u>

avec un composé de formule III :

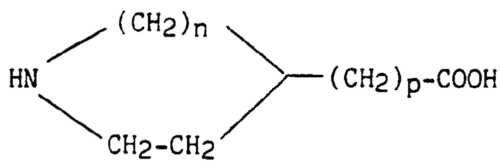

(III)

dans laquelle n et p ont la même signification que pour la formule I, en présence de N,N-diéthyléthylène diamine dans la diméthylformamide ou un autre solvant organique équivalent,

pour former un composé de formule IV :

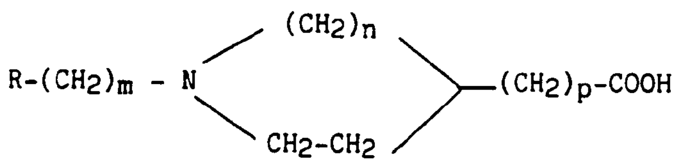

(IV)

dans laquelle R, m, n et p ont la signification indiquée ci-dessus pour la formule I,

que l'on soumet à l'action du chlorure de thionyle ou du chlorure d'oxalyle pour former un chlorure d'acide de formule V :

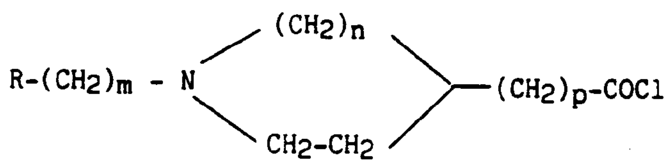

(V)

dans laquelle la signification de R, m, n et p reste identique à celle donnée précédemment pour la formule I,

que l'on condense en présence de chlorure d'aluminium avec un composé de formule VI :

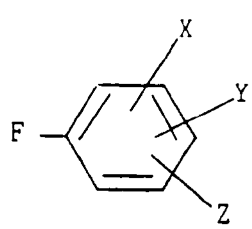

(VI)

dans laquelle X, Y et Z ont la même signification que pour la formule I,

pour former un composé de formule VII :

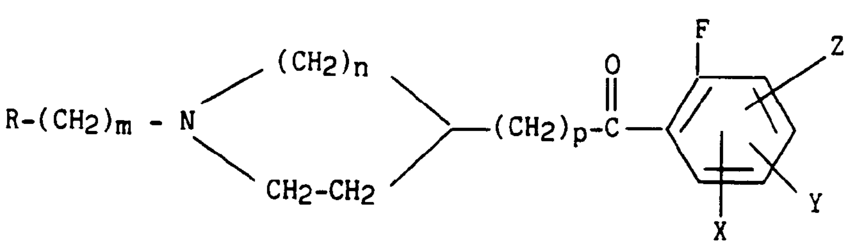

(VII)

dans laquelle R, X, Y, Z, m, n et p ont la même signification que pour la formule I,

que l'on soumet à l'action du chlorhydrate d'hydroxylamine en présence de 2-diéthylamino éthylamine en milieu alcoolique,

pour obtenir un composé de formule VIII :

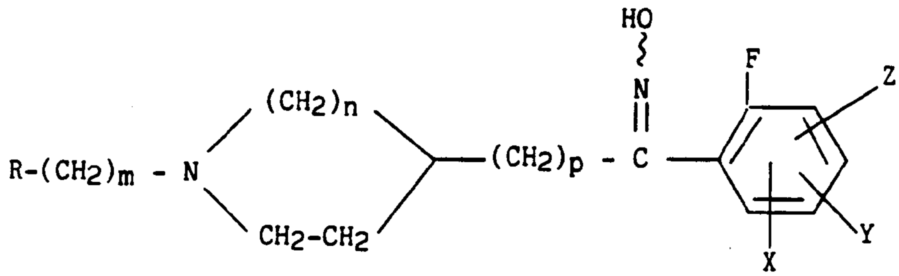

(VIII)

dans laquelle la signification de R, X, Y, Z, m, n et p reste identique à celle donnée précédemment pour la formule I,

que l'on cyclise à l'aide d'une base minérale forte pour obtenir les composés de la formule I,

soit

avec un composé de formule IX :

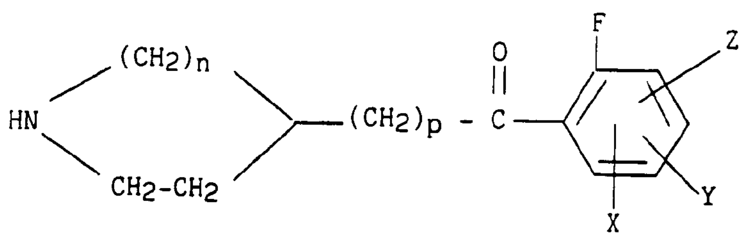

(IX)

dans laquelle X, Y, Z, n et p ont la même signification que pour la formule I, en présence de N,N-diéthyléthylène diamine dans la diméthylformamide ou un autre solvant organique équivalent,

pour former les composés de formule VII, à partir desquels on obtient les composés de formule VIII, puis les composés de formule I selon le procédé indiqué ci-dessus,

soit

avec un composé de formule X :

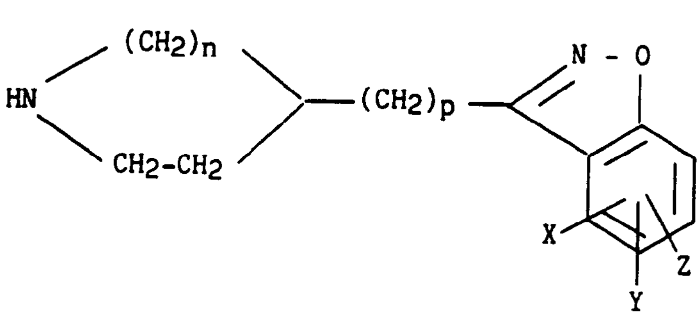

(X)

dans laquelle X, Y, Z, n et p ont la même signification que pour la formule I, en présence de N,N-diéthyléthylène diamine dans la diméthylformamide ou un autre solvant organique équivalent,

pour former les composés de formule I,

ou

un composé de formule $XI_A$ ou $XI_B$ :

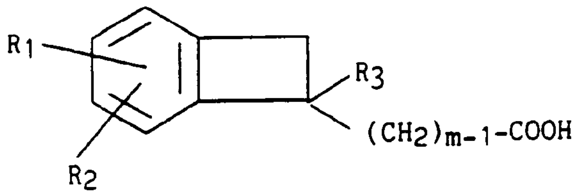

(XIA)

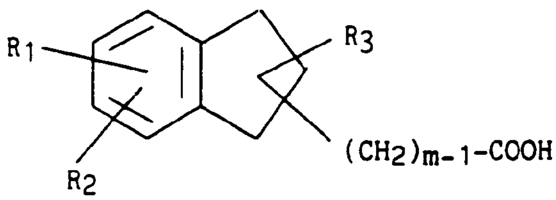

(XIB)

dans lesquelles $R_1$, $R_2$, $R_3$ et m ont la même signification que pour la formule I, sauf que dans ce cas, m, ne peut représenter le nombre 0,
avec un composé de formule X,
en présence de carbonyldiimidazole, pour former un composé de formule $XII_A$ ou $XII_B$ :

(XIIA)

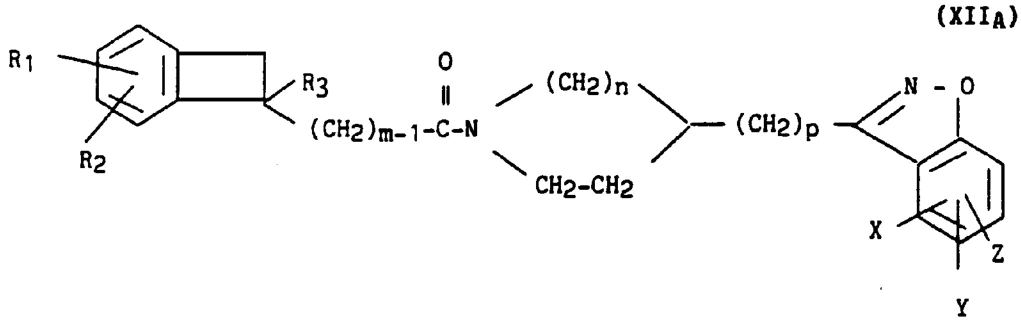

(XIIB)

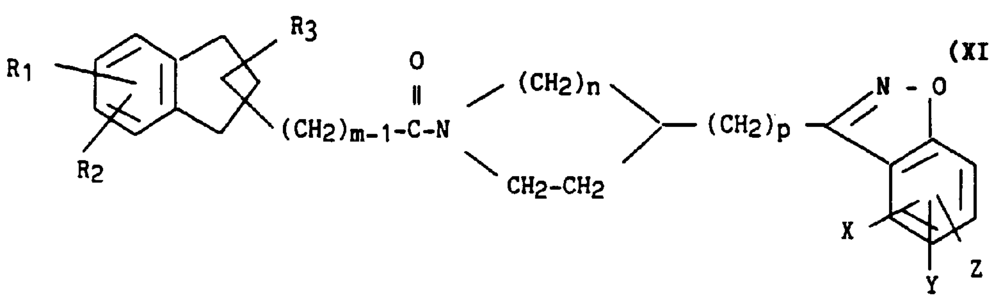

dans lesquelles $R_1$, $R_2$, $R_3$, m, n, p, X, Y, et Z ont la signification donnée pour la formule I, sauf que dans ce cas, m, ne représente pas le nombre 0,
que l'on soumet à l'action d'hydrure de lithium et d'aluminium, pour former les composés de formule I,

ou

un composé de formule XIII :

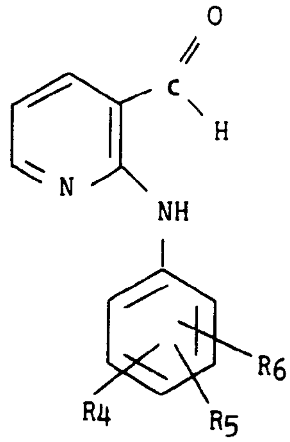

(XIII)

dans laquelle $R_4$, $R_5$, et $R_5$ ont la même signification que pour la formule I,
avec un composé de formule XIV :

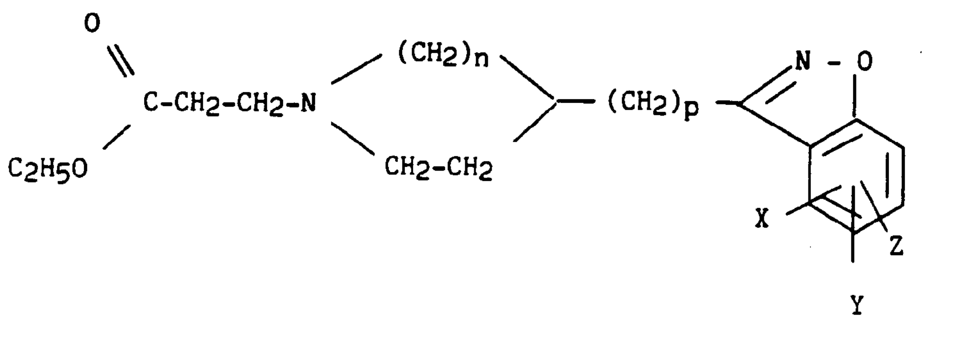

(XIV)

dans laquelle, la signification de n, p, X, Y, et Z reste identique à celle donnée pour la formule I, pour former les composés de formule I dans laquelle R représente un radical de formule C et m est égal à 1,

lesquels composés de formule I,

peuvent être salifiés avec un acide minéral ou organique pharmaceutiquement acceptable, pour former les sels correspondants, ou

être séparés en leurs isomères optiques et ensuite salifiés.

Les composés de la formule II, quand R représente un radical 4-oxo 4H-chromèn-2-yl, sont préparés à partir de la 2-hydroxy acétophénone et du 2-méthylthioacétate d'éthyle (J.Org.Chem.,(1984),49,p. 5038).

Quand R représente un radical benzofuran-2-yle ou 2,3-dihydrobenzofuran-2-yle, les composés de formule II sont préparés respectivement à partir du benzofuran-2-yl carboxylate d'éthyle (J.A.C.S.,(1951),73, p.872) ou de l'acide (2,3-dihydro benzofuran-2-yl) carboxylique (Chim. Ter.,(1973),3,p. 259). Ces deux composés sont soumis à l'action d'hydrure de lithium et d'aluminium pour obtenir les alcools correspondants, qui permettent par des méthodes classiques l'obtention des composés de formule II.

Les composés de formule II, quand R représente un radical (A) sont obtenus à partir des acides de formule $XI_A$, selon un procédé déjà décrit dans la littérature (J.A.C.S., (1975),154,p. 347). Les procédés de synthèse des acides de formule $XI_A$ ou de leurs dérivés sont aussi connus. (J.A.C.S., (1958), 80, p.2257 ; J.A.C.S., (1975), 154, p.347 ; J.Org.Chem.,(1972), 32, p.820 ; J.Org.Chem., (1968), 33, p.3327 ; Tet. Lett.,(1973), 29, p.73).

Les composés de formule IX quand n est égal à 2, peuvent être préparés à partir des composés de formule $III_A$ :

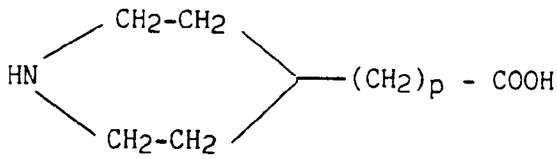

$(III_A)$

dans laquelle p a la même signification que pour la formule I.

Les composés de formule $III_A$ sont soumis à l'action d'anhydride acétique pour former les composés de formule XV :

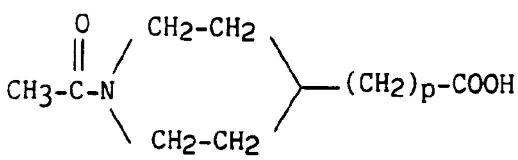

(XV)

dans laquelle la signification de p reste identique à celle donnée pour la formule I.

Ensuite, par des méthodes classiques, on obtient les halogénures d'acyles correspondants que l'on condense avec un composé de formule VI pour obtenir un composé de formule XVI :

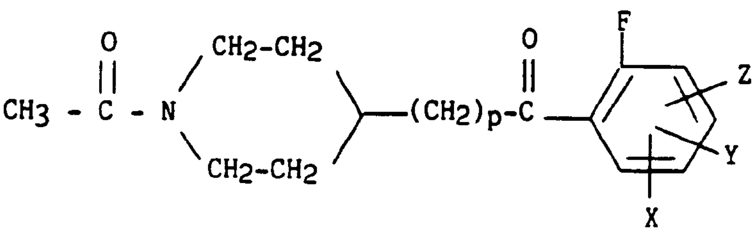

(XVI)

dans laquelle X, Y, Z et p ont la même signification que pour la formule I. A partir de ces composés avec des méthodes classiques (déprotection de l'amine), on obtient les composés attendus.

Pour obtenir les composés de formule X (n=2), on soumet les composés de formule IX, (n=2) ci-dessus mentionnés, à l'action du chlorhydrate d'hydroxylamine en présence de N,N-diéthyléthylène diamine pour obtenir les composés de formule XIV :

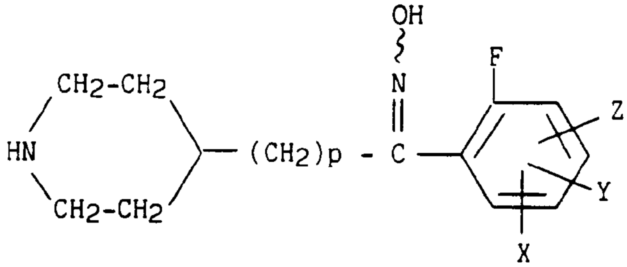

(XIV)

dans laquelle la signification de p, X, Y et Z reste identique à celle donnée pour la formule I. Ces composés permettent par des méthodes classiques déjà mentionnées l'obtention des composés attendus.

Les composés de formule X quand n est égal à 1 sont obtenus à partir de la benzylamine et de l'acide itaconique qui sont condensés à chaud pour obtenir l'acide (1-benzyl 2-oxo pyrrolidin-4-yl) carboxylique. Cet acide est soumis à l'action de l'hydrure de lithium et d'aluminium pour obtenir la 1-benzyl 3-hydroxyméthyl pyrrolidine, qui est transformée à l'aide de chlorure de thionyle en 1-benzyl 3-chlorométhyl pyrrolidine puis en 1-benzyl 3-cyanométhyl pyrrolidine par des méthodes classiques. Ce dernier composé est ensuite hydrolysé par des bases minérales fortes en acide 2-(1-benzyl pyrrolidin-3-yl) acétique qui est

soit, après transformation en chlorure d'acide, mis en réaction en présence de chlorure d'aluminium avec un composé de formule VI pour obtenir un composé de formule $XVII_A$ :

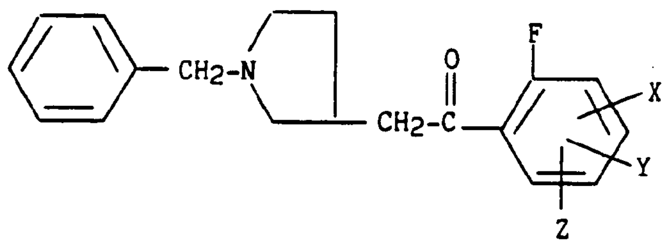

(XVII$_A$)

dans laquelle X, Y et Z ont la même signification que pour la formule I,

soit d'abord transformé avec des procédés connus en acide 3-(1-benzyl pyrrolidin-3-yl) propionique puis en chlorure d'acide et ensuite mis en réaction avec un composé de formule VI pour obtenir un composé de formule $XVII_B$ :

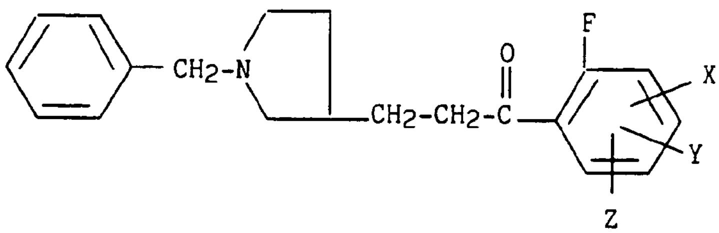

(XVII$_B$)

Les composés $XVII_A$ et $XVII_B$ sont soumis à l'action du chlorhydrate d'hydroxylamine, transformés en dérivés du 1,2-benzisoxazole à l'aide d'une base forte, puis, après déprotection de l'azote du noyau pyrrolidine, on obtient les composés attendus.

Les isomères optiques des composés de la formule I, qui font aussi l'objet de la présente invention peuvent être obtenus par des méthodes classiques. (Salification avec un acide optiquement actif comme par exemple l'acide (+) ou (-) camphosulfonique).

Parmi les acides pharmaceutiquement acceptables pour la préparation des sels d'addition aux composés de formule générale I, on peut citer, les acides phosphorique, chlorhydrique, citrique, iodhydrique, oxalique, maléïque, sulfurique, tartrique, mandélique, fumarique, méthanesulfonique, etc...

Les composés de l'invention ainsi que leurs sels d'addition sont doués de propriétés pharmacologiques fort intéressantes et se distinguent des autres dérivés du 1,2-benzisoxazole déjà connus.

Des essais pharmacologiques ont démontré leur activité antagoniste de la dopamine et de la sérotonine. On peut citer, par exemple, leur capacité à inhiber complètement les stéréotypies induites par le méthylphénidate chez le rat. Les doses actives sont environ 160 fois inférieures à la dose qui provoque la catalepsie. En effet, il est connu que les neuroleptiques classiques, antagonistes dopaminergiques -dont le composé de référence est l'halopéridol- bloquent les stéréotypies induites chez le rat par des agents dopaminergiques comme l'apomorphine, l'amphétamine ou le méthylphénidate. (Janssen P.A.J., Niemegeers (J.E. and Schellekens K.H.L. Arzneim. Forsch,(1965),15,p.104). Pour l'étude des produits de l'invention, on a utilisé comme agent dopaminergique le méthylphénidate parce qu'il permet d'obtenir un modèle particulièrement représentatif de la psychose humaine.

Lors de l'étude pharmacologique, il a été constaté que l'halopéridol inhibe la totalité des stéréotypies provoquées par le méthylphénidate à une dose identique à celle qui provoque la catalepsie. Or, il est connu que

l'induction de la catalepsie est le meilleur facteur d'évaluation des effets secondaires des neuroleptiques et notamment des effets extrapyramidaux. Il est aussi connu que ces effets sont un facteur qui limite leur utilisation en thérapeutique. Les composés de l'invention inhibent la totalité des stéréotypies induites par le méthylphénidate à des doses qui sont très inférieures à la dose cataleptique. En effet, à des doses actives, ils ne produisent pas de catalepsie, et constituent donc une nouvelle classe de neuroleptiques d'importance majeure pour la thérapeutique.

Les composés de l'invention trouvent donc leur application dans le traitement des maladies nécessitant des agents sédatifs, anxiolytiques, antiagressifs, et analgésiques, ou dans le traitement de la schizophrénie et de la dépression.

L'invention s'étend aussi aux compositions pharmaceutiques renfermant comme principe actif au moins un composé de formule générale I ou l'un de ses sels d'addition avec un acide minéral ou organique pharmaceutiquement compatible, en association avec un ou plusieurs excipients inertes et appropriés.

Les compositions pharmaceutiques ainsi obtenues sont présentées avantageusement sous des formes diverses telles que par, exemple comprimés, dragées, gélules, glossettes ou autres préparations galéniques appropriées pour une administration sublinguale, suppositoires, solutions injectables ou buvables.

La posologie peut varier largement en fonction de l'âge, du poids du patient, de la nature et de la sévérité de l'affection ainsi que de la voie d'administration.

La voie d'administration préférée est la voie orale ou parentérale. D'une manière générale, la posologie unitaire s'échelonnera entre 0,2 et 100 mg et la posologie journalière utilisable en thérapeutique humaine entre 0,5 et 500 mg.

Les exemples suivants, donnés à titre non-limitatif, illustrent l'invention.

Les points de fusion indiqués sont mesurés selon la technique Micro-Köfler. Les spectres de résonance magnétique nucléaire du proton (RMN) ont été enregistrés à 200 MHz. Les constantes physiques spectrales des composés de formule générale I sont indiquées dans le Tableau I.

**EXEMPLE 1**

**Iodhydrate de 3-[1-(benzocyclobutèn-1-yl méthyl) pipérid-4-yl] 6-fluoro 1,2-benzisoxazole (R,S)**

**PROCEDE N°1**

STADE A

Acide (1-acétyl piperid-4-yl) carboxylique

On mélange 100 g d'acide pipérid-4-yl carboxylique et 400 ml d'anhydride acétique. On porte 2 heures à reflux puis on abandonne une nuit à température ambiante. On concentre le milieu réactionnel, on triture à l'éther éthylique, filtre et ensuite on lave à l'éther éthylique pour obtenir le composé attendu.
Rendement : 79%
Point de fusion : 175°C

STADE B

Chlorure de (1-acetyl pipérid-4-yl) carbonyle

52 g de l'acide obtenu au stade précédent sont ajoutés par portions à 320 ml de chlorure de thionyle. Après une nuit d'agitation, on filtre le précipité formé qu'on lave plusieurs fois à l'éther isopropylique pour isoler le produit attendu, qu'on utilise immédiatement sans autre purification.
Rendement : 80%

STADE C

1-acétyl 4-(2,4-difluorobenzoyl) pipéridine

On mélange 54,3 g de chlorure d'aluminium et 130 ml de dichloroéthane. On ajoute 24 g de 1,3-difluorobenzène, puis par portions le chlorure d'acide obtenu au stade précédent. On porte le milieu réactionnel à reflux pendant 6 heures et ensuite on le laisse pendant une nuit à température ambiante. On hydrolyse avec de la glace et 190 ml d'acide chlorhydrique concentré. On extrait 5 fois par 100 ml de dichlorométhane, puis on

sèche sur sulfate de magnésium anhydre pour obtenir le composé attendu.
Rendement : 53%
Point de fusion : 97°C

STADE D

4-(2,4-difluorobenzoyl) pipéridine

30 g de 1-acétyl 4-(2,4-difluorobenzoyl) pipéridine sont portés à reflux dans 113 ml d'acide chlorhydrique 6H pendant 6 heures. Le milieu réactionnel est ensuite concentré et le résidu obtenu est cristallisé dans l'isopropanol. On isole après filtration le composé attendu.
Rendement : 83%
Point de fusion : 230°C.

STADE E

Chlorhydrate de 4-(2,4-difluorobenzoyl) pipéridine oxime

Dans 30 ml d'éthanol, on ajoute 3 g du composé obtenu au stade précédent, 3 g de chlorhydrate d'hydroxylamine et 2,7 g de N,N diéthyléthylènediamine et on porte 8 heures à reflux. On abandonne une nuit à température ambiante, filtre et rince à l'éthanol le résidu isolé, pour obtenir le chlorhydrate de 4-(2,4-difluorobenzoyl) pipéridine oxime.
Rendement : 54%
Point de fusion : > 260°C.

Spectre de résonance magnétique nucléaire du proton (solvant DMSO-$d_6$) :

1,7 ppm, m, 2H ; 1,9 ppm, m, 2H ; 2,70 ppm, m, 1H ; 2,9 ppm, m, 2H ; 3,25 ppm, m, 2H ; 7,15 ppm, td, 1H ; 7,3 ppm, m, 2H ; 9,0 ppm, 1H échangeable ; 11,1 ppm, 1H échangeable.

STADE F

6-fluoro 3-pipérid-4-yl 1,2-benzisoxazole

1 g du composé obtenu au stade E est mélangé à 2,3 g d'hydroxyde de potassium et 5 ml d'eau. Le milieu réactionnel est chauffé à reflux pendant 4 heures. Ensuite, on ajoute 20 ml d'eau et on extrait 4 fois par 50 ml de toluène. On sèche la phase organique pour obtenir après évaporation du solvant le 6-fluoro 3-pipérid-4-yl 1,2-benzisoxazole.
Rendement : 80%
Point de fusion : 90°C.

Spectre de résonance magnétique nucléaire du proton (solvant DMSO-$d_6$) :

1,75 ppm, qd, 2H : 1,95 ppm, m, 2H ; 2,7 pmm, m, 2H ; 3,05 ppm, m, 2H ; 3,25 ppm, t, 1H ; 3,3 ppm, 1H échangeable ; 7,3 ppm, td, 1H ; 7,7 ppm, dd, 1H ; 8 ppm, dd, 1H.

STADE G

1-iodométhyl benzocyclobutène

6 g de para-toluènesulfonate de benzocyclobutèn-1-yl méthyle (préparés selon le procédé décrit dans J.A.C.S.,(1975),154,p.347) sont mélangés avec 6,2 g d'iodure de sodium dans 85 ml d'acétone. Le milieu réactionnel est porté à reflux pendant 8 heures, puis coulé sur 150 ml d'eau et extrait plusieurs fois à l'éther éthylique. La phase organique est ensuite lavée avec une solution normale de thiosulfate de sodium, séchée sur sulfate de magnésium anhydre et concentrée pour obtenir le composé attendu sous forme d'huile.
Rendement : 88%

Spectre de résonance magnétique nucléaire du proton (solvant $CDCl_3$) :

2,85 ppm, d, 1H ; 3,3 à 3,6 ppm, m, 3H ; 3,9 ppm, m, 1H ; 7 à 7,3 ppm, m, 4H.

STADE H

5,54 g du composé obtenu au stade précédent, 5 g du composé obtenu au stade F, et 3,18 ml de triéthylamine sont mélangés dans 100 ml de diméthylformamide puis amenés à 60°C pendant 8 heures. On concentre ensuite et on reprend par l'eau. On filtre le précipité formé, on lave plusieurs fois à l'eau puis à l'éther éthylique. On sèche et on recristallise le composé obtenu dans le méthanol.
Rendement : 29%
Point de fusion : 270-273°C

Analyse élémentaire :

| | C% | H% | N% | I% |
|---|---|---|---|---|
| Théorie | 54,32 | 4,78 | 6,03 | 27,33 |
| Trouvé | 53,91 | 4,92 | 6,10 | 27,26 |

**PROCEDE N°2**

STADE A

Acide [1-(benzocyclobutèn-1-yl méthyl) piperid-4-yl] carboxylique

On mélange 0,028 mole de 1-iodométhyl benzocyclobutène (Procédé 1 Stade G), 0,028 mole d'acide pipérid-4yl carboxylique, 0,028 mole de triéthylamine dans 95 ml de diméthylfomamide. On porte 6 heures à 60°C. On évapore à sec, reprend par de l'acétate d'éthyle et de l'eau et on décante.

On sèche sur sulfate de magnésium et on concentre. On purifie sur colonne de silice en utilisant comme solvant d'élution un mélange de toluène et de méthanol (95 : 5 V/V).
Rendement : 30 %

STADE B

1-(benzocyclobutèn-1-yl methyl) 4-(2,4-difluorobenzoyl) pipéridine

Couler 0,025 mole de chlorure de [1-(benzocyclobutèn-1-yl méthyl) pipérid-4-yl] carbonyle (préparé par action du chlorure d'oxalyle sur l'acide décrit ci-dessus) en solution dans 50 ml de dichlorométhane sur une suspension de 0,025 mole de 1,3-difluorobenzène, et 0,026 mole de trichlorure d'aluminium dans 10 ml de dichlorométhane. A la fin du dégagement gazeux ou hydrolyse sur glace, on décante et on extrait par l'acide chlorhydrique 1 N. La phase aqueuse est ensuite alcalinisée et extraite par le dichlorométhane. Après évaporation du solvant on obtient le produit attendu.
Rendement : 35 %
Point de fusion : 70°C

Spectre de résonance magnétique nucléaire du proton (solvant $CDCl_3$) :

2,05-1,70 ppm m, 4H ; 2,2 ppm, m, 2H ; 2,65 ppm, dd, 1H ; 2,85 ppm, m, 2H 3,10 ppm, m, 3H ; 3,4 ppm, dd, 1H ; 3,7 ppm, m, 1H ; 6,8-7,3 ppm, m, 6H 7,9 ppm, m, 1H.

STADE C

1-(benzocyclobutèn-1-yl méthyl) 4-(2,4-difluorobenzoyl) pipéridine oxime.

Ce composé a été synthétisé à partir de la cétone décrite au stade B et en appliquant le procédé décrit dans le stade E du procédé N°1. Le composé a été purifié sur colonne de silice en utilisant comme éluant un mélange de dichlorométhane et de méthanol (95:5 V/V).

Spectre de résonance magnétique nucléaire du proton (solvant DMSO-$d_6$) :

1,6-2,1 ppm, m, 4H ; 2,1-2,6 ppm, m, 2H ; 2,5-3,6 ppm, m, 7H ; 3,7 ppm, m, 1H ; 7,05-7,35 ppm, m, 7H ; 10,95 ppm 1H échangeable.

STADE D

Le 3-[1-benzocyclobutèn-1-yl méthyl) pipérid-4-yl] 6-fluoro 1,2-benzisoxazole (R,S) a été synthétisé à partir de l'oxime décrite ci-dessus et selon la méthode donnée dans le stade F du procédé N°1.

**PROCEDE N°3**

STADE A

1-(benzocyclobutèn-1-yl methyl) 4-(2,4-difluorobenzoyl) pipéridine

Mélanger puis porter à reflux pendant 18 heures 0,0019 mole du chlorhydrate de 4-(2,4-difluorobenzoyl) pipéridine, 0,0019 mole de paratoluènesulfonate de benzocyclobutèn-1-yl méthyle et 0,04 mole de triéthylamine en solution dans 50 ml de toluène. Extraire ensuite le milieu réactionnel par l'acide chlorhydrique 1 N, basifier la phase aqueuse et extraire au dichlorométhane. L'huile obtenue est purifiée sur colonne de silice en utilisant comme éluant un mélange de cyclohexane et d'acétone (93 : 7 V/V).
Rendement : 30 %

STADE A

En appliquant le mode opératoire décrit dans le procédé N°2 stades C et D on obtient à partir de la cétone décrite ci-dessus le 3-[1-(benzocyclobutèn-1-yl méthyl) pipérid-4-yl] 6-fluoro 1,2-benzisoxazole (R,S).

EXEMPLE 2

Fumarate de 3-[1-(2-benzocyclobutèn-1-yl éthyl) pipérid-4-yl] 6-fluoro 1,2-benzisoxazole (R,S)

STADE A

2-benzocyclobutèn-1-yl éthanol

Sous courant d'azote, on charge dans un tricol 7,4 g d'hydrure de lithium et d'aluminium et ensuite on y ajoute 70 ml de tétrahydrofurane anhydre. On coule ensuite goutte à goutte une solution d'acide 2-benzocyclobutèn-1-yl acétique (préparé selon le procédé décrit dans J.Org.Chem.,(1979),44,p. 1036) dans 120 ml de tétrahydrofurane. On laisse la solution réactionnelle une nuit sous agitation. On refroidit ensuite le ballon par un bain glacé et on hydrolyse en ajoutant très doucement 6,6 ml d'eau, 6,6 ml d'hydroxyde de sodium 4H puis 19,8 ml d'eau. On essore le complexe, on lave à l'éther éthylique et on sèche sur sulfate de magnésium. Après filtration, on évapore à sec pour obtenir le composé attendu.
Rendement : 85%

Spectre de résonance magnétique nucléaire du proton (solvant $CDCl_3$) :

1,5 ppm, 1H échangeable ; 2,0 ppm, q, 2H ; 2,8 ppm, dd, 1H ; 3,4 ppm, dd, 1H ; 3,6 ppm, m, 1H ; 3,85 ppm, t, 2H ; 7,2 + 7,1 ppm, m+m, 2+2H.

STADE B

1-bromo 2-benzocyclobutèn-1-yl éthane

On charge dans un tricol 9,6 g de l'alcool obtenu ci-dessus en solution dans 13 ml de benzène anhydre. On ajoute ensuite lentement 2,07 ml de tribromure de phosphore. On refroidit à 0°C et on agite pendant 30 minutes. Ensuite, on porte à reflux pendant 30 minutes. On refroidit, on dilue à l'éther éthylique et on verse la solution ainsi obtenue sur de l'eau glacée. On décante et on lave avec une solution aqueuse de chlorure de sodium saturée jusqu'à neutralité. On sèche ensuite sur sulfate de magnésium anhydre, on filtre et on distille pour obtenir le composé attendu.
Rendement : 63%

Spectre de résonance magnétique nucléaire du proton (solvant $CDCl_3$) :

2,25 ppm, q, 2H ; 2,8 ppm, dd, 1H ; 3,4 ppm, dd, 1H ; 3,5 ppm, t, 2H ; 3,65 ppm, m, 1H ; 7,3-7,0 ppm, m, 4H.

STADE C

Dans une solution contenant 4,4 g de 6-fluoro 3-pipérid-4-yl 1,2-benzisoxazole dans 80 ml de diméthylformamide on ajoute 2,8 ml de triéthanolamine et ensuite goutte à goutte 4,2 g du composé obtenu au stade précédent. On chauffe à 60°C pendant 6 heures et ensuite on évapore à sec. On reprend le résidu sur 70 ml d'éther éthylique et 30 ml d'acide chlorhydrique 1N. On lave le précipité formé à l'éther éthylique. On reprend ensuite par 150 ml d'acétate d'éthyle et par 50 ml d'hydroxyde de sodium 1N. On décante et on lave à neutralité avec une solution aqueuse saturée de chlorure de sodium. On sèche sur sulfate de magnésium anhydre et on évapore pour obtenir le 3-[1-(2-benzocyclobutèn-1-yl éthyl) pipérid-4-yl] 6-fluoro 1,2-benzisoxazole (R,S).

Pour préparer le fumarate, on verse sur 5,65 g de la base en solution dans 5 ml d'éthanol 1,87 g d'acide fumarique en solution dans 27 ml d'éthanol. On filtre, on évapore à sec, et on recristallise dans l'acétonitrile.
Point de fusion : 170°-190°C (décomposition)

Analyse élémentaire :

| | C% | H% | N% |
|---|---|---|---|
| Théorie | 66,94 | 5,83 | 6,00 |
| Trouvé | 67,25 | 5,96 | 6,35 |

EXEMPLE 3

Chlorhydrate de 6-fluoro 3-{1-[(1-méthyl benzocyclobutèn-1-yl) méthyl] pipérid-4-yl} 1,2-benzisoxazole (R,S)

STADE A

1-iodométhyl 1-méthyl benzocyclobutène

A une solution de 5,0 g de tosylate de 1,1'-diméthyl benzocyclobutène (préparé selon le procédé décrit dans J.Org.Chem., (1972),32,p.820) dans 80 ml d'acétone, on ajoute 5,0 g d'iodure de sodium et on prépare le composé attendu selon le procédé décrit dans l'exemple 1 Stade G.
Rendement : 88%

Spectre de résonance magnétique nucléaire du proton (solvant $CDCl_3$) :

1,55 ppm, s, 3H ; 3,15 ppm, dd, 2H ; 3,55 ppm, dd, 2H ; 6,9 à 7,3 ppm, m, 4H.

STADE B

L'iodhydrate de 6-fluoro 3-{1-[1-méthyl benzocyclobutèn-1-yl méthyl] pipérid-4-yl} 1,2-benzisoxazole (R,S) est préparé à partir du composé décrit au stade précédent et le 6-fluoro 3-(pipérid-4-yl) 1,2-benzisoxazole selon le procédé décrit au dernier stade de l'exemple 1. Le résidu est repris par l'acide chlorhydrique 1N et lavé à l'éther éthylique. Puis on basifie avec l'hydroxyde de sodium 1N et on extrait à l'éther éthylique. On lave à l'eau, sèche sur sulfate de magnésium anhydre, filtre et évapore à sec. Le produit obtenu est purifié par flash chromatographie en utilisant comme éluant un mélange de dichlorométhane et de méthanol (95 : 5 V/V). A la base obtenue, solubilisée dans l'éther éthylique, on ajoute de l'acide chlorhydrique en solution dans l'éthanol (3,5 N).

Le sel formé est recristallisé dans un mélange de méthanol et d'éthanol.
Rendement : 14%
Point de fusion : 215-230°C

Analyse élémentaire :

| | C% | H% | N% | Halogène % |
|---|---|---|---|---|
| Théorie | 68,30 | 6,25 | 7,24 | 9,16 |
| Trouvé | 67,58 | 6,14 | 7,26 | 9,22 |

**EXEMPLE 4**

**3-[1-(2,3-dihydrobenzofuran-2-yl méthyl) pipérid-4-yl] 6-fluoro 1,2-benzisoxazole (R,S)**

STADE A

2,3-dihydrobenzofuran-2-yl méthanol

Dans un tricol, on charge 17 g d'hydrure de lithium et d'aluminium en solution dans l'éther éthylique anhydre. Ensuite, on coule 3 g d'acide 2,3-dihydrobenzofuran-2-yl carboxylique (Chim.Ler.,(1973),3,p.259) en solution dans 1 140 ml d'éther éthylique. On laisse le milieu réactionnel sous agitation pendant une nuit. Ensuite, dans un bain de glace, on hydrolyse par 15,2 ml d'eau, 15,2 ml d'hydroxyde de sodium 4N puis 45,2 ml d'eau. On essore le complexe, on lave à l'éther éthylique, on sèche le filtrat et on distille pour obtenir le composé attendu.
Rendement : 90%

Spectre de résonance magnétique nucléaire du proton (solvant $CDCl_3$) :

2,0 ppm, 1H échangeable ; 3,05 ppm, dd, 1H ; 3,25 ppm, dd, 1H ; 3,8 ppm, m, 2H ; 4,9 ppm, m, 1H ; 6,85 ppm, m, 2H ; 7,15 ppm, d, 2H.

STADE B

Para-toluènesulfonate de 2,3-dihydrobenzofuran-2-yl méthyle

On charge dans un tricol 25,5 g de l'alcool obtenu au stade précédent et 170 ml de pyridine. On y ajoute par portions 32,5 g de paratoluène sulfochlorure. On laisse le milieu réactionnel sous agitation pendant une nuit à température ambiante. On évapore à sec et on reprend par un mélange contenant 900 ml d'éther éthylique et 250 ml d'acide sulfurique 0,1N. On décante et on lave la phase organique avec une solution d'acide sulfurique 0,1N puis avec une solution de carbonate acide de sodium à 10% dans l'eau. On sèche la phase organique sur sulfate de magnésium anhydre et on évapore à sec. Le résidu est recristallisé dans le cyclohexane.
Rendement : 54%
Point de fusion : 69-71°C.

Spectre de résonance magnétique nucléaire du proton (solvant $CDCl_3$) :

2,5 ppm, s, 3H ; 3,0 ppm, dd, 1H ; 3,3 ppm, dd, 1H ; 4,2 ppm, d, 2H ; 4,95 ppm, m, 1H ; 6,7 ppm, d, 1H ; 6,85 ppm, t, 1H ; 7,10 ppm, t, 1H ; 7,15 ppm, d, 1H ; 7,35 ppm, d, 2H ; 7,8 ppm, d, 2H.

STADE C

2-iodométhyl 2,3-dihydrobenzofurane

On porte à reflux pendant 2 heures 6,1 g du produit obtenu au stade précédent, 40 ml d'acétone anhydre et 4,5 g d'iodure de sodium. On verse le milieu réactionnel sur 50 ml d'eau et 50 ml d'éther éthylique. On décante, et on extrait plusieurs fois à l'éther éthylique. Les extraits éthérés sont lavés au thiosulfate de sodium 0,5N, puis à l'eau saturée en chlorure de sodium. On sèche la phase organique sur sulfate de magnésium anhydre et on évapore à sec pour obtenir le composé attendu.
Rendement : 96%.

STADE D

Pour obtenir le 3-[1-(2,3-dihydrobenzofuran-2-yl méthyl) pipérid-4-yl] 6-fluoro 1,2-benzisoxazole (R,S), on fait réagir 5 g du composé obtenu au stade précédent avec 3,65 g de 6-fluoro 3-pipérid-4-yl 1,2-benzisoxazole en présence de 2,4 ml de triéthanolamine selon le procédé décrit dans le dernier stade de l'exemple 1. Le solide obtenu est repris par 100 ml d'acide chlorhydrique 1N et 50 ml d'éther éthylique. On essore le précipité formé et on le reprend par 150 ml d'éther éthylique et 100 ml d'hydroxyde de sodium 1N. On décante et on lave la phase organique à neutralité avec de l'eau saturée en chlorure de sodium. On sèche sur sulfate de magnésium, on évapore à sec et on recristallise le résidu obtenu dans l'acétate d'éthyle.
Rendement : 33%
Point de fusion : 115-117°C.

Analyse élémentaire :

| | C% | H% | N% |
|---|---|---|---|
| Théorie | 71,57 | 6,01 | 7,95 |
| Trouvé | 71,48 | 6,06 | 7,99 |

**EXEMPLE 5**

**6-fluoro 3-[1-(7-méthoxy 2,3-dihydrobenzofuran-2-yl méthyl) pipérid-4-yl] 1,2-benzisoxazole (R,S)**

STADE A

2-allyloxy 1-méthoxy benzène

124 g de 2-méthoxy phénol, et 152 g de carbonate de potassium dans 500 ml de diméthylformamide sont portés sous agitation à 60°C pendant 30 minutes. On coule 127 g de bromure d'allyle, puis on laisse le milieu réactionnel à 60°C pendant 1 heure. On dilue avec 2 l d'eau, extrait à l'éther éthylique, lave à l'hydroxyde de sodium. La phase organique est séchée et l'éther évaporé. Ensuite, on distille le produit à 115°C sous 20 mmHg pour obtenir 129,6 g du composé attendu.
Rendement : 79,5%

Spectre de résonance magnétique nucléaire du proton (solvant $CDCl_3$) :

3,8 ppm, s, 3H ; 4,55 ppm, m, 2H ; 5,1 à 5,5 ppm, m, 2H ; 4,4 à 5,75 ppm, m, 1H ; 6,85 ppm, m, 4H.

STADE B

2-allyl 6-méthoxy phénol

129 g du composé obtenu au stade précédent sont portés à 230°C pendant une heure. On reprend le milieu par 500 ml d'éther éthylique, lave à l'hydroxyde de sodium 2,5N puis à l'eau. On sèche la phase organique, puis on évapore. On distille à 145°C sous 24 mmHg pour obtenir le composé attendu.
Rendement : 96%

Spectre de résonance magnétique nucléaire du proton (solvant $CDCl_3$) :

3,4 et 3,3 ppm, m+m, 2H ; 3,85 ppm, s, 3H ; 4,0 ppm, m, 1H ; 5,05 ppm, m, 2H ; 5,7 à 5,5 ppm, 1H échangeable ; 6,9 à 6,65 ppm, m, 3H.

STADE C

(7-méthoxy 2,3-dihydro benzofuran-2-yl) méthanol

Dans un mélange refroidi à 15°C, contenant 160 ml d'acide peracétique à 32% dans l'acide acétique et 2,4 g d'acétate de sodium, on ajoute 124 g de 2-allyl 6-méthoxy phénol dissous dans 80 ml d'acide acétique. On laisse à température ambiante pendant 48 heures, puis on hydrolyse sur 2 l d'eau contenant 400 g de carbonate de sodium. On extrait à l'éther éthylique et on lave la phase organique à l'hydroxyde de sodium. On sèche, on évapore l'éther et on distille à 110°C sous 0,02 mmHg pour obtenir le 7-méthoxy 2,3-dihydrobenzofuran-2-yl méthanol.
Rendement : 16%.

Spectre de résonance magnétique nucléaire du proton (solvant $CDCl_3$) :

2,1 ppm, 1H échangeable ; 3,0 à 3,3 ppm, m, 2H ; 3,7 à 4,0 ppm, m+s, 2H+3H 5,0 ppm, m, 1H ; 6,7 ppm, m, 3H.

STADE D

Tosylate de (7-méthoxy 2,3-dihydrobenzofuran-2-yl) méthyle

A 22 g de l'alcool obtenu au stade précédent, dissous dans 100 ml de pyridine, on ajoute par portions 22,8 g de para-toluène sulfochlorure. Ensuite, on procède selon la méthode décrite dans l'exemple 4, stade B, en utilisant comme solvant organique d'extraction l'acétate d'éthyle.
Rendement : 85%
Point de fusion : 108-110°C.

Spectre de résonance magnétique nucléaire du proton (solvant $CDCl_3$) :

2,5 ppm, s, 3H ; 3,1 ppm, m, 1H ; 3,4 ppm, m, 1H ; 3,9 ppm, s, 3H ; 4,2 ppm, m, 2H ; 5,0 ppm, m, 1H ; 6,8 ppm, m, 3H ; 7,3 ppm, d, 2H ; 7,8 ppm, d, 2H.

STADE E

2-iodométhyl 7-méthoxy 2,3-dihydrobenzofurane

Ce composé a été obtenu selon le procédé décrit dans l'exemple 4, stade C, à partir du tosylate de (7-méthoxy 2,3-dihydrobenzofuran-2-yl) méthyle et de l'iodure de sodium.
Rendement : 95%

Spectre de résonance magnétique nucléaire du proton (solvant $CDCl_3$) :

3,2 à 3,5 ppm, m, 2H ; 3,0 à 3,6 ppm, m, 2H ; 3,9 ppm, s, 3H ; 5,0 ppm, m, 1H ; 6,8 ppm, m, 3H.

STADE F

Le 6-fluoro 3-[1-(7-méthoxy 2,3-dihydrobenzofuran-2-yl méthyl) pipérid-4-yl] 1,2-benzisoxazole (R,S) est obtenu à partir du 2-iodométhyl 7-méthoxy 2,3-dihydrobenzofurane et le 6-fluoro 3-pipérid-4-yl 1,2-benzisoxazole selon le procédé décrit dans l'exemple 4, stade D.
Rendement : 31,5%
Point de fusion : 120-122°C.

Analyse élémentaire :

| | C% | H% | N% |
|---|---|---|---|
| Théorie | 69,09 | 6,06 | 7,32 |
| Trouvé | 68,90 | 6,00 | 7,55 |

EXEMPLE 6

(R,S) 6-fluoro 3-{1-[(5-fluoro 2,3-dihydrobenzofuran-2-yl) méthyl] pipérid-4-yl} 1,2-benzisoxazole

STADE A

1-allyloxy 4-fluoro benzène

Ce composé a été préparé à partir du 4-fluorophénol et du bromure d'allyle selon le procédé décrit dans l'exemple 5 stade A. Le solvant utilisé est l'acétone et la réaction a été réalisée en présence de soude.
Rendement : 75%

Spectre de résonance magnétique nucléaire du proton (solvant $CDCl_3$) :

4,5 ppm, m, 2H ; 5,15 à 5,55 ppm, m, 2H ; 5,6 à 6,5 ppm, m, 1H ; 4,65 à 7,1 ppm, m, 4H.

STADE B

2-allyl 4-fluoro phénol

Ce composé a été obtenu à partir du 1-allyloxy 4-fluoro benzène selon le procédé décrit dans l'exemple 5 stade B.
Rendement : 86%

Spectre de résonance magnétique nucléaire du proton (solvant $CDCl_3$) :

3,4 ppm, m, 2H ; 5,1 ppm, 1H échangeable ; 4,95 à 5,4 ppm, m, 2H ; 5,7 à 6,4 ppm, m, 1H ; 6,6 à 7,1 ppm, m, 3H.

STADE C

(5-fluoro 2,3-dihydrobenzofuran-2-yl) méthanol

Ce composé a été préparé à partir du phénol obtenu au stade précédent selon le procédé décrit dans l'exemple 5 stade C.
Rendement : 46%

Spectre de résonance magnétique nucléaire du proton (solvant $CDCl_3$) :

2,0 ppm, 1H échangeable; 2,9 à 3,3 ppm, m, 2H ; 3,6 à 3,9 ppm, m, 2H ; 5,0 ppm, m, 1H ; 6,5 à 6,9 ppm, m, 3H.

STADE D

Tosylate de (5-fluoro 2,3-dihydrobenzofuran-2-yl) méthyle

En utilisant le procédé décrit dans l'exemple 5, stade D, on obtient à partir du (5-fluoro 2,3-dihydrobenzofuran-2-yl) méthanol le composé attendu.
Rendement : 83%
Point de fusion : < 50°C

Spectre de résonance magnétique nucléaire du proton (solvant $CDCl_3$) :

2,45 ppm, s, 3H ; 3,0 ppm, m, 1H ; 3,3 ppm, m, 1H ; 4,15 ppm, d, 2H ; 4,95 ppm, m, 1H ; 6,5 à 6,9 ppm, m, 3H ; 7,8 ppm, d, 2H ; 7,75 ppm, d, 2H.

STADE E

5-fluoro 2-iodométhyl 2,3-dihydrobenzofurane

Ce composé a été obtenu à partir du tosylate de (5-fluoro 2,3-dihydrobenzofuran-2-yl) méthyle, selon le procédé décrit dans l'exemple 4 stade C.
Rendement : 96%

Spectre de résonance magnétique nucléaire du proton (solvant $CDCl_3$) :

3,0 ppm, m, 1H ; 3,2 à 3,5 ppm, m, 3H ; 4,9 ppm, m, 1H ; 6,5 à 6,9 ppm, m, 3H.

STADE F

Le 6-fluoro 3-{1-[(5-fluoro 2,3-dihydrobenzofuran-2-yl) méthyl] pipérid-4-yl} 1,2-benzisoxazole (R,S) est obtenu à partir du composé préparé au stade précédent selon le procédé décrit dans l'exemple 4, stade D.
Rendement : 11%
Point de fusion : 129-130°C

Analyse élémentaire :

| | C% | H% | N% |
|---|---|---|---|
| Théorie | 68,10 | 5,44 | 7,56 |
| Trouvé | 67,84 | 5,61 | 7,76 |

**EXEMPLE 7**

**Chlorhydrate de 6-fluoro 3-[1-(benzofuran-2-yl méthyl) pipérid-4-yl] 1,2-benzisoxazole (R,S)**

STADE A

benzofuran-2-yl méthanol

Ce composé a été préparé à partir de benzofuran-2-yl carboxylate d'éthyle (J.A.C.S.,(1951),73,p. 872) selon le procédé décrit dans l'exemple 4 stade A.

Rendement : 85%

Spectre de résonance magnétique nucléaire du proton (solvant $CDCl_3$) :

2,15 ppm, 1H échangeable, 4,8 ppm, s, 2H ; 6,65 ppm, s, 1H ; 7,20 à 7,35 ppm, m, 2H ; 7,5 ppm, dd, 1H ; 7,6 ppm, dd ,1H.

STADE B

2-chlorométhyl benzofurane

Dans un tricol, on charge 33,2 g de benzofuran-2-yl méthanol en solution dans 450 ml de chloroforme anhydre, puis on coule goutte à goutte 48,8 ml de chlorure de thionyle et on monte doucement la température à reflux et on la maintient pendant 3 heures et 30 mn. On refroidit ensuite le milieu réactionnel et on le verse sur 1 l d'eau, puis on le dilue avec 500 ml de dichlorométhane. Après décantation, on lave la phase organique à neutralité, on la sèche sur sulfate de magnésium anhydre, on évapore le solvant et on distille le résidu sous vide pour obtenir le composé attendu.
Rendement : 80%

Spectre de résonance magnétique nucléaire du proton (solvant $CDCl_3$) :

4,7 ppm, s, 2H ; 6,75 ppm, s, 1H ; 7,25 ppm, td, 1H ; 7,3 ppm, td, 1H ; 7,5 ppm, d, 1H ; 7,6 ppm, dd, 1H.

STADE C

Le 6-fluoro 3-[1-(benzofuran-2-yl méthyl) pipérid-4-yl] 1,2-benzoisoxazole (R,S) est préparé selon le procédé décrit dans l'exemple 1, à partir du 2-chlorométhyl benzofurane et du 6-fluoro 3-(pipérid-4-yl) 1,2-benzisoxazole.
Rendement : 54%

Pour obtenir le chlorhydrate correspondant, la base est dissoute dans l'acétonitrile et ensuite on y ajoute une quantité adéquate d'acide chlorhydrique en solution dans l'éther éthylique. Après évaporation du solvant, on obtient le sel attendu, qui est ensuite recristallisé dans l'acétonitrile.
Point de fusion : Décomposition à partir de 190°C.

Analyse élémentaire :

| | C% | H% | N% | Cl% |
|---|---|---|---|---|
| Théorie | 65,20 | 5,21 | 7,24 | 9,16 |
| Trouvé | 64,90 | 5,50 | 7,48 | 9,18 |

**EXEMPLE 8**

**Chlorhydrate de 6-fluoro 3-{1-[(4-oxo 4H-chromèn-2-yl) méthyl] pipérid-4-yl} 1,2-benzisoxazole**

STADE A

2-méthylthiométhyl 4-oxo 4H-chromène

A 20 g d'hydrure de sodium à 50% en suspension dans 15 ml de tétrahydrofurane, on ajoute rapidement un mélange contenant 14,3 g de 2-hydroxyacétophénone et 28,3 g de méthylthioacétate d'éthyle dans 25 ml de tétrahydrofurane. On porte ensuite 30 mn à reflux. On hydrolyse sous courant d'azote par 500 ml d'eau et on ajoute 500 ml de méthanol, 70 ml d'acide chlorhydrique concentré et on porte à reflux pendant une heure. On évapore le méthanol, on extrait à l'éther éthylique, on lave la phase organique à la soude et on évapore le

solvant pour obtenir le produit attendu.
Rendement : 38%
Point de fusion : 77-79°C

Spectre de résonance magnétique nucléaire du proton (solvant $CDCl_3$) :

2,2 ppm, s, 3H ; 3,6 ppm, s, 2H ; 6,3 ppm, s, 1H ; 7,3 à 7,5 ppm, m, 2H ; 7,7 ppm, m, 1H ; 8,2 ppm, dd, 1H.

STADE B

2-iodométhyl 4-oxo 4H-chromène

8 g de 2-méthylthiométhyl 4-oxo 4H-chromène, 200 ml d'iodure de méthyle et 26 ml de dichlorométhane sont portés à reflux sous agitation pendant 4 jours. On filtre le précipité, on évapore l'excès des solvants et réactifs, on reprend par l'ether éthylique, on lave avec une solution de thiosulfate de sodium, on sèche et on évapore.
Rendement : 83%
Point de fusion : 144-146°C

Spectre de résonance magnétique nucléaire du proton (solvant $CDCl_3$)

4,25 ppm, s, 2H ; 6,35 ppm, s, 1H ; 7,3 à 7,5 ppm, m, 2H ; 7,65 ppm, td, 1H ; 8,15 ppm, dd, 1H.

STADE C

Le 6-fluoro 3-{1-[(4-oxo 4H-chromèn-2-yl) méthyl] pipérid-4-yl} 1,2-benzisoxazole est obtenu par condensation du composé obtenu au stade précédent avec le 6-fluoro 3-pipérid-4-yl 1,2-benzisoxazole selon le procédé décrit dans le dernier stade de l'exemple 1. Le chlorhydrate est obtenu après addition d'une quantité adéquate d'acide chlorhydrique N. Recristallisation dans le méthanol.
Rendement : 38%
Point de fusion : > 260°C

Analyse élémentaire :

| | C% | H% | N% | Cl% |
|---|---|---|---|---|
| Théorie | 63,69 | 4,86 | 6,75 | 8,55 |
| Trouvé | 63,27 | 5,12 | 6,79 | 8,59 |

**EXEMPLE 9**

**Chlorhydrate de 3-{1-{[1,2-dihydro 2-oxo 1-(3-trifluorométhyl phényl) 1,8-naphthyridin-3-yl] éthyl} pipérid-4-yl} 6-fluoro 1,2-benzisoxazole**

STADE A

2-[(3-trifluorométhyl phényl) amino] pyrid-3-yl méthanol

On coule doucement 500 ml d'éther éthylique anhydre sur 26 g d'hydrure de lithium et d'aluminium, puis on ajoute goutte à goutte 100 g d'acide niflumique dissous dans 250 ml de tétrahydrofurane anhydre. On porte ensuite à reflux pendant 3 heures. Après refroidissement, on hydrolyse avec 150 ml d'acétate d'éthyle et 100 ml d'une solution de sulfate de sodium saturée. Après filtration, on concentre le filtrat pour obtenir le composé attendu sous forme de solide jaune.
Rendement : 85%
Point de fusion: 102°C

Spectre de résonance magnétique nucléaire du proton (solvant $CDCl_3$) :

2,1 ppm, 1H échangeable, 4,7 ppm, s, 2H ; 6,75 ppm, dd, 1H ; 7,15 à 7,5 ppm, m, 3H ; 7,75 ppm, d, 1H ; 7,85 ppm, 1H échangeable ; 7,9 ppm, s, 1H ; 8,25 ppm, dd, 1H.

STADE B

2-[(3-trifluorométhyl phényl) amino] pyrid-3-yl carbaldéhyde

A 80 g de l'alcool précédemment préparé dissous dans un litre de chloroforme, on ajoute 260 g d'oxyde de manganèse, et on laisse réagir pendant environ 48 heures à température ambiante. Ensuite, on filtre sur célite, on lave au chlorure de méthylène et on concentre. Le résidu obtenu est recristallisé dans l'heptane.
Rendement : 54%
Point de fusion : 76°C

Spectre de résonance magnétique nucléaire du proton (solvant $CDCl_3$) :

6,95 ppm, 2d, 1H ; 7,35 ppm, d, 1H ; 7,45 ppm, t, 1H ; 7,9 ppm ,dd+m, 2H ; 8,2 ppm, s, 1H ; 8,5 ppm, dd, 1H ; 9,95 ppm, s, 1H ; 10,6 ppm, s, 1H.

STADE C

2-[1,2-dihydro 2-oxo 1-(3-trifluorométhyl phényl) 1,8-naphthyridin-3-yl] éthanol

On coule un mélange contenant 20 g du composé préparé au stade précédent et 20 g de γ-butyrolactone dans 100 ml de benzène sur une suspension contenant 4,6 g d'hydrure de sodium (à 60%) dans 100 ml de benzène. Après la fin de la coulée, on abandonne une nuit à température ambiante puis, on hydrolyse avec 50 ml d'eau. Après décantation, la phase organique est lavée plusieurs fois à l'eau, séchée et concentrée pour obtenir le composé attendu.
Rendement : 69%
Point de fusion : 176°C

Spectre de résonance magnétique nucléaire du proton (solvant $CDCl_3$) :

2,7 ppm, 1H échangeable ; 2,95 ppm, t, 2H ; 3,95 ppm, q, 2H ; 7,2 ppm, dd, 1H ; 7,5 ppm, dd, 1H ; 7,6 ppm, s, 1H ; 7,65 à 7,8 ppm, m, 3H ; 7,95 ppm, dd, 1H ; 8,4 ppm, dd, 1H.

STADE D

1-chloro 2-[1,2-dihydro 2-oxo 1-(3-trifluorométhyl phényl) 1, 8-naphthyridin-3-yl] éthane

Ce composé a été préparé à partir du composé obtenu au stade précédent selon le procédé décrit dans l'exemple 7 stade B.
Rendement : 92%

STADE E

3,5 g du composé obtenu au stade D, 2,18 g du composé obtenu au stade F de l'exemple 1 et 1,73 ml de N,N-diisopropyl éthylamine en solution dans 70 ml de diméthylformamide sont portés à 60°C pendant 30 heures. On concentre ensuite le milieu réactionnel, on reprend par l'acétate d'éthyle et on extrait par l'acide chlorhydrique N. On filtre et on lave le précipité formé par l'éther éthylique. On recristallise dans le méthanol pour obtenir le composé attendu.
Rendement : 92%
Point de fusion : 143-147°C

Analyse élémentaire :

| | C% | H% | N% | Cl% |
|---|---|---|---|---|
| Théorie | 60,79 | 4,40 | 9,78 | 6,19 |
| Trouvé | 60,76 | 4,26 | 9,81 | 6,29 |

**EXEMPLE 10**

**3-{1-[(5-chloro benzocyclobutèn-1-yl) méthyl] pipérid-4-yl} 6-fluoro 1,2-benzisoxazole (R,S)**

Ce composé a été obtenu à partir du 5-chloro 1-iodométhyl benzocyclobutène (préparé à partir du 5-chloro 1-cyanométhyl benzocyclobutène décrit dans J.Org.Chem (1968) 33 (8) p. 3327) et du 6-fluoro 3-pipérid-4-yl 1,2-benzisoxazole selon le procédé décrit dans l'exemple 1, stade H, procédé 1.
Rendement : 8 %
Point de fusion : 115-116°C

Analyse élémentaire :

| | C% | H% | N% | Cl% |
|---|---|---|---|---|
| Théorie | 68,01 | 5,44 | 7,55 | 9,56 |
| Trouvé | 68,41 | 5,56 | 7,59 | 9,52 |

**EXEMPLE 11**

**Fumarate de 6-fluoro 3-{1-[(7-isopropyl 2,3-dihydrobenzofuran-2-yl) méthyl] pipérid-4-yl} 1,2-benzisoxazole (R,S)**

Ce composé a été obtenu sous forme de base, selon le procédé décrit dans l'exemple 5, mais en utilisant au stade A, le 2-isopropyl phénol au lieu du 2-méthoxyphénol.
Rendement : 12 % (stade final)

Pour obtenir le fumarate correspondant, la base est dissoute dans une quantité adéquate d'une solution éthanolique d'acide fumarique à 2 %. On glace et on filtre le précipité formé pour obtenir le sel attendu. Point de fusion : 206-208°C

Analyse élémentaire :

| | C% | H% | N% |
|---|---|---|---|
| Théorie | 65,87 | 6,12 | 5,49 |
| Trouvé | 65,70 | 6,00 | 5,23 |

**EXEMPLE 12**

**3-{1-[(7-fluoro 2,3-dihydrobenzofuran-2-yl) méthyl] pipérid-4-yl} 6-fluoro 1,2-benzisoxazole (R,S)**

Ce composé a été obtenu selon le procédé décrit dans l'exemple 5 mais en utilisant au stade A, le 2-fluorophénol au lieu du 2-méthoxyphénol.
Rendement : 15 % (stade final)
Point de fusion : 126-128°C

Analyse élémentaire :

| | C% | H% | N% |
|---|---|---|---|
| Théorie | 68,10 | 5,44 | 7,56 |
| Trouvé | 67,88 | 5,49 | 7,53 |

**EXEMPLE 13**

**Fumarate de 6-fluoro 3-{1-[(3,4-méthylènedioxybenzocyclobutèn-1-yl) méthyl] pipérid-4-yl} 1,2-benzisoxazole (R,S)**

STADE A

Acide 2-cyano 3-(2,3-méthylènedioxyphén-1-yl) acrylique

Mélanger 0,13 mole de 2,3-méthylènedioxybenzaldéhyde, 0,13 mole d'acide cyanoacétique et 0,022 mole d'acétate d'ammonium dans 18,6 ml de pyridine et 118 ml de benzène. Porter à reflux jusqu'à obtention d'un volume d'eau égal à 2,4 ml. Abandonner la nuit à température ambiante, filtrer le précipité formé et concentrer le filtrat.

Reprendre le précipité et le filtrat évaporé dans l'eau, acidifier avec de l'acide chlorhydrique à 18 %. Filtrer. Le produit obtenu est cristallisé dans l'acide acétique.
Rendement : 44 %
Point de fusion : 230°C (sublimation)

Spectre de résonance magnétique nucléaire du proton (solvant DMSO-$d_6$) :

3,5 ppm, s large, 1H ; 6,2 ppm, s, 2H ; 7,0 ppm, t, 1H ; 7,15 ppm, d, 1H ; 7,7 ppm, d, 1H ; 8,2 ppm, s, 1H.

STADE B

Acide 2-cyano 3-(2,3-méthylènedioxyphèn-1-yl) propionique

Mélanger 0,055 mole de l'acide obtenu au stade précédent dans 129 ml de méthanol et 43 ml d'une solution aqueuse saturée en bicarbonate de sodium. Ajouter à 18°C, 0,17 mole de borohydrure de sodium. Laisser ensuite revenir à température ambiante.

Concentrer le milieu réactionnel et acidifier (pH = 1) à l'aide d'acide chlorhydrique. Extraire à l'éther éthylique. Laver les phases organiques à l'eau jusqu'à neutralité. Sécher.
On obtient le composé attendu.
Rendement : 82 %
Point de fusion : 118°C

Spectre de résonance magnétique nucléaire du proton (solvant DMSO-$d_6$) :

3,1 ppm, dd, 1H ; 3,3 ppm, dd, 1H ; 3,85 ppm, dd, 1H ; 6 ppm, m, 2H ; 6,8 ppm, m, 3H ; 8,8 ppm, s large, 1H.

STADE C

1-cyano 2-(2,3-méthylèndioxyphèn-1-yl) éthane

Mélanger 0,046 mole de l'acide obtenu au stade B à 19 ml de diméthylformamide puis chauffer à 150°C pendant 2 heures. Reprendre par l'eau et extraire à l'éther éthylique. Laver les phases organiques avec une solution de bicarbonate de sodium, puis à l'eau ; sécher et évaporer le solvant pour obtenir un résidu huileux.
Rendement : 83 %

Spectre de résonance magnétique nucléaire du proton (solvant $CDCl_3$) :

2,65 ppm, t, 2H ; 2,95 ppm, t, 2H ; 5,95 ppm, s , 2H ; 6,65 - 6,9 ppm, m, 3H.

STADE D

1-cyano 2-(6-bromo 2,3-méthylèndioxyphényl) éthane

Dissoudre 0,31 mole du nitrile décrit au stade C dans 179 ml d'acide acétique. Couler sur cette solution goutte à goutte à 18°C, une solution de brome (0,32 mole dans 35 ml d'acide acétique). Abandonner la nuit à température ambiante. Hydrolyser par un mélange d'acétate de potassium, eau et glace. Extraire à l'éther éthylique puis, laver plusieurs fois la phase éthérée à l'eau.

Purifier le composé obtenu sur une colonne de silice, en utilisant comme éluant un mélange de cyclohexane et de dichlorométhane (20 : 80 V/V).
Rendement : 42 %

Spectre de résonance magnétique nucléaire du proton (solvant $CDCl_3$) :

2,65 ppm, t, 2H ; 3,05 ppm, t, 2H ; 6 ppm, s, 2H ; 6,65 ppm, d, 1H : 7 ppm, d, 1H.

STADE E

1-cyano 3,4-méthylèndioxybenzocyclobutane

On prépare l'amidure en introduisant 0,16 atome-gramme de sodium en morceaux dans 45 ml d'ammoniac liquide, contenant 0,12 g de ferricyanure de potassium hexahydraté et 0,12 g de nitrate ferrique.

On ajoute ensuite rapidement le composé obtenu au stade précédent, on laisse au contact, et on décompose en rajoutant lentement 6,9 g de chlorure d'ammonium. On abandonne la nuit, puis on extrait à l'éther éthylique et on lave plusieurs fois à l'eau.

Le composé ainsi obtenu est purifié sur colonne de silice en utilisant comme éluant un mélange de dichlorométhane et de cyclohexane (50 : 50 V/V).
Rendement : 49 %
Point de fusion : 85°C

Spectre de résonance magnétique nucléaire du proton (solvant $CDCl_3$) :

3,55 ppm, m, 2H ; 4,2 ppm, m, 1H ; 5,95 ppm, s, 2H ; 6,6 - 6,9 ppm, 2d, 2H.

STADE F

Acide 2-(2,3-méthylèndioxybenzocyclobutèn-1-yl) carboxylique

Mélanger 0,0335 mole du composé obtenu au stade E, dans un mélange de potasse alcoolique (6,71 g d'hydroxyde de potassium dans 47,6 ml d'éthanol) et laisser agiter la nuit à température ambiante. Ajouter ensuite 9 ml d'eau et amener à reflux pendant 4 heures. Concentrer.

Reprendre par l'eau, laver plusieurs fois à l'éther éthylique, acidifier la phase aqueuse avec de l'acide chlorhydrique, puis extraire à l'éther éthylique, pour obtenir le composé attendu.
Rendement : 98 %
Point de fusion : 125°C

Spectre de résonance magnétique nucléaire du proton (solvant $CDCl_3$) :

3,45 ppm, d, 2H ; 4,3 ppm, t, 1H ; 5,95 ppm, s, 2H ; 6,7 ppm, 2d, 2H.

STADE G

2-(2,3-méthylèndioxybenzocyclobutèn-1-yl) méthanol

Ce composé a été préparé à partir de l'acide obtenu ci-dessus et selon le procédé décrit dans l'exemple 2 stade A.
Rendement : 87 %

Spectre de résonance magnétique nucléaire du proton (solvant $CDCl_3$) :

1,5 ppm, 1H échangeable ; 2,9 ppm, dd, 1H ; 3,25 ppm, dd, 1H ; 3,65 ppm, m, 1H ; 3,9 ppm, m, 2H ; 5,9 ppm, s, 2H ; 6,65 ppm, 2d, 2H.

STADE H

para-toluènesulfonate de (2,3-méthylèndioxybenzocyclobutèn-1-yl) méthyle.

Ajouter à 0°C, 0,1 mole de chlorure de l'acide p-toluènesulfonique, sur 0,07 mole de l'alcool précédemment décrit, dissous dans 84 ml de pyridine. Abandonner à température ambiante. Concentrer et reprendre par l'eau. Filtrer le précipité formé, le laver à l'eau puis par l'acide chlorhydrique N.
Rendement : 82 %
Point de fusion : 102°C

Spectre de résonance magnétique nucléaire du proton (solvant DMSO-$d_6$) :

2,4 ppm, s, 3H ; 2,7-2,8 ppm, 2d, 1H ; 3,1-3,3 ppm, 2d, 1H ; 3,7 ppm, m, 1H ; 4,25 ppm, m, 2H ; 5,95 ppm, s, 2H ; 6,55 ppm, d, 1H ; 6,75 ppm, d, 1H ; 7,45 ppm, d, 2H ; 7,75 ppm, d, 2H.

STADE I

Mélanger 0,01 mole du composé obtenu au stade H avec 0,02 mole de 6-fluoro 3-pipérid-4-yl 1,2-benzisoxazole en solution dans 50 ml de toluène et amener à reflux pendant 12 heures. Reprendre par un mélange d'eau et d'éther éthylique. Relaver la phase éthérée plusieurs fois à l'eau.

Purifier le 6-fluoro 3-{1-[(3,4-méthylèndioxy benzocyclobutèn-1-yl) méthyl] pipérid-4-yl} 1,2-benzisoxazole (R,S) ainsi obtenu sous forme d'huile, sur colonne de silice en utilisant comme éluant un mélange de dichlorométhane et d'acétate d'éthyle (90 : 10 V/V).
Rendement : 37 %

Salifier ensuite avec une solution d'acide fumarique dans le méthanol pour obtenir le sel attendu.
Point de fusion : 185-188°C

Analyse élémentaire :

| | C% | H% | N% |
|---|---|---|---|
| Théorie | 62,90 | 5,08 | 5,64 |
| Trouvé | 62,85 | 4,94 | 5,45 |

**EXEMPLE 14**

**3-{1-[(1,2-dihydro 2-oxo 1-phényl 1,8-naphthyridin-3-yl) éthyl] pipérid-4-yl} 6-fluoro 1,2-benzisoxazole**

STADE A

Acide 2-phénylamino nicotinique

Mélanger dans 126 ml de xylène, 50 ml d'aniline distillée et 43,2 g d'acide 2-chloro nicotinique. Amener à reflux pendant 4 heures. Laisser refroidir, filtrer le précipité et le laver plusieurs fois à l'eau.
Rendement : 74 %
Point de fusion : 148°C

Spectre de résonance magnétique nucléaire du proton (solvant $CDCl_3$) :

6,8 ppm, dd, 1H ; 7,1 ppm, t, 1H ; 7,35 ppm, t, 2H ; 7,5 ppm, d, 2H ; 8,35 ppm, dd, 2H ; 9,8 et 10,6 ppm 2H échangeables.

STADE B

Le 3-{1-[(1,2-dihydro 2-oxo 1-phényl 1,8-naphthyridin-3-yl) éthyl] pipérid-4-yl} 6-fluoro 1,2-benzisoxazole (R,S) a été obtenu selon le procédé décrit dans l'exemple 9 (stades A à E) mais en utilisant au stade A l'acide 2-phénylamino nicotinique au lieu de l'acide niflumique.
Rendement : 14 % (stade final)
Point de fusion: 203-206°C

Analyse élémentaire :

| | C% | H% | N% |
|---|---|---|---|
| Théorie | 71,78 | 5,38 | 11,96 |
| Trouvé | 71,78 | 5,53 | 11,94 |

**EXEMPLE 15**

**3-{1-[(1,2-dihydro 2-oxo 1-(2-fluorophényl) 1,8-naphthyridin-3-yl) éthyl] pipérid-4-yl} 6-fluoro 1,2-benzisoxazole**

STADE A

Acide 2-[(2-fluorophényl) amino] nicotinique

Ce composé a été préparé selon le procédé décrit dans l'exemple 14 stade A mais en utilisant la 2-fluoroaniline au lieu de l'aniline.
Rendement : 80 %
Point de fusion 144°C

Spectre de résonance magnétique nucléaire du proton (solvant DMSO-$d_6$) :

6,8 à 7,4 ppm, m, 5H ; 8,1 à 8,6 ppm, m + échangeable, 2H+1H ; 10,65 ppm, 1H échangeable.

STADE B

2-[(2-fluorophényl) amino] pyrid-3-yl carbaldéhyde

Ce composé a été obtenu à partir de l'acide décrit au stade précédent et selon le procédé décrit dans l'exemple 9, stades A et B.
Rendement : 18 %
Point de fusion : 94°C

Spectre de résonance magnétique nucléaire du proton (solvant $CDCl_3$) :

6,9 ppm, dd, 1H ; 7,2-7 ppm, m, 3H ; 7,9 ppm, dd, 1H ; 8,45 ppm, dd, 1H ; 8,55 ppm, dd, 1H ; 9,95 ppm, s , 1H ; 10,6 ppm, s, 1H.

STADE C

3-[1-(6-fluoro 1,2-benzisoxazol-3-yl) pipérid-1-yl] propionate d'éthyle

Dissoudre 0,045 mole de 6-fluoro 3-pipérid-1-yl 1,2-benzisoxazole dans 15 ml d'éthanol. Couler ensuite 6,2 ml d'acrylate d'éthyle dissous dans 15 ml d'éthanol et laisser agiter la nuit. Concentrer, reprendre par l'eau et extraire à l'éther éthylique.
Rendement : 95 %
Point de fusion : 58-59°C

Spectre de résonance magnétique nucléaire du proton (solvant $CDCl_3$) :

1,3 ppm, t, 3H ; 2,3-2,0 ppm, m, 6H ; 2,55 ppm, t, 2H ; 2,75 ppm, t, 2H ; 3,05 ppm, m, 3H ; 4,15 ppm, q, 2H ; 7,05 ppm, dd, 1H ; 7,25 ppm, dd, 1H ; 7,7 ppm, dd ,1H.

STADE D

Couvrir de 20 ml de benzène 0,015 mole d'hydrure de sodium, 0,01 mole du composé obtenu au stade C et 0,01 mole du composé obtenu au stade B. Amorcer la réaction avant le début de la coulée avec quelques gouttes d'éthanol. Laisser agiter à température ambiante, filtrer après une nuit. Recristallisation dans le dichlorométhane.
Rendement : 11 %
Point de fusion : 255-259°C

Analyse élémentaire :

| | C% | H% | N% |
|---|---|---|---|
| Théorie | 68,64 | 4,69 | 11,86 |
| Trouvé | 68,50 | 4,62 | 11,84 |

**EXEMPLE 16**

**6-fluoro 3-[1-(indan-2-yl) pipérid-4-yl] 1,2-benzisoxazole**

Mélanger 0,173 mole de para-toluénesulfonate d'indan-2-yl (préparé à partir d'indan-2-ol selon le mode opératoire décrit dans l'exemple 4 stade B) et 0,173 mole de 6-fluoro 3-pipérid-4-yl 1,2-benzisoxazole en solution dans 50 ml de toluène et 0,0346 mole de triéthylamine.

Amener à reflux pendant 12 heures. Eliminer par filtration le trouble. Décanter le toluène et laver à l'eau. Purifier sur colonne de silice en utilisant comme solvant le dichlorométhane.
Rendement : 16 %

Point de fusion : 153-155°C

Analyse élémentaire :

| | C% | H% | N% |
|---|---|---|---|
| Théorie | 74,98 | 6,29 | 8,33 |
| Trouvé | 74,91 | 6,54 | 8,35 |

**EXEMPLE 17**

**Chlorhydrate de 3-{1-[(3-chloro benzocyclobutèn-1-yl) méthyl] pipérid-4-yl} 6-fluoro 1,2-benzisoxazole (R,S)**

STADE A

Acide (3-chloro benzocyclobutèn-1-yl) carboxylique

Ajouter 5,0 g de 3-chloro 1-cyano benzocyclobutane, (préparé selon le procédé décrit dans le brevet EP 119.107) à une solution de 6,0 g d'hydroxyde de potassium dans 58 ml d'éthanol. Agiter à température ambiante pendant une nuit. Puis, ajouter 7,5 ml d'eau et chauffer à reflux pendant 6 heures. Evaporer à sec l'éthanol. Reprendre à l'eau, laver à l'éther éthylique, puis acidifier (pH = 1) avec de l'acide chlorhydrique 1N et extraire au dichlorométhane. Sécher, filtrer et évaporer à sec.
Rendement : 95 %
Point de fusion : 106°C

Spectre de résonance magnétique nucléaire du proton (solvant $CDCl_3$) :

3,5 ppm, d, 2H ; 4,3 ppm, t, 1H ; 7,4 à 6,9 ppm, m, 3H.

STADE B

(3-chloro benzocyclobutèn-1-yl) méthanol

Ce composé a été préparé à partir de l'acide décrit dans le stade A et selon le procédé décrit dans l'exemple 2 stade A.
Rendement : 86 %

Spectre de résonance magnétique nucléaire du proton (solvant $CDCl_3$) :

1,8 ppm, m, 1H échangeable ; 2,9 ppm, m, 1H ; 3,3 ppm, m, 1H ; 4,1 à 3,5 ppm, m, 3H ; 7,3 à 6,9 ppm, m, 3H.

STADE C

3-chloro 1-iodométhyl benzocyclobutène

Ce composé a été obtenu à partir de l'alcool décrit au stade précédent et selon le procédé décrit dans l'exemple 4 stades B et C.
Rendement : 61 %

Spectre de résonance magnétique nucléaire du proton (solvant $CDCl_3$) :

2,85 ppm, dd, 1H ; 3,5 ppm, m, 3H ; 3,9 ppm, m, 1H ; 7,2 ppm, m, 3H.

STADE D

Le 3-{1-[(3-chloro benzocyclobutèn-1-yl) méthyl] pipérid-4-yl} 6-fluoro 1,2-benzisoxazole (R,S) a été préparé selon le procédé décrit dans l'exemple 1 stade H mais en remplaçant 1-iodométhyl benzocyclobutène par le 3-chloro 1-iodométhyl benzocyclobutène.
Rendement : 15 %

Le chlorhydrate correspondant a été obtenu après addition d'une quantité adéquate d'éthanol chlorhydrique et recristallisation dans l'acétate d'éthyle.
Point de fusion : 205-209°C

Analyse élémentaire :

| | C% | H% | N% | CL% |
|---|---|---|---|---|
| Théorie | 61,93 | 5,20 | 6,88 | 17,41 |
| Trouvé | 61,69 | 5,14 | 6,95 | 17,45 |

**EXEMPLE 18**

**6-fluoro 3-{1-[(2,3,6,7-tétrahydro benzo[1,2-b:4,5-b']difuran-2-yl) méthyl] pipérid-4-yl} 1,2-benzisoxazole (R,S)**

STADE A

Acide (6,7-dihydro benzo[1,2-b:4,5-b']difuran-2-yl) carboxylique

Mélanger à température ambiante 30,9 g de 2,3-dihydro 6-formyl 5-hydroxy benzofurane (préparé selon le procédé décrit dans J. Med. Chem. (1989) 32 p. 1006), 32,8 ml de diéthylbromomalonate, 25,35 g de carbonate de potassium dans 115 ml de méthyléthylcétone. Porter à reflux pendant 5 heures. Laisser refroidir, filtrer le solide et le rincer par 200 ml de méthyléthylcétone. Evaporer le solvant et le reprendre par 500 ml de dichlorométhane, laver par 200 ml d'une solution aqueuse saturée de chlorure de sodium et sécher sur sulfate de magnésium. Après filtration et évaporation, le produit obtenu est redissous dans 40 ml d'éthanol. Y ajouter une solution chaude de 21,9 g d'hydroxyde de potassium en solution dans 220 ml d'éthanol. Agiter à température ambiante, filtrer le solide et le redissoudre dans un minimum d'eau. Acidifier par 100 ml d'acide chlorhydrique 6N et maintenir la nuit sous agitation.

Evaporer l'éthanol et filtrer le précipité formé. Recristalliser dans le méthanol.
Rendement : 53 %

Spectre de résonance magnétique nucléaire du proton (solvant DMSO-$d_6$) :

3,3 ppm, t, 2H ; 4,6 ppm, t, 2H ; 7,0 ppm, s, 1H ; 7,6 et 7,5 ppm, s+s, 2H ; 13,5 ppm, 1H échangeable.

STADE B

Acide (2,3,6,7-tétrahydro benzo[1,2-b:4,5-b']difuran-2-yl) carboxylique

A l'aide d'un bec bunsen, faire fondre 11,1 g de sodium dans 160 ml de toluène. Couler alors goutte à goutte 540 g de mercure. Laisser reposer 2 heures à température ambiante, puis laisser décanter le toluène et sous courant d'azote l'amalgame obtenu.
Sur cet amalgame, couler 20,0 g de l'acide obtenu au stade A en solution dans 460 ml d'hydroxyde de sodium 0,3 N.

Maintenir la nuit sous agitation à température ambiante, décanter le mercure et acidifier à froid la phase aqueuse par 45 ml d'acide chlorhydrique concentré. Filtrer puis extraire 3 fois par 500 ml d'acétate d'éthyle. Les phases organiques réunies sont lavées par 200 ml d'une solution aqueuse saturée de chlorure de sodium

et séchées sur sulfate de magnésium. Après filtration et évaporation, récupérer l'acide attendu.
Rendement : 54 %
Point de fusion : 185°C

Spectre de résonance magnétique nucléaire du proton (solvant $CDCl_3$) :

3,1 ppm, t, 2H ; 3,2-3,6 ppm, m, 2H ; 4,5 ppm, t, 2H ; 5,15 ppm, dd, 1H ; 6,55 et 6,7 ppm, s+s, 2H.

STADE C

(2,3,6,7-tétrahydro benzo[1,2-b:4,5-b']difuran-2-yl) méthanol

Ce composé a été préparé selon le procédé décrit dans l'exemple 2 stade A à partir de l'acide obtenu ci-dessus.
Rendement : 85 %
Point de fusion : 103-105°C

Spectre de résonance magnétique nucléaire du proton (solvant $CDCl_3$) :

2,05 ppm, t, 1H échangeable ; 2,8 à 3 ppm, m, 1H ; 3 à 3,3 ppm, m+t, 1H+2H ; 3,7 ppm, m, 2H ; 4,5 ppm, t, 2H ; 4,85 ppm, m, 1H ; 6,6 ppm, s+s, 2H.

STADE D

para-toluènesulfonate de (2,3,6,7-tétrahydro benzo[1,2-b:4,5-b']difuran-2-yl) méthyle

Ce composé a été préparé à partir de l'alcool décrit au stade précédent et selon le procédé décrit dans l'exemple 4 stade B.
Rendement : 92 %
Point de fusion : 113°C

Spectre de résonance magnétique nucléaire du proton (solvant $CDCl_3$) :

2,45 ppm, s, 3H ; 2,85 ppm, m, 1H ; 3,1 ppm, t, 2H ; 3,2 ppm, m, 1H ; 4,15 ppm, d, 2H ; 4,5 ppm, t, 2H ; 4,90 ppm, m, 1H ; 6,50 ppm, s+s, 2H ; 7,30 ppm, d, 2H ; 7,75 ppm, d, 2H.

STADE E

Le 6-fluoro 3-{1-[(2,3,6,7-tétrahydro benzo[1,2-b:4,5-b'] difuran-2-yl) méthyl] pipérid-4-yl} 1,2-benzisoxazole (R,S) a été obtenu selon le procédé décrit dans l'exemple 13 stade I mais en utilisant le para-toluènesulfonate de (2,3,6,7-tétrahydro benzo[1,2-b:4,5-b'] difuran-2-yl) méthyle au lieu du para-toluènesulfonate de (2,3-méthylènedioxybenzocyclobutèn-1-yl) méthyle.
Rendement : 20 %
Point de fusion : 117-121°C

Analyse élémentaire :

| | C% | H% | N% |
|---|---|---|---|
| Théorie | 70,04 | 5,88 | 7,10 |
| Trouvé | 70,08 | 6,10 | 7,15 |

## EXEMPLE 19

**Fumarate de 6-fluoro 3-[1-(indan-1-yl méthyl) pipérid-4-yl] 1,2-benzisoxazole (R,S)**

STADE A

6-fluoro 3-[1-(indan-1-yl carbonyl) pipérid-4-yl] 1,2-benzisoxazole.

A 3,7 g d'acide indan-1-yl carboxylique (Synthésis (1987) 845) dissous dans 15 ml de dichlorométhane, sont ajoutés 4,2 g de carbonyl diimidazole. Après agitation pendant 1 heure et 30 minutes à température ambiante, sont ajoutés 5 g de 6-fluoro 3-pipérid-4-yl 1,2 benzisoxazole dissous dans 10 ml de dichlorméthane. On agite 48 heures à température ambiante, puis on concentre à siccité et on extrait à l'acétate d'éthyle. On lave à l'acide chlorhydrique, puis avec une solution de bicarbonate de sodium et enfin à l'eau. On sèche sur sulfate de magnésium et on concentre. L'huile obtenue est utilisée telle quelle.

Spectre de résonance magnétique nucléaire du proton (solvant $CDCl_3$) :

2,15 ppm, m, 2H ; 2,25 ppm, m, 2H ; 2,45 ppm, m, 2H ; 29 à 3,2 ppm, m+t, 3H ; 3,3 à 3,6 ppm, m, 2H ; 4,2 à 4,5 ppm, t+m, 2H ; 4,75 ppm, t, 1H ; 7 à 7,4 ppm, m+m+t , 6H ; 7,7 ppm, d, 1H.

STADE B

A la suspension maintenue à 0°C de 1,67 g d'hydrure de lithium et d'aluminium dans 60 ml de tétrahydrofurane, on coule en 15 minutes 8 g du composé décrit au stade A dans 120 ml de tétrahydrofurane, en veillant à ce que la température ne dépasse pas 5°C. On laisse remonter la température du milieu réactionnel à 20°C. On hydrolyse 40 minutes après la fin de la coulée, par 1,15 ml d'eau, 0,92 ml d'hydroxyde de sodium à 20 % et 4,2 ml d'eau. On filtre et on concentre.

L'huile obtenue est purifiée sur colonne de silice en utilisant comme éluant un mélange de dichlorométhane et d'acétate d'éthyle (95 : 5 V/V).

Rendement : 62%

Le fumarate de 6 fluoro 3-[1-(indan-1-yl méthyl) pipérid-4-yl] 1,2-benzisoxazole (R,S) est obtenu par salification de la base avec une solution éthanolique d'acide fumarique à 2 %.

Point de fusion : 192-196°C

Analyse élémentaire :

| | C% | H% | N% |
|---|---|---|---|
| Théorie | 66,94 | 5,83 | 6,00 |
| Trouvé | 66,97 | 5,91 | 5,90 |

## EXEMPLE 20

**Chlorhydrate de 3-{1-[(4,5-diméthoxy benzocyclobutèn-1-yl) méthyl] pipérid-4-yl} 1,2-benzisoxazole (R,S)**

STADE A

3-{1-[(4,5-diméthoxybenzocyclobuten-1-yl) carbonyl] pipérid-4-yl} 6-fluoro 1,2-benzisoxazole

Dissoudre 8,0 g d'acide (4,5-diméthoxy benzocyclobutèn-1-yl) carboxylique (préparé selon le procédé décrit dans Tetrahedron (1973) 29 p.73) dans 130 ml de tétrahydrofurane, ajouter 6,9 g de carbonyl diimidazole. Agiter à température ambiante pendant 2 heures jusqu'à la fin du dégagement gazeux. Puis, ajouter goutte à goutte, une solution de 8,5 g de 6-fluoro 3-pipérid-4-yl 1,2-benzisoxazole dans 100 ml de tétrahydrofurane. Agiter à température ambiante pendant une nuit. Le produit attendu précipite. Filtrer et sécher au dessicateur

sur pentoxyde de phosphore.
Rendement : 63 %
Point de fusion : 178°C

Spectre de résonance magnétique nucléaire du proton (solvant $CDCl_3$) :

1,8 à 2,3 ppm, m, 4H ; 2,95 ppm, m, 1H ; 3,2 à 3,5 ppm, 3m, 4H ; 3,85 ppm, s, 6H ; 4,15 ppm, m, 1H ; 4,4 ppm, t, 1H ; 4,65 ppm, m, 1H ; 6,65 et 6,80 ppm, 2s, 2H ; 7,05 ppm, td, 1H ; 7,3 ppm, dd, 1H ; 7,65 ppm, dd, 1H.

STADE B

Le chlorhydrate de 3-{1-[(4,5-diméthoxy benzocyclobutèn-1-yl) méthyl] pipérid-4-yl} 1,2-benzisoxazole (R,S) a été obtenu à partir du produit décrit ci-dessus et selon la méthode décrite dans l'exemple 19 stade B.

Pour la purification de la base, on a utilisé comme solvant d'élution un mélange de dichlorométhane et de méthanol (95 : 5 V/V).
Rendement : 52 %

La base a été salifiée avec une solution d'acide chlorhydrique dans l'éther éthylique.
Point de fusion : 259-268°C

Analyse élémentaire :

| | C% | H% | N% | Cl% |
|---|---|---|---|---|
| Théorie | 63,81 | 6,05 | 6,47 | 8,19 |
| Trouvé | 64,02 | 6,02 | 6,46 | 8,26 |

**EXEMPLE 21**

**3-{1-[(4-éthyl 5-méthoxy benzocyclobutèn-1-yl) méthyl] pipérid-4-yl} 6-fluoro 1,2-benzisoxazole (R,S)**

STADE A

Acide 2-cyano 3-(2,3-dihydrobenzofuran-5-yl) acrylique

On mélange 39,5 g de 2,3-dihydrobenzofuran-5-yl carbaldéhyde (préparée selon le procédé décrit dans J. Org. Chem. (1984), 49, p.409), 22,7 g d'acide 2-cyano acétique et 4,08 g d'acétate d'ammonium dans 37 ml de pyridine et 210 ml de benzène. On porte à reflux pendant 6 heures 30 minutes. On isole le précipité formé puis on le met en suspension dans 600 ml d'acide chlorhydrique 6 N. On filtre, on lave le solide isolé à l'eau et on sèche à l'air.
Rendement : 48 %
Point de fusion : 234°C

Spectre de résonance magnétique nucléaire du proton (solvant $CDCl_3$+DMSO-$d_6$) :

3,3 ppm, t, 2H ; 4,7 ppm, t, 2H ; 6,9 ppm, d, 1H ; 7,7 ppm, dd, 1H ; 8,05 ppm, s, 1H ; 8,15 ppm, s, 1H.

STADE B

Acide 2-cyano 3-(2,3-dihydrobenzofuran-5-yl) propionique

Ce produit a été préparé à partir du composé obtenu au stade A et selon le procédé décrit dans l'exemple 13 stade B.
Rendement : 89 %

Spectre de résonance magnétique nucléaire du proton (solvant $CDCl_3$) :

3,0-3,3 ppm, m, 4H ; 3,6 ppm, m, 1H ; 4,55 ppm, t, 2H ; 6,75 ppm, d, 1H ; 7,0 ppm, d, 1H ; 7,15 ppm, s, 1H ; 8,0 ppm, 1H échangeable

STADE C

5-(2-cyano éth-1-yl) 2,3-dihydrobenzofurane

Ce composé a été obtenu à partir de l'acide décrit au stade B et selon le procédé décrit dans l'exemple 13 stade C.
Rendement: 76 %
Point de fusion : 64°C

Spectre de résonance magnétique nucléaire du proton (solvant $CDCl_3$) :

2,85 ppm, t, 2H ; 3,2 ppm, t, 2H ; 4,55 ppm, t, 2H ; 6,7 ppm, d, 1H ; 6,9 ppm, d, 1H ; 7,1 ppm, s, 1H.

STADE D

7-bromo 5-(2-cyano éth-1-yl) 2,3-dihydrobenzofurane

Ce composé a été obtenu selon le procédé décrit dans l'exemple 13 stade D à partir du 5-(2-cyano éth-1-yl) 2,3-dihydrobenzofurane.
Rendement : 96 %
Point de fusion : 73-74°C

Spectre de résonance magnétique nucléaire du proton (solvant $CDCl_3$) :

2,6 ppm, t, 2H ; 2,9 ppm, t, 2H ; 3,25 ppm, t, 2H ; 4,6 ppm, t, 2H ; 7,0 ppm, s, 1H ; 7,1 ppm, s, 1H.

STADE E

2-bromo 4-(2-cyano éth-1-yl) 6-éthylényl phénol

On ajoute dans 600 ml d'ammoniac 300 mg de ferricyanure de potassium et quelques cristaux de nitrate ferrique et on agite pendant 15 minutes. On dissout 9,2 g de sodium par morceaux en 30-40 minutes. On agite environ 1 heure, puis on ajoute 25 g par fractions de produit obtenu au stade D en 20 minutes environ. On maintient 3 heures sous agitation, puis on ajoute 32 g de nitrate d'ammonium. On arrête l'agitation et on laisse l'ammoniac s'évaporer.
Sous atmosphère d'azote, on ajoute quelques ml de méthanol, puis 1,5 litre d'eau. La phase aqueuse est extraite 4 fois par 500 ml de dichlorométhane. Les phases organiques réunies, sont lavées par 500 ml d'acide chlorhydrique 1N et 500 ml d'eau, puis séchées sur sulfate de magnésium. Le produit brut obtenu après filtration et évaporation est recristallisé dans 185 ml d'éthanol.
Rendement : 56 %
Point de fusion : 128°C

Spectre de résonance magnétique nucléaire du proton (solvant $CDCl_3$) :

2,55 pppm, t, 2H ; 2,85 ppm, t, 2H ; 5,65 ppm, s 1H échangeable ; 5,35 ppm, dd, 1H ; 5,75 ppm, dd, 1H ; 6,8 à 7,1 ppm, dd, 1H ; 7,2 ppm, 2s, 2H.

STADE F

1-(3-bromo 4-méthoxy 5-éthylényl phèn-1-yl) 2-cyano éthane

A 13,4 g du phénol obtenu au stade E en solution dans la potasse, à 8-10°C, on ajoute goutte à goutte en 15 minutes sous agitation très énergique 5,05 ml de sulfate de diméthyle. On maintient 19 heures sous agitation à température ambiante et on extrait par 3 fois 100 ml d'acétate d'éthyle. Les phases organiques réunies sont lavées 2 fois par 50 ml d'hydroxyde de sodium 1 N puis par 50 ml d'eau et séchées sur sulfate de magnésium. Après filtration et évaporation, on obtient le produit attendu sous forme d'huile.
Rendement : 85 %

Spectre de résonance magnétique nucléaire du proton (solvant $CDCl_3$) :

2,6 ppm, t, 2H ; 2,9 ppm, t, 2H ; 3,8 ppm, s, 3H ; 5,4 ppm, d, 1H ; 5,8 ppm, d, 1H ; 7,0 ppm, dd, 1H ; 7,35 ppm, m, 2H.

STADE G

1-(3-bromo 4-méthoxy 5-éthyl phènyl) 2-cyano éthane

Le composé obtenu au stade F est hydrogéné à température ambiante, sous pression atmosphérique pendant 5 heures en présence de 220 mg d'oxyde de platine. On filtre le catalyseur, on rince par un peu d'acétonitrile. Après évaporation, on obtient le produit attendu sous forme d'huile.
Rendement : 95 %

Spectre de résonance magnétique nucléaire du proton (solvant $CDCl_3$) :

1,2 ppm, t, 3H ; 2,5 à 2,8 ppm, q+t, 2H+2H ; 2,9 ppm, t, 2H ; 3,75 ppm, s, 3H ; 7,0 ppm, d, 1H ; 7,25 ppm, d, 1H.

STADE H

3-{1-[(4-éthyl 5-méthoxybenzocyclobutèn-1-yl) carbonyl] pipérid-4-yl} 6-fluoro 1,2-benzisoxazole

Ce composé a été préparé à partir du 6-fluoro 3-pipérid-4-yl 1,2-benzisoxazole et de l'acide (4-éthyl 5-méthoxy benzocyclobutèn-1-yl) carboxylique, selon le procédé décrit dans l'exemple 20 stade A.
L'acide (4-éthyl 5-méthoxy benzocyclobutèn-1-yl) carboxylique a été préparé à partir du composé décrit au stade G et selon le procédé décrit dans l'exemple 13 stades E et F.
Rendement : 95 %

Spectre de résonance magnétique nucléaire du proton (solvant $CDCl_3$) :

1,15 ppm, t, 3H ; 1,8 à 2,3 ppm, m, 4H ; 2,6 ppm, q, 2H ; 3,0 ppm, m, 1H ; 3,3 à 3,5 ppm, m+m+m, 2H+1H+1H ; 3,75 ppm, s, 3H ; 4,15 ppm, m, 1H ; 4,4 ppm, t, 1H ; 4,65 ppm, m, 1H ; 6,7 ppm, s, 1H ; 6,85 ppm, s, 1H ; 7,05 ppm, td, 1H ; 7,25 ppm, dd, 1H ; 7,6 ppm, dd, 1H.

STADE I

Le 3-{1-[(4-éthyl 5-méthoxybenzocyclobutèn-1-yl) méthyl] pipérid-4-yl} 6-fluoro 1,2-benzisoxazole (R,S) a été obtenu selon le procédé décrit dans l'exemple 19 stade B à partir du composé décrit ci-dessus.
Rendement : 24 %
Point de fusion : 92-97°C

Analyse élémentaire :

| | C% | H% | N% |
|---|---|---|---|
| Théorie | 73,07 | 6,90 | 7,10 |
| Trouvé | 72,62 | 7,02 | 7,01 |

**EXEMPLE 22**

**Fumarate de 3-[1-(3-benzocyclobutèn-1-yl propyl) pipérid-4-yl] 6-fluoro 1,2-benzisoxazole (R,S)**

STADE A

2-(benzocyclobutèn-1-yl-méthyl) malonate de diéthyle

Préparer l'éthylate de sodium en dissolvant 0,07 mole de sodium dans 35,5 ml d'éthanol. Couler ensuite 0,073 mole de diéthyle malonate. Laisser agiter 1 heure à température ambiante puis ajouter, 0,069 mole de para-toluènesulfonate de benzocyclobutèn-1-yl méthyle. Abandonner 1 heure puis chauffer à reflux 14 heures. Concentrer, reprendre au dichlorométhane et laver plusieurs fois à l'eau. Purifier sur colonne de silice en utilisant comme éluant un mélange de cyclohexane et de dichlorométhane (95 : 5 V/V).
Rendement : 55 %

Spectre de résonance magnétique nucléaire du proton (solvant $CDCl_3$) :

1,2 à 1,45 ppm, m ; 6H ; 2,2 à 2,4 ppm, m, 2H ; 2,75 ppm, 2d, 1H ; 3,3 à 3,6 ppm, m, 3H ; 4,1 à 4,3 ppm, m, 4H ; 7,0 à 7,2 ppm, m, 4H.

STADE B

Acide 2-(benzocyclobutèn-1-yl méthyl) malonique

Dissoudre 0,16 mole d'hydroxyde de potassium dans 10 ml d'eau. Chauffer à 100°C puis couler en 1 heure, le diester décrit ci-dessus (0,041 mole), tout en distillant l'alcool formé. Continuer le chauffage pendant 3 heures. Laver la phase aqueuse plusieurs fois à l'ether éthylique. Acidifier avec l'acide chlorhydrique concentré. Extraire au dichlorométhane et sécher.
Rendement : 67 %
Point de fusion : 164-167°C

STADE C

Acide 3-benzocyclobutèn-1-yl propionique

Mélanger 0,027 mole du diacide obtenu au stade B dans 20 ml de NN-diméthylacétamide et chauffer à 125°C pendant 5 heures. Reprendre par l'eau, extraire à l'éther éthylique. Relaver la phase éthérée plusieurs fois à l'eau, et concentrer.
Rendement : 75 %
Point de fusion : < 50°C

STADE D

Le 3-[1-(3-benzocyclobutèn-1-yl propyl) pipérid-4-yl] 6-fluoro 1,2-benzisoxazole a été obtenu selon le procédé décrit dans l'exemple 19 (stades A et B) en utilisant comme acide, le composé décrit au stade C ci-dessus.
Rendement : 11 %

Le fumarate a été recristallisé dans l'éthanol.
Point de fusion : 171-174°C

Analyse élémentaire :

| | C% | H% | N% |
|---|---|---|---|
| Théorie | 67,49 | 6,08 | 5,83 |
| Trouvé | 67,00 | 6,10 | 5,59 |

## EXEMPLE 23

**Fumarate de 6-fluoro 3-[1-(indan-2-yl méthyl) pipérid-4-yl] 1,2-benzisoxazole**

Ce composé a été préparé à partir de l'acide indan-2-yl carboxylique et du 6-fluoro 3-pipérid-4-yl 1,2-benzisoxazole, selon le procédé décrit dans l'exemple 19 stades A et B.
Rendement : 13 %

Le sel a été obtenu après addition d'une quantité adéquate d'acide fumarique en solution dans l'éthanol.
Point de fusion : 211-216°C

Analyse élémentaire :

| | C% | H% | N% |
|---|---|---|---|
| Théorie | 66,94 | 5,83 | 6,00 |
| Trouvé | 66,72 | 5,84 | 5,91 |

## EXEMPLE 24

**3-[1-(3-fluoro benzocyclobutèn-1-yl méthyl) pipérid-4-yl] 6-fluoro 1,2-benzisoxazole (R,S)**

Ce composé a été obtenu selon le procédé décrit dans l'exemple 19 stades A et B mais en utilisant l'acide (3-fluoro benzocyclobutèn-1-yl) carboxylique au lieu de l'acide indan-1-yl carboxylique.

L'acide (3-fluoro benzocyclobutèn-1-yl) carboxylique a été préparé à partir de la 3-fluoro benzaldéhyde selon le procédé décrit dans l'exemple 13 stades A-F.
Rendement : 63 % (stade final)
Point de fusion : 81-83°C

Analyse élémentaire :

| | C% | H% | N% |
|---|---|---|---|
| Théorie | 71,17 | 5,69 | 7,90 |
| Trouvé | 71,16 | 5,80 | 7,73 |

## EXEMPLE 25

**6-fluoro 3-{1-[(5-méthoxy benzocyclobutèn-1-yl) méthyl] pipérid-4-yl} 1,2-benzisoxazole (R,S)**

Ce composé a été obtenu selon le procédé décrit dans l'exemple 19 stades A et B. L'acide utilisé pour sa préparation est l'acide (5-méthoxy benzocyclobutèn-1-yl) carboxylique.

Ce dernier composé a été synthétisé selon le mode opératoire décrit dans Tetrahedron (1974) 30 p 1053 et dans l'exemple 13 stade F.
Rendement : 33 % (stade final)
Point de fusion : 115-116°C

Analyse élémentaire

| | C% | H% | N% |
|---|---|---|---|
| Théorie | 72,11 | 6,33 | 7,64 |
| Trouvé | 71,89 | 6,40 | 7,66 |

EXEMPLE 26

6-fluoro 3-{1-[(4-méthoxy benzocyclobutèn-1-yl) méthyl] pipérid-4-yl} 1,2-benzisoxazole (R,S)

Ce composé a été obtenu selon le procédé décrit dans l'exemple 19 stades A et B, en utilisant au stade A comme acide, l'acide (4-méthoxy benzocyclobutèn-1-yl) carboxylique.

Ce composé a été préparé à partir du 1-cyano 4-méthoxy benzocyclobutène (J. Am. Chem. Soc (1976), 98 (11), p 3378) selon le procédé décrit dans l'exemple 13 stade F.

Rendement : 22 % (stade final)

Point de fusion: 97-99°C

Analyse élémentaire

| | C% | H% | N% |
|---|---|---|---|
| Théorie | 72,11 | 6,33 | 7,64 |
| Trouvé | 71,85 | 6,34 | 7,56 |

EXEMPLE 27

Chlorhydrate de 3-[1-(benzocyclobutèn-1-yl) méthyl] pipérid-4-yl} 1,2-benzisoxazole (R,S)

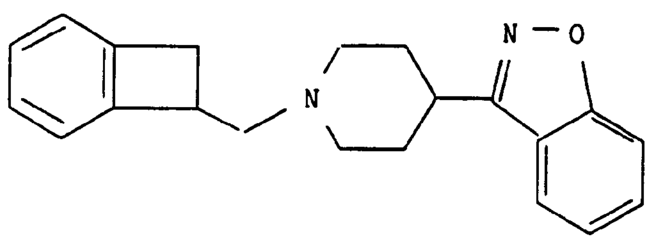

STADE A

2-fluorobenzoyl 1-méthyl pipéridine

Préparer le magnésien à partir de 0,067 mole de 4-chloro 1-méthyl pipéridine et de 0,055 atome-gramme de magnésium dans le tétrahydrofurane. Amorcer avec quelques gouttes de bromoéthane.

Couler ensuite 0,07 mole de 2-fluorobenzonitrile sur le magnésien. Amener à reflux 2 heures, puis laisser une nuit à température ambiante. Hydrolyser sur une solution de 15,3 g de chlorure d'ammonium, 45 g de glace et 50 ml d'eau. Amener à reflux pendant 3 heures. Laisser refroidir. Extraire plusieurs fois à l'éther éthylique. Sécher l'huile obtenue.

Rendement : 50 %

Spectre de résonance magnétique nucléaire du proton (solvant $CDCl_3$) :

1,7-2,15 ppm, m, 6H ; 2,25 ppm, s, 3H ; 2,85 ppm, m, 2H ; 3,1 ppm, m, 1H ; 7,0-7,3 ppm, m, 4H.

STADE B

1-éthoxycarbonyl 4-(2-fluorobenzoyl) pipéridine

A une solution de 0,19 mole du composé obtenu au stade précédent en solution dans 340 ml de toluène, ajouter goutte à goutte 37 ml de chloroformiate d'éthyle. Chauffer 8 heures à 85°C puis ajouter 10 ml de chloroformiate d'éthyle. Remettre à chauffer 4 heures. Laver le milieu réactionnel à l'eau, puis à l'acide chlorhydrique.
Rendement : 54 %

Spectre de résonance magnétique nucléaire du proton (solvant $CDCl_3$) :

1,05-1,15 ppm, m, 4H ; 1,25 ppm, t, 3H ; 2,95 ppm, m, 2H ; 3,3 ppm, m, 1H 3,4 ppm, m, 1H ; 3,75 ppm, m, 1H ; 4,15 ppm, q, 2H ; 7,05-7,3 ppm, m, 2H ; 7,55 ppm, m, 1H ; 7,8 ppm, dd, 1H.

STADE C

3-pipérid-4-yl 1,2-benzisoxazole

Ce produit a été obtenu à partir du composé décrit dans l'exemple B selon le procédé décrit dans l'exemple 1 stades D-F.
Rendement : 30 %

Spectre de résonance magnétique nucléaire du proton (solvant $CDCl_3$) :

1,7 ppm, 1H échangeable ; 1,8-2,2 ppm, m, 4H ; 2,85 ppm, td, 2H ; 3,15-3,4 ppm, m, 3H ; 7,25 ppm, td, 1H ; 7,5 ppm, m, 2H ; 7,75 ppm, d, 1H.

STADE D

Le 3-[1-(benzocyclobutèn-1-yl méthyl) pipérid-4-yl] 1,2-benzisoxazole a été préparé à partir de l'acide benzocyclobutèn-1-yl carboxylique et le 3-pipérid-4-yl 1,2-benzisoxazole selon le procédé décrit dans l'exemple 19 stades A et B.

Le chlorhydrate a été obtenu après salification par l'éther chlorhydrique.
Rendement : 23 %
Point de fusion : 208-212°C

Analyse élémentaire

| | C% | H% | N% | Cl% |
|---|---|---|---|---|
| Théorie | 71,08 | 6,53 | 7,89 | 9,99 |
| Trouvé | 70,70 | 6,51 | 7,72 | 10,28 |

Spectre de résonance magnétique nucléaire du proton (solvant DMSO-$d_6$) :

2,1-2,5 ppm, m, 4H ; 3-3,85 ppm, m, 9H ; 4,05 ppm, m, 1H ; 7,1-7,3 ppm, m, 4H ; 7,45 ppm, td, 1H ; 7,7 ppm, td, 1H ; 7,75 ppm, d, 1H ; 8,2 ppm, d, 1H 11-11,4 ppm, m échangeable, 1H.

**EXEMPLE 28**

**(-) 3-[1-(benzocyclobutèn-1-yl méthyl) pipérid-4-yl] 6-fluoro 1,2-benzisoxazole**

Le composé de l'exemple 1 est dissous dans l'eau alcalinisée par l'hydroxyde de sodium. La solution aqueuse est extraite par l'éther. Après décantation, la phase organique est séchée sur sulfate de magnésium anhydre évaporée à sec pour obtenir le 3-[1-(benzocyclobutèn-1-yl méthyl) pipérid-4-yl) 6-fluoro 1,2-benzisoxazole (R,S).

A 8,8 g de 3-[1-(benzocyclobutèn-1-yl méthyl) pipérid-4-yl] 6-fluoro 1,2-benzisoxazole (R,S) sont ajoutés 6,54 g d'acide (+) camphosulfonique dissous dans 327 ml d'éthanol. On laisse la solution pendant une nuit à température ambiante, puis on isole le précipité formé. On recristallise dans 230 ml d'éthanol, on alcalinise avec l'hydroxyde de sodium, extrait par l'éther. La phase organique est séchée sur sulfate de magnésium anhydre et évaporée à sec pour obtenir le (-) 3-[1(benzocyclobutèn-1-yl méthyl) pipérid-4-yl] 6-fluoro 1,2-benzisoxazole.
Point de fusion : 78-82°C

Spectre de résonance magnétique nucléaire du proton (solvant $CDCl_3$) :

2 à 2,4 ppm, m+m, 4H+2H ; 2,65 ppm, dd, 1H ; 2,75 ppm, m, 2H ; 3 à 3,2 ppm, m+m, 1H+2H ; 3,4 ppm, dd, 1H ; 3,7 ppm, m, 1H ; 6,9 à 7,3 ppm, m, 6H 7,7 ppm, dd, 1H.

Pouvoir rotatoire (C=1% dans le $CHCl_3$) :

| λ nm | $[\alpha]_D^{20°C}$ |
|---|---|
| 589 | - 9,1° |
| 578 | - 9,45° |
| 546 | - 10,75° |
| 436 | - 18,8° |

**EXEMPLE 29**

**Fumarate de (-) 3-[1-(benzocyclobutèn-1-yl méthyl) pipérid-4-yl] 6-fluoro 1,2-benzisoxazole**

A 2,5 g de base optiquement active, obtenue dans l'exemple 28, sont ajoutés 45 ml d'une solution d'acide fumarique 0,072M dans l'éthanol. On laisse précipiter une nuit pour obtenir le fumarate de (-) 3-[1-(benzocyclobutén-1-yl méthyl) pipérid-4-yl] 6-fluoro 1,2-benzisoxazole.
Point de fusion : 156-160°C.

Spectre de résonance magnétique nucléaire du proton (solvant $CDCl_3$) :

1,9 à 2,1 ppm, m, 4H ; 2,6 ppm, m, 2H ; 2,7 à 3,0 ppm, m, 3H ; 3,1 à 3,5 ppm, m, 4H ; 3,8 ppm, m, 1H ; 6,6 ppm, s, 2H ; 7,0 à 7,2 ppm, m, 4H ; 7,3 ppm, m, 1H ; 7,7 ppm, dd, 1H ; 8,05 ppm, dd, 1H.

Pouvor rotatoire (C=1% dans le DMSO) :

| λ nm | $[\alpha]_D^{25°C}$ |
|---|---|
| 589 | - 5,85° |
| 578 | - 6,40° |
| 546 | - 7,35° |
| 436 | - 12,1° |

**EXEMPLE 30**

**(+) 3-[1-(benzocyclobutèn-1-yl méthyl) pipérid-4-yl] 6-fluoro 1,2-benzisoxazole**

Ce composé a été obtenu selon le procédé décrit dans l'exemple 28 mais en utilisant l'acide (-) camphosulphonique.
Point de fusion : Huile

Pouvoir rotatoire (C=1% dans le $CHCl_3$) :

| λ nm | $[\alpha]_D^{22°C}$ |
|---|---|
| 589 | + 9,35° |
| 578 | + 9,85° |
| 546 | + 11,40° |
| 436 | + 20,95° |

**EXEMPLE 31**

**Fumarate de (+) 3-[1-(benzocyclobutèn-1-yl méthyl) pipérid-4-yl] 6-fluoro 1,2-benzisoxazole**

Ce composé a été préparé à partir du (+) 3-[1-(benzocyclobutèn-1-yl méthyl) pipérid-4-yl] 6-fluoro 1,2-benzisoxazole selon le procédé décrit dans l'exemple 29.

Pouvoir rotatoire (C=1% dans le DMSO) :

| λ nm | $[\alpha]_D^{25°C}$ |
|---|---|
| 589 | + 6,1° |
| 578 | + 6,4° |
| 546 | + 7,25° |
| 436 | + 12,7° |

**EXEMPLE 32**

**6-fluoro 3-{1-[(4,5-méthylènedioxy benzocyclobutèn-1-yl) méthyl] pipérid-4-yl} 1,2-benzisoxazole (R,S)**

STADE A

6-fluoro 3-{1-[(4,5-méthylènedioxy benzocyclobutèn-1-yl) carbonyl] pipérid-4-yl} 1,2-benzisoxazole

Ce composé a été préparé selon le procédé décrit dans l'exemple 19 stade A, mais en utilisant comme acide, l'acide (4,5-méthylènedioxy benzocyclobutèn-1-yl) carboxylique. Ce dernier produit a été obtenu à partir du 3,4-méthylènedioxy benzaldéhyde en utilisant le protocole décrit dans l'exemple 13 stades A-F.
Rendement : 30 %
Point de fusion : 228-230°C

Spectre de résonance magnétique nucléaire du proton (solvant $CDCl_3$) :

1,8-2,3 ppm, m, 4H ; 3 ppm, t 1H ; 3,2 à 3,5 ppm, 3m, 4H ; 4,2 ppm, d, 1H 4,35 ppm, d, 1H ; 4,65 ppm, d, 1H ; 5,9 ppm, 2s, 2H ; 6,65 et 6,75 ppm, 2s, 2H ; 7,1 ppm, td, 1H ; 7,25 ppm, dd, 1H ; 7,65 ppm, dd, 1H.

STADE B

A partir du composé décrit ci-dessus et en utilisant le protocole décrit dans l'exemple 19 stade B, on obtient le 6-fluoro 3-{1-[(4,5-méthylènedioxy benzocyclobutèn-1-yl) méthyl] pipérid-4-yl} 1,2-benzisoxazole (R,S).
Point de fusion : 125-126°C

**EXEMPLE 33**

**6-fluoro 3-{1-[(4,5-éthylènedioxy benzocyclobutèn-1-yl) méthyl] pipérid-1-yl} 1,2-benzisoxazole (R,S)**

STADE A

3-{1-[(4,5-éthylènedioxy benzocyclobutèn-1-yl) carbonyl] pipérid-4-yl} 6-fluoro 1,2-benzisoxazole

Ce composé a été préparé selon le procédé décrit dans l'exemple 32 stade A. L'acide utilisé est l'acide (4,5-éthylènedioxy benzocyclobutèn-1-yl) carboxylique.
Rendement : 48 %

Spectre de résonance magnétique nucléaire du proton (solvant $CDCl_3$) :

1,8 à 2,3 pmm, m, 4H ; 3 ppm, t, 1H ; 3,2 à 3,6 ppm, 3m, 4H ; 4,15 ppm, m, 1H ; 4,2 ppm, s, 4H ; 4,4 ppm, d,

1H ; 4,65 ppm, d, H ; 6,6 ppm, s, 1H ; 6,7 ppm, s, 1H ; 7,05 ppm td, 1H ; 7,25 ppm, m, 1H ; 7,65 ppm, dd, 1H.

STADE B

A partir du composé décrit ci-dessus et en utilisant le protocole décrit dans l'exemple 19 stade B, on obtient le 6-fluoro 3-{1-[(4,5-éthylènedioxy benzocyclobutèn-1-yl) méthyl] pipérid-4-yl} 1,2-benzisoxazole (R,S).

**EXEMPLE 34**

**6-fluoro 3-{1-[(4,5,6-triméthoxy benzocyclobutèn-1-yl) méthyl] pipérid-4-yl} 1,2-benzisoxazole (R,S)**

STADE A

6-fluoro 3-{1-[(4,5,6-triméthoxy benzocyclobutèn-1-yl) carbonyl] pipérid-4-yl} 1,2-benzisoxazole

Ce composé a éé préparé en utilisant le protocole décrit dans l'exemple 32 stade A. L'acide utilisé est l'acide (4,5,6-triméthoxy benzocyclobutèn-1-yl) carboxylique.
Rendement : 26 %

Spectre de résonance magnétique nucléaire du proton (solvant $CDCl_3$) :

2,3 à 1,8 ppm, m, 4H ; 3 ppm, m, 1H ; 3,2 à 4 ppm, m+m+m, 4H ; 3,9-3,8-3,65 ppm, 3s, 9H ; 4,15 ppm, m, 1H ; 4,4 ppm, m, 1H ; 4,6 ppm, m, 1H ; 6,5 ppm, s, 1H ; 7,1 ppm, m, 1H ; 7,65 ppm, m, 1H ; 7,75 ppm, m, 1H.

STADE B

Le 6-fluoro 3-{1-[(4,5,6-triméthoxy benzocyclobutèn-1-yl) méthyl] pipérid-4-yl} 1,2-benzisoxazole (R,S) est obtenu à partir du composé décrit ci-dessus et en utilisant le procédé décrit dans l'exemple 19 stade B.
Point de fusion : 141-143°C

TABLEAU I

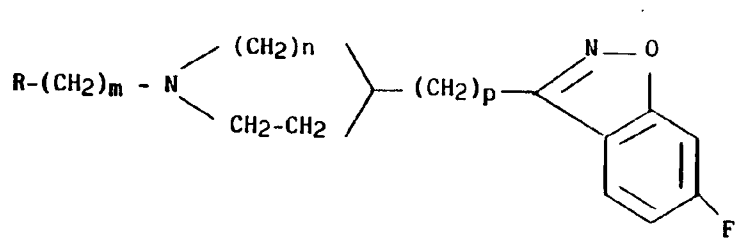

| EX. | R | m | n | p | R.M.N. (Solvant) |
|---|---|---|---|---|---|
| 1 | 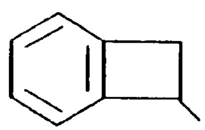 | 1 | 2 | 0 | ($CD_3OD$) (iodhydrate) 2,1 à 2,6 ppm, m, 4H ; 3,0 à 4,0 ppm, m, 9H ; 4 ppm, m, 1H 7,0 à 7,35 ppm, m, 5H ; 7,4 ppm, dd, 1H ; 8 ppm, m, 1H. |
| 2 | 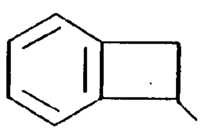 | 2 | 2 | 0 | (DMSO-$d_6$) (fumarate) 1,2 à 1,7 ppm, m, 6H ; 2,2 à 2,8 ppm, m+m+m, 1H+2H+2H ; 3,0 à 3,5 ppm, m, 4H+2H échangeables ; 3,4 ppm, m, 1H ; 6,55 ppm, d, 2H ; 7,0 à 7,3 ppm, m, 4H ; 7,2 ppm, t, 1H ; 7,7 ppm, dd, 1H ; 8,05 ppm, dd, 1H. |

TABLEAU I (SUITE 1)

| EX. | R | m | n | p | R.M.N. (Solvant) |
|---|---|---|---|---|---|
| 3 | 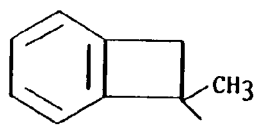 | 1 | 2 | 0 | ($CDCl_3$) (fumarate)<br>1,85 ppm, s, 3H ; 2,0 à 2,3 ppm, m, 4H ; 2,7 à 4,0 ppm, m,9H ; 7,0 à 7,3 ppm, m, 5H ; 7,6 ppm, m, 1H ; 8,55 ppm, m, 1H ; 12,1 ppm, 1H échangeable ; 12,7 ppm, 1H échangeable |
| 4 | 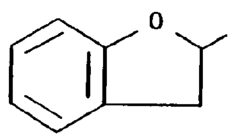 | 1 | 2 | 0 | ($CDCl_3$) (base)<br>2,1 ppm, m, 4H ; 2,4 ppm, m, 2H ; 2,6 à 3,5 ppm, m, 7H ; 5,1 ppm, m, 1H ; 6,4 à 7,3 ppm, m, 4H+2H ; 7,75 ppm, dd, 1H |

TABLEAU I (SUITE 2)

| EX. | R | m | n | p | R.M.N. (Solvant) |
|---|---|---|---|---|---|
| 5 | 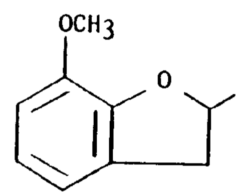 | 1 | 2 | 0 | $(CDCl_3)$ (base)<br>1,95 à 2,25 ppm, m, 4H ; 2,4 ppm, m, 2H ; 2,7 ppm, dd, 1H ; 2,9 ppm, dd, 1H ; 2,95 à 3,25 ppm, m, 4H ; 3,35 ppm, dd, 1H 3,9 ppm, s, 3H ; 5,05 ppm, m, 1H ; 6,7 à 6,85 ppm, m, 3H ; 7,05 ppm, td, 1H ; 7,2 ppm, dd, 1H ; 7,7 ppm, dd, 1H |
| 6 | 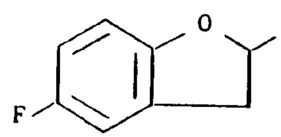 | 1 | 2 | 0 | $(CDCl_3)$ (base)<br>2,0 à 2,5 ppm, m, 6H ; 2,6 à 3,4 ppm, m, 7H ; 5,0 ppm, m, 1H ; 6,6 à 6,95 ppm, m, 3H ; 7,05 ppm, td, 1H ; 7,25 ppm, dd, 1H ; 7,5 ppm, dd, 1H |

T A B L E A U I (SUITE 3)

| EX. | R | m | n | p | R.M.N. (Solvant) |
|---|---|---|---|---|---|
| 7 | 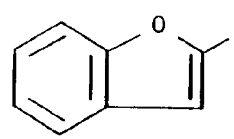 | 1 | 2 | 0 | **(DMSO-d6) (chlorhydrate)** 2,1 à 2,5 ppm, m, 4H ; 3,25 ppm, m, 2H ; 3,5 ppm, m, 1H ; 3,65 ppm, m, 2H ; 4,65 ppm, s, 2H ; 7,2 à 7,5 ppm, m, 4H ; 7,7 ppm, m, 3H ; 8,2 ppm, dd, 1H ; 11,7 ppm, 1H échangeable |
| 8 | 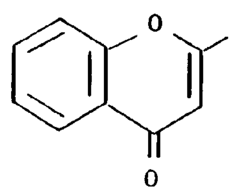 | 1 | 2 | 0 | **(DMSO-d6) (chlorhydrate)** 2,0 à 2,6 ppm, m, 4H ; 3,0 à 3,2 ppm, m, 5H ; 4,5 ppm, s, 2H 6,8 ppm, s, 1H ; 7,35 ppm, m, 1H ; 7,55 ppm, t, 1H ; 7,75 ppm, m, 2H ; 7,9 ppm, m, 1H ; 8,10 ppm, m, 1H ; 8,25 ppm, m, 1H |

TABLEAU I (SUITE 4)

| EX. | R | m | n | p | R.M.N. (Solvant) |
| --- | --- | --- | --- | --- | --- |
| 9 | 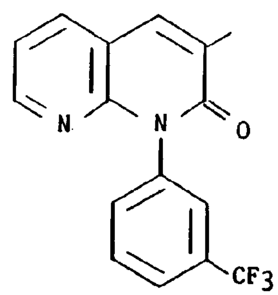 | 2 | 2 | 0 | (DMSO-d6) (chlorhydrate)<br>2,1 à 2,6 ppm, m, 4H ; 3,05 à 3,8 ppm, m, 9H ; 7,3 ppm, m, 2H ; 7,45 à 7,9 ppm, m, 5H ; 8,05 à 8,3 ppm, m, 3H ; 8,4 ppm, dd, 1H; 11,05 ppm, 1H échangeable |
| 10 | 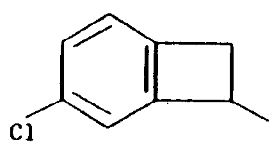 | 1 | 2 | 0 | ($CDCl_3$) (base)<br>2 à 2,4 ppm, m+m, 6H ; 2,6 à 2,9 ppm, m+m, 3H ; 3,1 ppm, m+m, 3H ; 3,3 ppm, dd, 1H ; 3,65 ppm, m, 1H ; 6,9 à 7,3 ppm, m+m, 5H ; 7,7 ppm, dd, 1H |

TABLEAU I (SUITE 5)

| EX. | R | m | n | p | R.M.N. (solvant) |
|---|---|---|---|---|---|
| 11 | 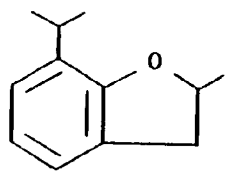 | 1 | 2 | 0 | ($DMSO-d_6$) (fumarate)<br>1,2 ppm, dd, 6H ; 1,7 à 2,2 ppm, m, 4H ; 2,4 ppm, m, 1H ; 2,60 à 2,85 ppm, m, 2H ; 2,85 à 3,55 ppm, m, 7H ; 5,0 ppm, m, 1H ; 6,6 ppm, s, 2H ; 6,75 ppm, t, 1H ; 6,95 à 7 ppm, 2d, 2H ; 7,25 ppm, td, 1H ; 7,7 ppm, dd, 1H ; 8,0 ppm, dd, 1H |
| 12 | 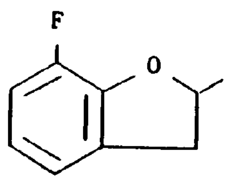 | 1 | 2 | 0 | ($CDCl_3$) (base)<br>2 ppm, m, 4H ; 2,5 à 2,25 ppm, m, 2H ; 3,0 à 2,5 ppm, m, 2H 3,0 à 3,5 ppm, m, 5H ; 5,1 ppm, m, 1H ; 6,5 à 7,1 ppm, m, 4H 7,2 ppm, m, 1H ; 7,7 ppm, dd, 1H |
| 13 | 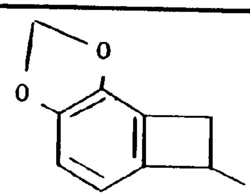 | 1 | 2 | 0 | ($DMSO-d_6$) (fumarate)<br>2,1-2,6 ppm, m, 4H ; 3-4 ppm, m, 9H ; 4 ppm, m, 1H ; 6,0 ppm, d, 2H ; 6,6 ppm, s, 2H ; 6,8 ppm, (syst. AB), 2H ; 7,35 ppm, td, 1H ; 7,6 ppm, dd, 1H ; 8,05 ppm, dd, 1H |

TABLEAU I (SUITE 6)

| EX. | R | m | n | p | R.M.N. (solvant) |
|---|---|---|---|---|---|
| 14 | 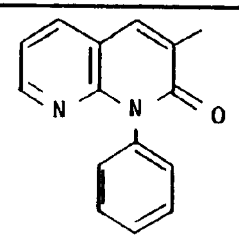 | 2 | 2 | 0 | ($CDCl_3$) (base)<br>2,05 ppm, m, 4H ; 2,25 ppm, m, 2H ; 2,7-3,0 ppm, m, 4H ; 3,10 ppm, m, 1H ; 3,20 ppm, m, 2H ; 7,0-7,3 ppm, m, 5H ; 7,4-7,75 ppm, m, 5H ; 7,9 ppm, dd, 1H ; 8,4 ppm, dd, 1H |
| 15 | 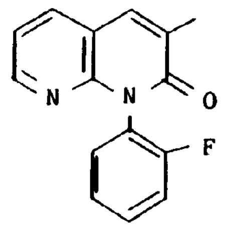 | 1 | 2 | 0 | ($CDCl_3$) (base)<br>2-2,3 ppm, m, 4H ; 2,4 ppm, m, 2H ; 3,1 ppm, m+m, 1H+2H ; 3,65 ppm, s, 2H ; 6,9-7,4 ppm, m+m+td, 2H+3H+1H ; 7,5 ppm, m, 1H ; 7,7 ppm, dd, 1H ; 7,9 ppm, s, 1H ; 7,9 ppm, dd, 2H 8,4 ppm, dd, 1H |
| 16 | 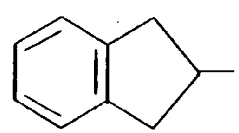 | 0 | 2 | 0 | ($CDCl_3$) (base)<br>2-2,4 ppm, m, 6H ; 2,8 à 3,4 ppm, m, 8H ; 6,9 à 7,3 ppm, m+m, 2H+4H ; 7,7 ppm, dd, 1H |

TABLEAU I (SUITE 7)

| EX. | R | m | n | p | R.M.N. (solvant) |
|---|---|---|---|---|---|
| 17 | 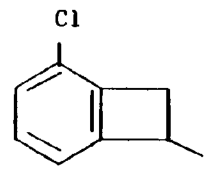 | 1 | 2 | 0 | ($CDCl_3$) (chlorhydrate) 2,1 à 2,5 ppm, m, 4H ; 3,0 à 3,8 ppm, 5m, 9H ; 4,05 ppm, m, 1H ; 7,1 à 7,4 ppm, 2m, 4H ; 7,75 ppm, dd, 1H ; 8,25 ppm, dd, 1H ; 11,15 ppm, m, 1H échangeable |
| 18 | 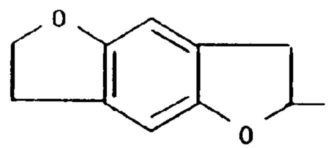 | 1 | 2 | 0 | ($CDCl_3$) (base) 1,9 à 2,3 ppm, m, 4H ; 2,3 ppm, m, 2H ; 2,6 ppm, dd, 1H ; 2,7 à 3,3 ppm, m+t+dd+m+m, 1H+2H+1H+2H+2H ; 4,5 ppm, t, 2H 4,95 ppm, m, 1H ; 6,6 ppm, s+s, 2H ; 7,05 ppm, td, 1H ; 7,2 ppm, dd, 1H ; 7,7 ppm, dd, 1H |
| 19 | 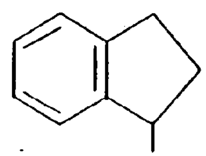 | 1 | 2 | 0 | (DMSO-$d_6$) (fumarate) 2,5 à 1,7 ppm, m, 8H ; 2,6 ppm, m, 1H ; 2,85 ppm, m, 3H ; 3,25 ppm, m, 3H ; 3,45 ppm, m, 1H ; 5 à 9 ppm, s, 1H ; 6,6 ppm, s, 2H ; 7,1 à 7,4 ppm, m, 5H ; 7,7 ppm, d, 1H ; 8,05 ppm, d, 1H |

TABLEAU I (SUITE 8)

| EX. | R | m | n | p | R.M.N. (solvant) |
|---|---|---|---|---|---|
| 20 | 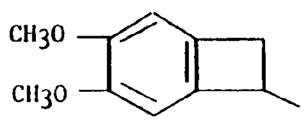 | 1 | 2 | 0 | **(DMSO-d6) (fumarate)** 2,2 ppm, m, 2H ; 2,5 ppm, m, 2H ; 2,9 à 3,8 ppm, 4m, 9H ; 3,75 ppm, s, 6H ; 3,95 ppm, m, 1H ; 6,8 et 6,9 ppm, 2s, 2H 7,35 ppm, td, 1H ; 7,75 ppm, dd, 1H ; 8,3 ppm, m, 1H ; 11,3 ppm, m, 1H échangeable. |
| 21 | 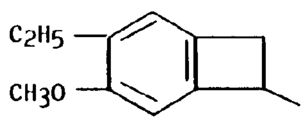 | 1 | 2 | 0 | **($CDCl_3$) (base)** 1,15 ppm, t, 3H ; 2,0 à 2,4 ppm, m, 2H+4H ; 2,53 ppm, q, 2H 2,6 à 2,9, m, 2H ; 2,89 ppm, dd, 1H ; 3,0 à 3,25 ppm, m, 1H+2H ; 3,34 ppm, dd, 1H, 3,58 à 3,75 ppm, m, 1H ; 3,82 ppm, s 3H ; 6,68 ppm, s, 1H ; 6,87 ppm, s, 1H ; 7,07 ppm, td, 1H ; 7,25 ppm, dd, 1H ; 7,72 ppm, dd, 1H |
| 22 | 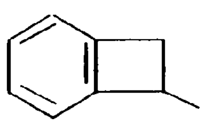 | 3 | 2 | 0 | **(DMSO-d6) (fumarate)** 1,75-2,15 ppm, m, 8H ; 2,5-2,85 ppm, m, 5H ; 3,3 ppm, m, 4H 3,5 ppm, m, 1H ; 6,6 ppm, s, 2H ; 7,15 ppm, m, 4H ; 7,3 ppm, td, 1H ; 7,7 ppm, dd, 1H ; 8,05 ppm, dd, 1H |

TABLEAU I (SUITE 9)

| EX. | R | m | n | p | R.M.N. (solvant) |
|---|---|---|---|---|---|
| 23 | 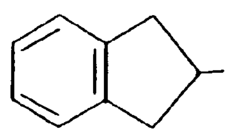 | 1 | 2 | 0 | (DMSO-d6) (fumarate)<br>1,75-2,2 ppm, m, 4H ; 2,35 ppm, td, 2H ; 2,5-2,85 ppm, m, 5H ; 2,85-3,3 ppm, m, 5H ; 4-7 ppm, 2H échangeables ; 6,55 ppm, s, 2H ; 7-7,3 ppm, m, 4H ; 7,3 ppm, td, 1H ; 7,7 ppm, dd, 1H ; 8,05 ppm, dd, 1H |
| 24 | 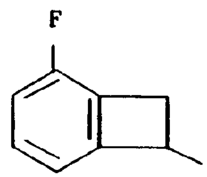 | 1 | 2 | 0 | ($CDCl_3$) (base)<br>2,0 à 2,4 ppm, m, 6H ; 2,6 à 3,2 ppm, m+m+m+m, 6H ; 3,4 ppm, m, 1H ; 3,7 ppm, m, 1H ; 6,7 à 7,3 ppm, m+m, 5H ; 7,7 ppm, dd, 1H |
| 25 | 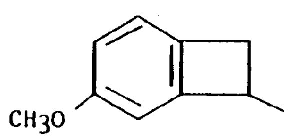 | 1 | 2 | 0 | ($CDCl_3$) (base)<br>2,0 à 2,35 ppm, m, 6H ; 2,6 à 2,95 ppm, m, 3H ; 3,15 ppm, m, 3H ; 3,3 ppm, dd, 1H ; 3,65 ppm, m, 1H ; 3,8 ppm, s, 3H 6,75 ppm, d, 1H ; 7 ppm, d, 2H ; 7,1 ppm, t, 1H ; 7,25 ppm, d, 1H ; 7,75 ppm, dd, 1H |

TABLEAU I (SUITE 10)

| EX. | R | m | n | p | R.M.N. (solvant) |
|---|---|---|---|---|---|
| 26 | 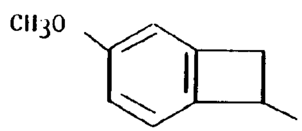 | 1 | 2 | 0 | **($CDCl_3$-d6) (base)** 2,0 à 2,4 ppm, m+m, 6H ; 2,6 à 2,9 ppm, dd+m, 3H ; 3,1 ppm, m, 1H ; 3,15 ppm, d, 2H ; 3,3 ppm, dd, 1H ; 3,65 ppm, m, 1H 3,8 ppm, s, 3H ; 6,7 ppm, m, 2H ; 7 ppm, d, 1H ; 7,05 ppm, td, 1H ; 7,25 ppm, dd, 1H ; 7,7 ppm, dd, 1H. |
| 32 | 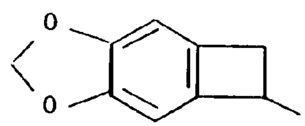 | 1 | 2 | 0 | **($CDCl_3$-d6 )(base)** 2,0 à 2,4 ppm, m+m, 2H+4H ; 2,5 à 2,8 ppm, m+dd+dd, 1H+1H+1H ; 3 à 3,3 ppm, dd+m+d, 1H+1H+2H ; 3,5 ppm, m, 1H 5,85 ppm, s, 2H ; 6,65 ppm, 2s, 2H ; 7 ppm, td, 1H ; 7,2 ppm, dd, 1H ; 7,7 ppm, dd, 1H. |
| 34 | 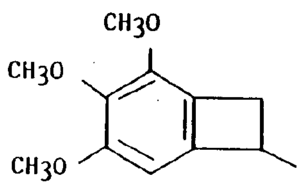 | 1 | 2 | 0 | **($CDCl_3$-d6) (base)** 2,15 ppm, m, 4H ; 2,3 ppm, m, 2H ; 2,6 à 3 ppm, m, 2H ; 3 à 3,3 ppm, d+m+dd, 2H+1H+1H ; 3,6 ppm, dd, 1H ; 3,7 ppm, m, 1H ; 3,8 à 3,95 ppm, 3s, 9H ; 6,4 ppm, s, 1H ; 7,1 ppm, td, 1H ; 7,25 ppm, dd, 1H ; 7,7 ppm, dd, 1H |

## ETUDE PHARMACOLOGIQUE

## EXEMPLE 35

### Test des stéréotypies induites par le méthylphénidate chez le rat

Pour l'essai, on a utilisé 24 rats Wistar mâles d'un poids compris entre 220 et 240 g étant à jeun depuis environ 24 heures. Chaque animal reçoit un premier traitement (halopéridol, produits de l'invention ou solvant), à un temps déterminé avant le deuxième traitement (méthylphénidate ou sérum physiologique) effectué au temps To par voie i.p.. Parmi les 24 animaux utilisés lors d'un essai, quatre servent de contrôle et reçoivent les traitements suivants: sérum + sérum i.p. 1 animal (contrôle sérum), solvant et méthylphénidate 40 mg/kg i.p. 2 animaux (contrôles méthylphénidate 40 mg/kg i.p.) et solvant + méthylphénidate 0,16, 0,63, 2,5 ou 10 mg/kg i.p. 1 animal (contrôles méthylphénidate 0,16, 0,63, 2,5 ou 10).

Les produits étudiés ont été administrés aux 20 animaux restants à des temps donnés, avant l'injection à To de 40 mg/kg i.p. de méthylphénidate. (Temps d'observation pour chaque animal 10 sec.). Les observations comportementales ont eu lieu 30 minutes (T30 mn) après le traitement avec le méthylphénidate. Un total de 10 périodes d'observations est réalisé pour chaque rat à T30mn. Pendant ces observations, la présence (1) ou l'absence (0) des stéréotypies et la présence du signe d'aplatissement du corps sont notées. La dose des composés à examiner qui provoque une catalepsie est aussi notée. L'analyse statistique a consisté à comparer pour une stéréotypie donnée, les taux des scores (0 à 10) obtenus par un groupe d'animaux ayant reçu le même traitement à ceux obtenus par le groupe d'animaux "Contrôles méthylphénidate 40", selon un test de Mann et Whitney avec la signification à P 0.05 (Siegel S et Castelan N.J.,1988).

Les résultats de l'étude ont démontré que la dose minimale d'halopéridol, administrée par voie i.p., qui provoque une catalepsie est de 0,63 mg/kg. La dose cataleptique des composés de l'invention, évaluée sous les mêmes conditions est très supérieure. Par exemple, pour le composé de l'exemple 1 la dose cataleptique est > 160 mg/kg et cette dose était la dose maximale testée. En effet, les composés de l'invention sont très peu cataleptogènes, ce qui constitue un avantage très considérable par rapport à l'halopéridol.

Les rapports des doses cataleptiques sur les doses qui inhibent les différents paramètres étudiés après traitement par l'halopéridol, ou après traitement par les composés de l'invention, et injection de 40 mg/kg i.p. de méthylphénidate sont donnés dans le Tableau II.

TABLEAU II

| COMPOSES | MACHONNEMENT | LOCOMOTION | RENIFLEMENT | REDRESSEMENT |
|---|---|---|---|---|
| HALOPERIDOL | 0,63/0,16=3,9 | 0,63/0,63=1 | 0,63/0,63=1 | 0,63/0,63=1 |
| EXEMPLE 1 | >160/2,5=>64 | >160/2,5=>64 | >160/2,5=>64 | >160/2,5=>64 |

Les résultats de ce tableau démontrent l'activité intéressante des produits de l'invention par rapport à l'halopéridol. La dose nécessaire de l'halopéridol pour avoir une inhibition totale de toutes les stéréotypies induites par le méthylphénidate chez le rat est identique à la dose cataleptique (rapport égal à 1). Par contre, pour le composé de l'exemple 1, la dose cataleptique est 64 fois supérieure à la dose qui inhibe la totalité des stéréotypies induites par le méthylphénidate.

Il est connu que l'induction de la catalepsie est le meilleur facteur d'évaluation des effets secondaires des neuroleptiques. Les résultats obtenus permettent donc de conclure que les composés de l'invention n'induisent aux doses actives aucun effet secondaire de nature extra-pyramidale.

**EXEMPLE 36**

Evaluation de l'activité antipsychotique

Le protocole utilisé pour évaluer la dose minimale qui inhibe le mâchonnement chez le rat après injection de 40 mg/kg i.p. de méthylphénidate est identique à celui décrit dans l'exemple 35. Les résultats du tableau III démontrent l'activité antipsychotique des composés de l'invention.

**TABLEAU III**

| COMPOSES | DOSE MINIMALE (mg/kg i.p.) |
|---|---|
| EXEMPLE 20 | 0,31 |
| EXEMPLE 22 | 1,25 |
| EXEMPLE 25 | 0,63 |
| EXEMPLE 26 | 0,31 |
| EXEMPLE 27 | 1,25 |

**PREPARATION PHARMACEUTIQUE**

**EXEMPLE 37**

**Gélules dosées à 2 mg de fumarate de 3-[1-(benzocyclobutèn-1-yl méthyl) pipérid-4-yl] 6-fluoro 1,2-benzisoxazole (R,S) [D.C.B.P.F.B.]**

| | |
|---|---|
| D.C.B.P.F.B. | 2 mg |
| Amidon de maïs | 15 mg |
| Lactose | 25 mg |
| Talc | 5 mg |

**Revendications**

**Revendications pour les Etats contractants suivants : AT, BE, CH, DE, DK, FR, GB, IT, LI, LU, NL, SE**

1. Composés de formule I :

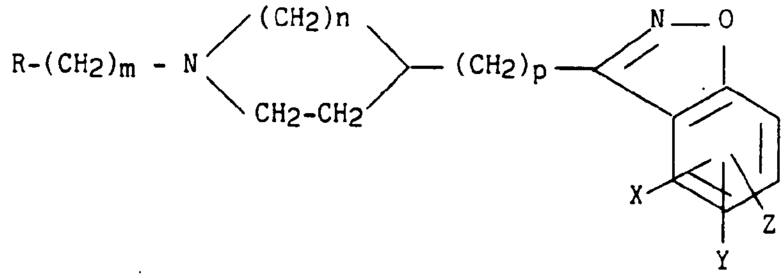

(I)

dans laquelle

- m représente un nombre entier de 0 à 5,
- n représente un nombre entier de 1 à 2,
- p est égal à 0, 1, ou 2,
- X, Y et Z identiques ou différents représentent chacun un atome d'hydrogène, un atome d'halogène, un radical alkyle linéaire ou ramifié de 1 à 6 atomes de carbone, un radical trifluorométhyle, un radical alcoxy de 1 à 6 atomes de carbone, un radical alkylthio de 1 à 6 atomes de carbone ou un radical hydroxy,
- R représente un radical benzofuran-2-yle ou 2,3-dihydro benzofuran-2-yle (chacun pouvant être substitué sur le cycle benzénique par un ou plusieurs atomes d'halogène, par des radicaux alcoxy de 1 à 6 atomes de carbone, par des radicaux alkylthio de 1 à 6 atomes de carbone, ou par des radicaux alkyle linéaires ou ramifiés de 1 à 6 atomes de carbone), un radical 2,3,6,7-tétrahydro benzo[1,2-b:4,5b']difuran-2-yl, un radical 4-oxo 4H-chromèn-2-yl (éventuellement substitué sur le cycle benzénique par un ou plusieurs atomes d'halogène, par des radicaux alkyle linéaires ou ramifiés de 1 à 6 atomes de carbone ou par des radicaux alcoxy de 1 à 6 atomes de carbone), un radical benzocyclobutènyle de formule A ou un radical indanyle de formule B :

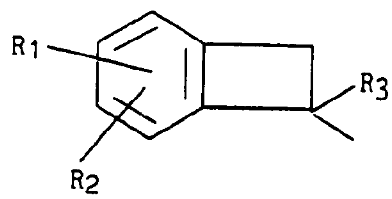

**(A)**

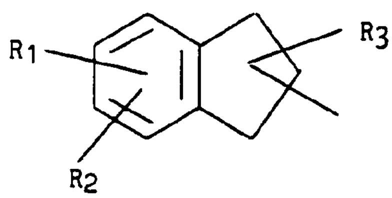

**(B)**

(dans lesquelles :

$R_1$ et $R_2$ identiques ou différents représentent chacun un atome d'hydrogène, un atome d'halogène, un radical trifluorométhyle, un radical alkyle linéaire ou ramifié de 1 à 6 atomes de carbone, un radical alcoxy de 1 à 6 atomes de carbone, un radical hydroxy, un radical hydroxyalkyle de 1 à 6 atomes de carbone, un radical alkylthio de 1 à 6 atomes de carbone, ou forment ensemble un radical méthylènedioxy ou un radical éthylènedioxy,

et $R_3$ représente un atome d'hydrogène ou un radical alkyle linéaire ou ramifié de 1 à 6 atomes de carbone),

ou un radical de formule C :

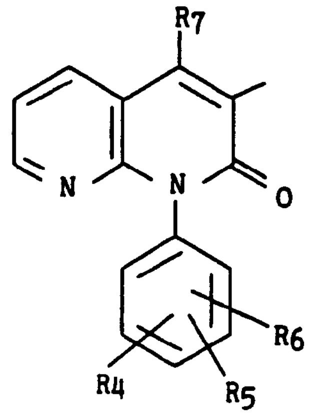

(C)

(dans laquelle

$R_4$, $R_5$ et $R_5$ identiques ou différents représentent chacun un atome d'hydrogène, un atome d'halogène, un radical trifluorométhyle, un radical hydroxy, un radical alkyle linéaire ou ramifié de 1 à 6 atomes de carbone, un radical alcoxy de 1 à 6 atomes de carbone, ou un radical alkylthio de 1 à 6 atomes de carbone,

et $R_7$ représente un atome d'hydrogène ou un radical hydroxy,)

leurs isomères optiques et leurs sels d'addition avec un acide organique ou minéral pharmaceutiquement acceptable.

**2.** Le 3-[1(benzocyclobutèn-1-yl méthyl) pipérid-4-yl] 6-fluoro 1,2-benzisoxazole (R,S), composé de formule I selon la revendication 1, et ses sels d'addition avec un acide minéral ou organique pharmaceutiquement acceptable.

**3.** Le (+) 3-[1-(benzocyclobutèn-1-yl méthyl) pipérid-4-yl] 6-fluoro 1,2-benzisoxazole, composé de formule I selon la revendication 1 et ses sels d'addition avec un acide minéral ou organique pharmaceutiquement acceptable.

**4.** Le (-) 3-[1-(benzocyclobutèn-1-yl méthyl) pipérid-4-yl] 6-fluoro 1,2-benzisoxazole, composé de formule I selon la revendication 1 et ses sels d'addition avec un acide minéral ou organique pharmaceutiquement acceptable.

**5.** Le 3-{1-{[1,2-dihydro 2-oxo 1-(3-trifluorométhyl phényl) 1,8-naphthyridin-3-yl] éthyl} pipérid-4-yl} 6-fluoro 1,2-benzisoxazole, composé de formule I selon la revendication 1, ses sels d'addition avec un acide minéral ou organique pharmaceutiquement acceptable.

**6.** Le 3-[1-(2-benzocyclobutèn-4-yl éthyl) pipérid-4-yl] 6-fluoro 1,2-benzisoxazole (R,S), composé de formule I selon la revendication 1, ses isomères optiques et ses sels d'addition avec un acide minéral ou organique pharmaceutiquement acceptable.

**7.** Le 6-fluoro 3-{1-[(1-méthyl benzocyclobutèn-1-yl) méthyl] pipérid-4-yl} 1,2-benzisoxazole (R,S), composé de formule I selon la revendication 1, ses isomères optiques et ses sels d'addition avec un acide minéral ou organique pharmaceutiquement acceptable.

**8.** Le 3-{1-[(4,5-diméthoxy benzocyclobutèn-1-yl) méthyl] pipérid-4-yl} 6-fluoro 1,2-benzisoxazole (R,S), composé de formule I selon la revendication 1, ses isomères optiques et ses sels d'addition avec un acide minéral ou organique pharmaceutiquement acceptable.

**9.** Le 6-fluoro 3-[1-(indan-1-yl méthyl) pipérid-4-yl] 1,2-benzisoxazole (R,S), composé de formule I selon la revendication 1, ses isomères optiques et ses sels d'addition avec un acide minéral ou organique pharmaceutiquement acceptable.

**10.** Procédé de préparation des composés de formule I, selon la revendication 1, caractérisé en ce que l'on

fait réagir :

ou

un composé de formule II :

$$R - (CH_2)_m - W \qquad (II)$$

dans laquelle R et m ont la même signification que pour la formule I selon la revendication 1, et W représente un atome d'halogène, ou un radical tosyloxy ou un radical mésyloxy,

soit

avec un composé de formule III :

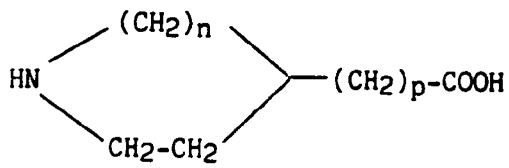

(III)

dans laquelle n et p ont là même signification que pour la formule I, selon la revendication 1 en présence de N,N-diéthyléthylène diamine dans la diméthylformamide ou un autre solvant organique équivalent,

pour former un composé de formule IV :

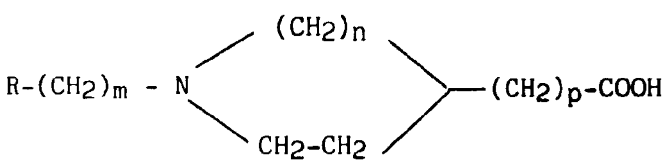

(IV)

dans laquelle R, m, n et p ont la même signification que pour la formule I, selon la revendication 1,

que l'on soumet à l'action du chlorure de thionyle ou du chlorure d'oxalyle pour former un chlorure d'acide de formule V :

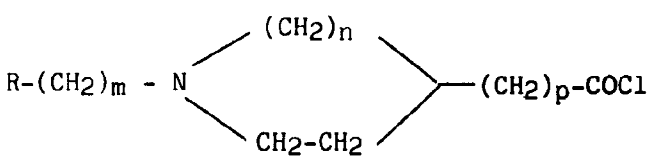

(V)

dans laquelle la signification de R, m, n et p reste identique à celle donnée précédemment pour la formule I, selon la revendication 1,

que l'on condense en présence de chlorure d'aluminium avec un composé de formule VI :

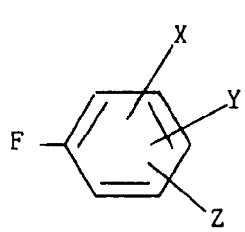

(VI)

dans laquelle X, Y et Z ont la même signification que pour la formule I, selon la revendication 1, pour former un composé de formule VII :

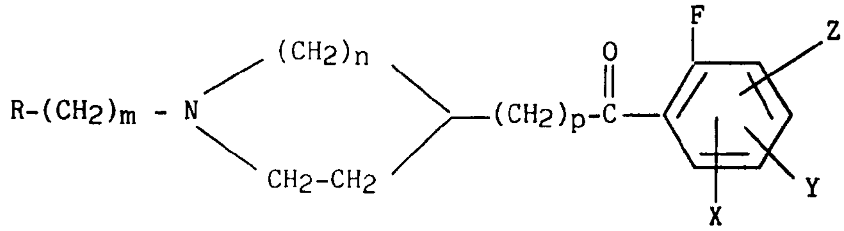

(VII)

dans laquelle R, X, Y, Z, m, n et p ont la même signification que pour la formule I, selon la revendication 1,

que l'on soumet à l'action du chlorhydrate d'hydroxylamine en présence de 2-diéthylamino éthylamine en milieu alcoolique,

pour obtenir un composé de formule VIII :

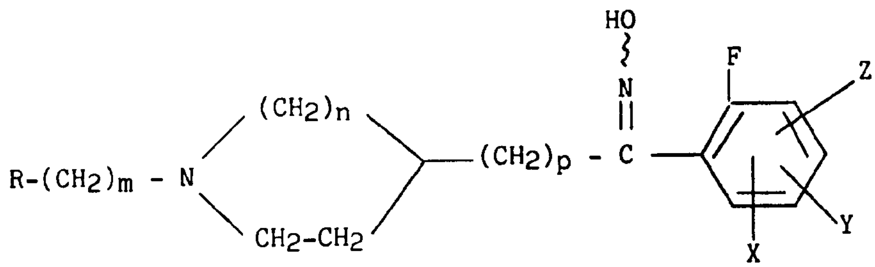

(VIII)

dans laquelle la signification de R, X, Y, Z, m, n et p reste identique à celle donnée pour la formule I, selon la revendication 1,

que l'on cyclise à l'aide d'une base minérale forte pour obtenir les composés de la formule I, selon la revendication 1,

soit

avec un composé de formule IX :

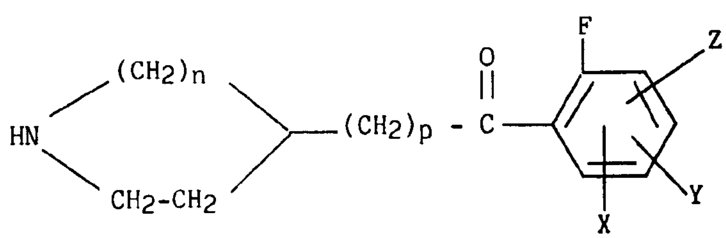

(IX)

dans laquelle X, Y, Z, n et p ont la même signification que pour la formule I, selon la revendication 1, en présence de N,N-diéthyléthylène diamine dans la diméthylformamide ou un autre solvant organique équivalent,

pour former les composés de formule VII, à partir desquels on obtient les composés de formule VIII, puis les composés de formule I, selon la revendication 1, en utilisant le procédé indiqué ci-dessus,

soit

avec un composé de formule X :

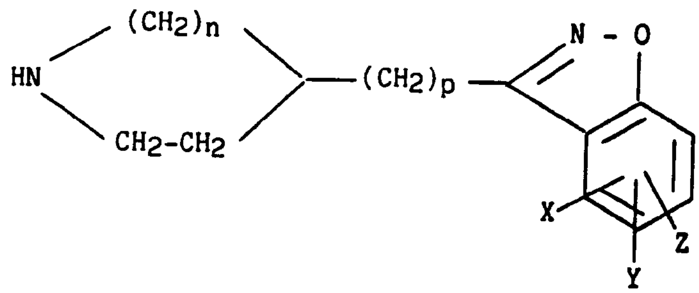

(X)

dans laquelle X, Y, Z, n et p ont la même signification que pour la formule I, selon la revendication 1 en présence de N,N-diéthyléthylènediamine dans la diméthylformamide ou un autre solvant organique équivalent,

pour former les composés de formule I, selon la revendication 1,

ou

un composé de formule $XI_A$ ou $XI_B$ :

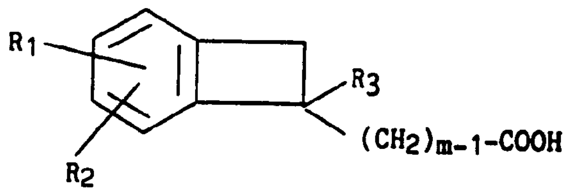

($XI_A$)

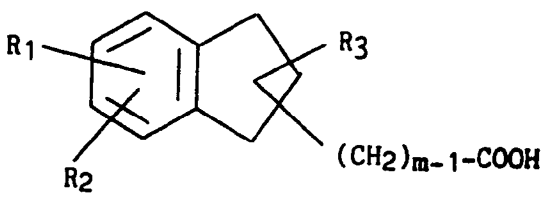

($XI_B$)

dans lesquelles $R_1$, $R_2$, $R_3$ et m ont la même signification que pour la formule I, sauf que dans ce cas, m, ne peut représenter le nombre 0,
avec un composé de formule X,
en présence de carbonyldiimidazole, pour former un composé de formule $XII_A$ ou $XII_B$ :

(XIIA)

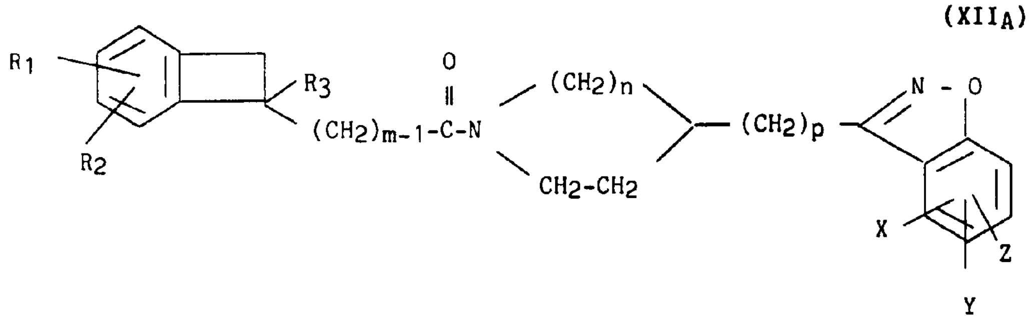

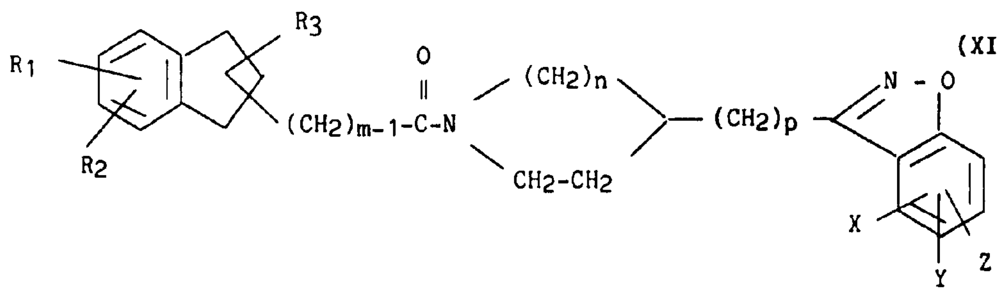

(XIIB)

dans lesquelles $R_1$, $R_2$, $R_3$, m, n, p, X, Y, et Z ont la signification donnée pour la formule I, sauf que dans ce cas, m, ne représente pas le nombre 0,
que l'on soumet à l'action d'hydrure de lithium et d'aluminium,
pour former les composés de formule I,

ou

un composé de formule XIII :

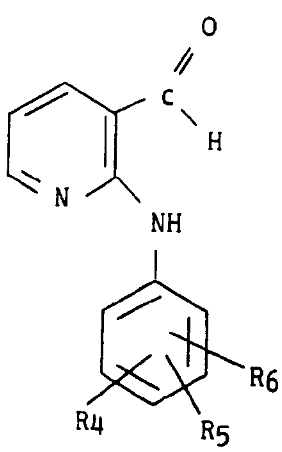

(XIII)

dans laquelle $R_4$, $R_5$, et $R_6$ ont la même signification que pour la formule I,
avec un composé de formule XIV :

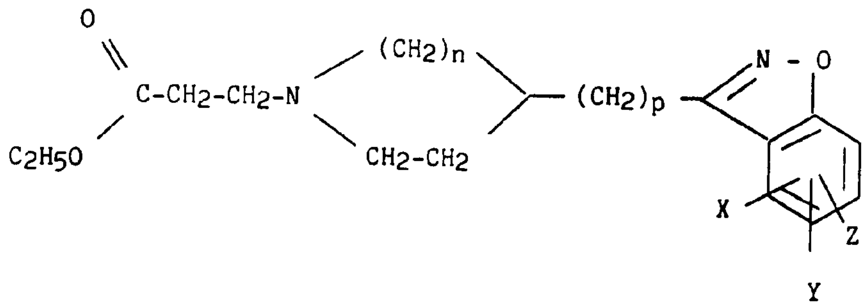

(XIV)

dans laquelle, la signification de n, p, X, Y, et Z reste identique à celle donnée pour la formule I selon la revendication 1, pour former les composés de formule I, selon la revendication 1, dans laquelle R représente un radical de formule C et m est égal à 1,

lesquels composés de formule I, selon la revendication 1,

peuvent être salifiés avec un acide minéral ou organique pharmaceutiquement acceptable, pour former les sels correspondants,
ou
être séparés en leurs isomères optiques et ensuite salifiés.

**11.** Composition pharmaceutique contenant comme principe actif un composé d'une quelconque des revendications 1 à 9, en association ou en mélange avec un excipient ou un véhicule inerte, non toxique, pharmaceutiquement acceptable.

**12.** Composition pharmaceutique selon la revendication 11 renfermant le principe actif à la dose de 0,2 à 100 mg.

**13.** Composition pharmaceutique selon les revendications 11 et 12 renfermant comme principe actif au moins un composé selon les revendications 1 à 9 utilisable dans le traitement des maladies nécessitant des agents antipsychotiques, sédatifs, anxiolytiques, antiagressifs et analgésiques, ou dans le traitement de la schizophrénie et de la dépression.

**Revendication pour l'Etat contractant suivant : ES**

**1.** Le procédé de préparation des composés de formule I :

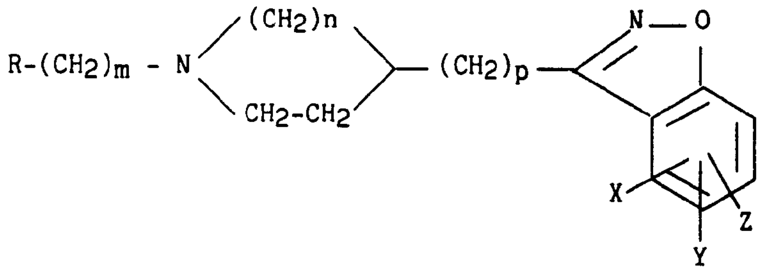

(I)

dans laquelle :

- m représente un nombre entier de 0 à 5,

- n représente un nombre entier de 1 à 2,
- p est égal à 0, 1, ou 2,
- X, Y et Z identiques ou différents représentent chacun un atome d'hydrogène, un atome d'halogène, un radical alkyle linéaire ou ramifié de 1 à 6 atomes de carbone, un radical trifluorométhyle, un radical alcoxy de 1 à 6 atomes de carbone, un radical alkylthio de 1 à 6 atomes de carbone ou un radical hydroxy,
- R représente un radical benzofuran-2-yle ou 2,3-dihydro benzofuran-2-yle (chacun pouvant être substitué sur le cycle benzénique par un ou plusieurs atomes d'halogène, par des radicaux alcoxy de 1 à 6 atomes de carbone, par des radicaux alkylthio de 1 à 6 atomes de carbone, ou par des radicaux alkyle linéaires ou ramifiés de 1 à 6 atomes de carbone),
  un radical 2,3,6,7-tétrahydro benzo[1,2-b:4,5b']difuran-2-yl, un radical 4-oxo 4H-chromèn-2-yl (éventuellement substitué sur le cycle benzénique par un ou plusieurs atomes d'halogène, par des radicaux alkyle linéaires ou ramifiés de 1 à 6 atomes de carbone ou par des radicaux alcoxy de 1 à 6 atomes de carbone), un radical benzocyclobutènyle de formule A ou un radical indanyle de formule B :

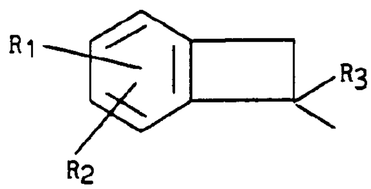

(A)

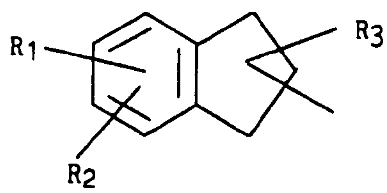

(B)

(dans lesquelles :

$R_1$ et $R_2$ identiques ou différents représentent chacun un atome d'hydrogène, un atome d'halogène, un radical trifluorométhyle, un radical alkyle linéaire ou ramifié de 1 à 6 atomes de carbone, un radical alcoxy de 1 à 6 atomes de carbone, un radical hydroxy, un radical hydroxyalkyle de 1 à 6 atomes de carbone, un radical alkylthio de 1 à 6 atomes de carbone, ou forment ensemble un radical méthylènedioxy ou un radical éthylènedioxy,

et $R_3$ représente un atome d'hydrogène ou un radical alkyle linéaire ou ramifié de 1 à 6 atomes de carbone),

ou un radical de formule C :

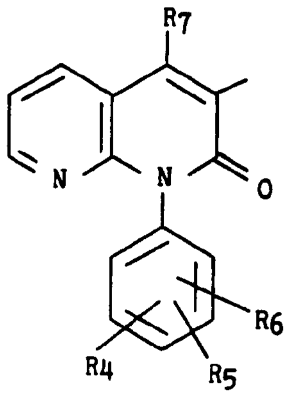

(C)

(dans laquelle

$R_4$, $R_5$ et $R_5$ identiques ou différents représentent chacun un atome d'hydrogène, un atome d'halogène, un radical trifluorométhyle, un radical hydroxy, un radical alkyle linéaire ou ramifié de 1 à 6 atomes de carbone, un radical alcoxy de 1 à 6 atomes de carbone, ou un radical alkylthio de 1 à 6 atomes de carbone,

et $R_7$ représente un atome d'hydrogène ou un radical hydroxy,)

de leurs isomères optiques et de leurs sels d'addition avec un acide organique ou minéral pharmaceutiquement acceptable ;

caractérisé en ce que l'on fait réagir :

ou

**A) un composé de formule II :**

$$R - (CH_2)_m - W \qquad (II)$$

dans laquelle R et m ont les significations précédemment définies, et W représente un atome d'halogène, ou un radical tosyloxy ou un radical mésyloxy,

a) soit avec un composé de formule III :

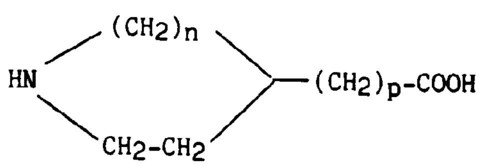

(III)

dans laquelle n et p ont les significations précédemment définies en présence de N,N-diéthyléthylène diamine dans la diméthylformamide ou un autre solvant organique équivalent,

pour former un composé de formule IV :

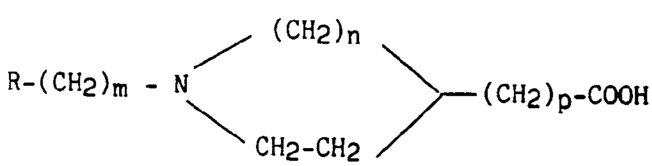

(IV)

dans laquelle R, m, n et p ont les significations précédemment définies,

que l'on soumet à l'action du chlorure de thionyle ou du chlorure d'oxalyle pour former un chlorure d'acide de formule V :

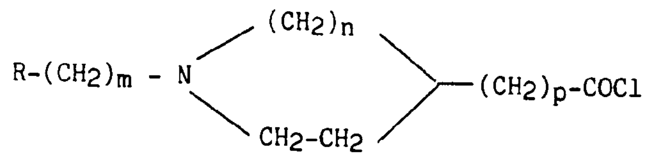

(V)

dans laquelle les significations de R, m, n et p reste identique à celle donnée précédemment, que l'on condense en présence de chlorure d'aluminium avec un composé de formule VI :

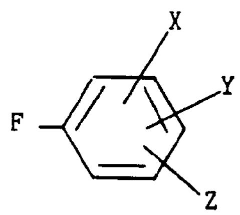

(VI)

dans laquelle X, Y et Z ont les significations précédemment définies, pour former un composé de formule VII :

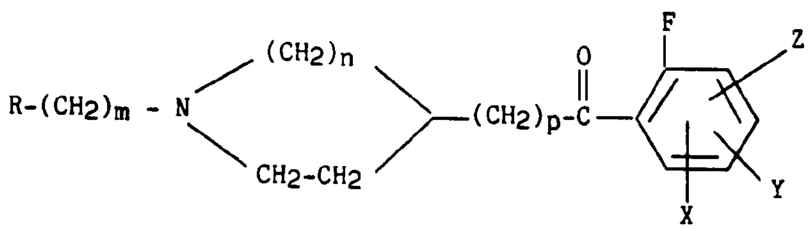

(VII)

dans laquelle R, X, Y, Z, m, n et p ont les significations précédemment définies, que l'on soumet à l'action du chlorhydrate d'hydroxylamine en présence de 2-diéthylamino éthylamine en milieu alcoolique, pour obtenir un composé de formule VIII :

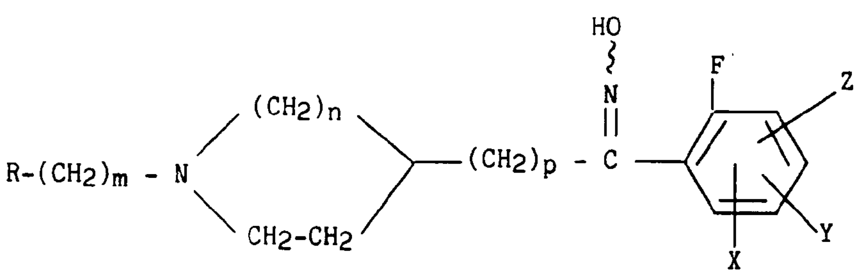

(VIII)

dans laquelle les significations de R, X, Y, Z, m, n et p sont telles que précédemment définies, que l'on cyclise à l'aide d'une base minérale forte pour obtenir les composés de formule I,

b) soit avec un composé de formule IX :

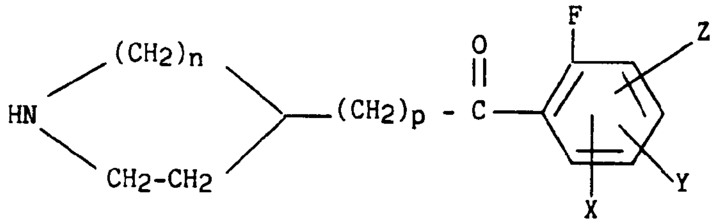

(IX)

dans laquelle X, Y, Z, n et p ont les significations précédemment définies, en présence de N,N-diéthyléthylène diamine dans la diméthylformamide ou un autre solvant organique équi-valent,

pour former les composés de formule VII, à partir desquels on obtient les composés de formule VIII, puis les composés de formule I, en utilisant le procédé indiqué ci-dessus,

c) soit avec un composé de formule X :

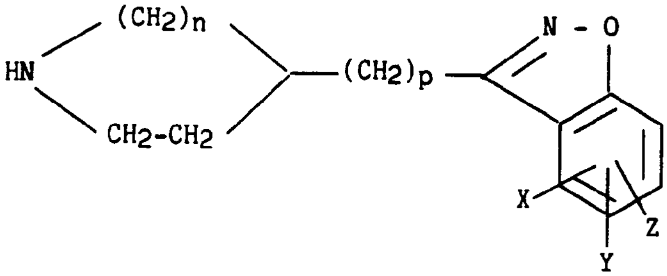

(X)

dans laquelle X, Y, Z, n et p ont les significations précédemment définies, en présence de N,N-diéthyléthylène- diamine dans la diméthylformamide ou un autre solvant organique équivalent,

pour former les composés de formule I,

ou

**B) un composé de formule $XI_A$ou $XI_B$:**

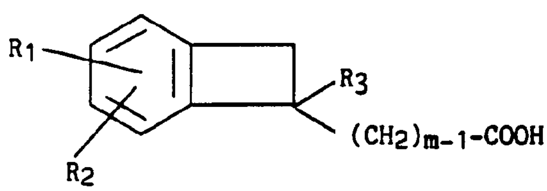

($XI_A$)

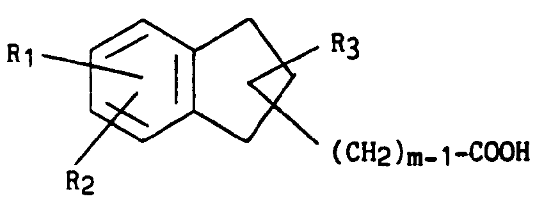

($XI_B$)

dans lesquelles $R_1$, $R_2$, $R_3$ et m ont la même signification que pour la formule I, sauf que dans ce cas, m, ne peut représenter le nombre 0, avec un composé de formule X,en présence de carbonyldiimidazole, pour former un composé de formule $XII_A$ ou $XII_B$ :

(XII$_A$)

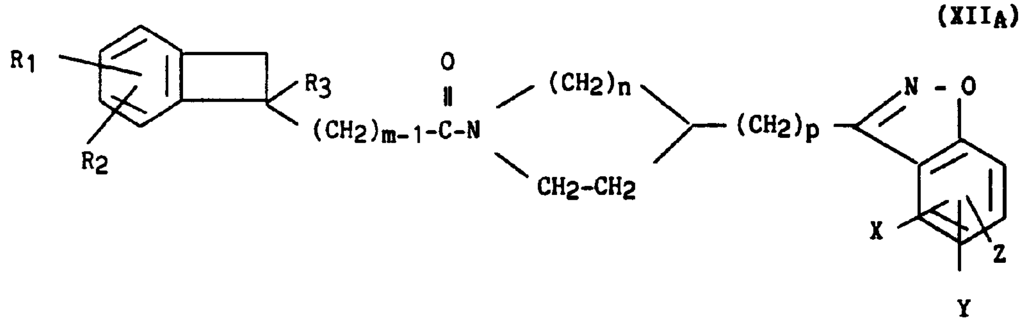

(XII$_B$)

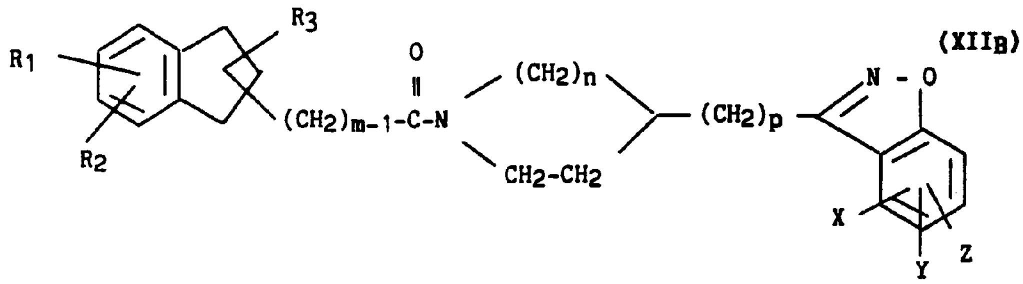

dans lesquelles $R_1$, $R_2$, $R_3$, m, n, p, X, Y, et Z ont les significations donnée pour la formule I, sauf que dans ce cas, m, ne représente pas le nombre 0,
que l'on soumet à l'action d'hydrure de lithium et d'aluminium,
pour former les composés de formule I,

ou

**C) un composé de formule XIII :**

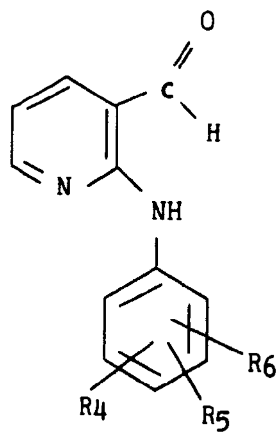

(XIII)

dans laquelle $R_4$, $R_5$, et $R_5$ ont les significations précédemment définies, avec un composé de formule XIV :

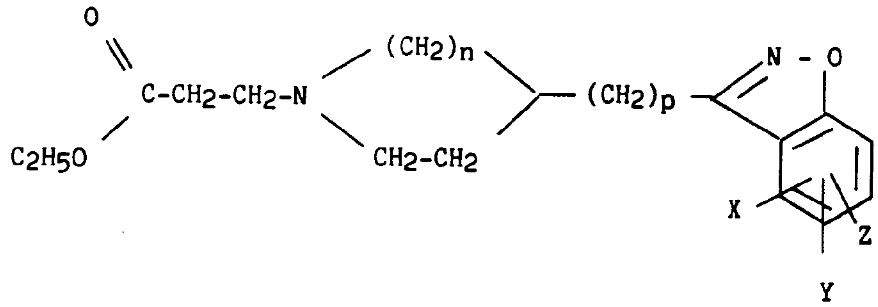

(XIV)

dans laquelle, la signification de n, p, X, Y, et Z reste identique à celle donnée pour la formule I précédemment définies, pour former les composés de formule I, dans laquelle R représente un radical de formule C et m est égal à 1,

lesquels composés de formule I

peuvent être salifiés avec un acide minéral ou organique pharmaceutiquement acceptable, pour former les sels correspondants,

ou

être séparés en leurs isomères optiques et ensuite salifiés.

**Revendication pour l'Etat contractant suivant : GR**

**1.** Le procédé de préparation des composés de formule I :

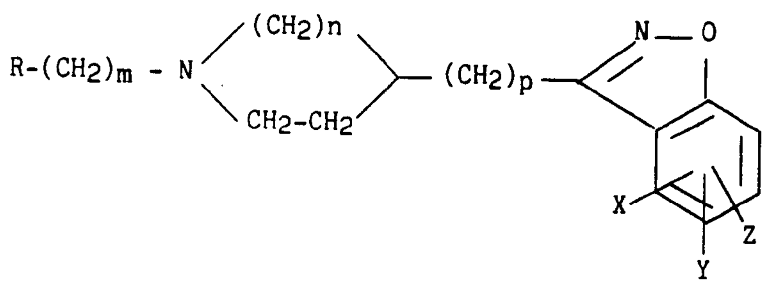

(I)

dans laquelle :

- m représente un nombre entier de 0 à 5,
- n représente un nombre entier de 1 à 2,
- p est égal à 0, 1, ou 2,
- X, Y et Z identiques ou différents représentent chacun un atome d'hydrogène, un atome d'halogène, un radical alkyle linéaire ou ramifié de 1 à 6 atomes de carbone, un radical trifluorométhyle, un radical alcoxy de 1 à 6 atomes de carbone, un radical alkylthio de 1 à 6 atomes de carbone ou un radical hydroxy,
- R représente un radical benzofuran-2-yle ou 2,3-dihydro benzofuran-2-yle (chacun pouvant être substitué sur le cycle benzénique par un ou plusieurs atomes d'halogène, par des radicaux alcoxy de 1 à 6 atomes de carbone, par des radicaux alkylthio de à 6 atomes de carbone, ou par des radicaux alkyle linéaires ou ramifiés de 1 à 6 atomes de carbone),
  un radical 2,3,6,7-tétrahydro benzo[1,2-b:4,5b']difuran-2-yl, un radical 4-oxo 4H-chromèn-2-yl (éventuellement substitué sur le cycle benzénique par un ou plusieurs atomes d'halogène, par des radicaux alkyle linéaires ou ramifiés de 1 à 6 atomes de carbone ou par des radicaux alcoxy de 1 à 6 atomes de carbone), un radical benzocyclobutènyle de formule A ou un radical indanyle de formule B :

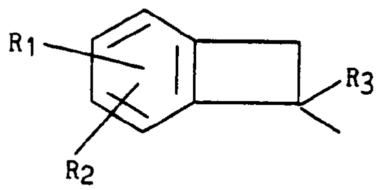

(A)

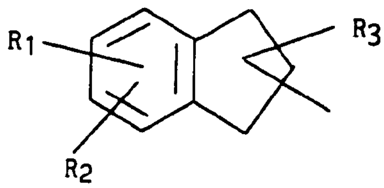

(B)

dans lesquelles :

$R_1$ et $R_2$ identiques ou différents représentent chacun un atome d'hydrogène, un atome d'halogène, un radical trifluorométhyle, un radical alkyle linéaire ou ramifié de 1 à 6 atomes de carbone, un radical alcoxy de 1 à 6 atomes de carbone, un radical hydroxy, un radical hydroxyalkyle de 1 à 6 atomes de carbone, un radical alkylthio de 1 à 6 atomes de carbone, ou forment ensemble un radical méthylènedioxy ou un radical éthylènedioxy,

et $R_3$ représente un atome d'hydrogène ou un radical alkyle linéaire ou ramifié de 1 à 6 atomes de carbone),

ou un radical de formule C :

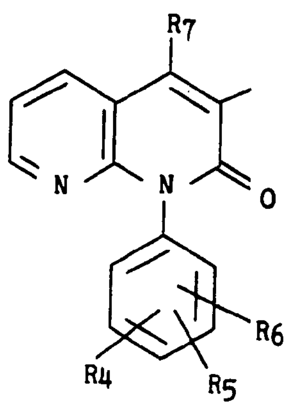

(C)

(dans laquelle

$R_4$, $R_5$ et $R_5$ identiques ou différents représentent chacun un atome d'hydrogène, un atome d'halogène, un radical trifluorométhyle, un radical hydroxy, un radical alkyle linéaire ou ramifié de 1 à 6 atomes de carbone, un radical alcoxy de 1 à 6 atomes de carbone, ou un radical alkylthio de 1 à 6 atomes de carbone,

et $R_7$ représente un atome d'hydrogène ou un radical hydroxy,)

de leurs isomères optiques et de leurs sels d'addition avec un acide organique ou minéral pharmaceutiquement acceptable ;

caractérisé en ce que l'on fait réagir :

ou

**A) un composé de formule II :**

R - $(CH_2)_m$ - W (II)

dans laquelle R et m ont les significations précédemment définies, et W représente un atome d'halogène, ou un radical tosyloxy ou un radical mésyloxy,

a) soit avec un composé de formule III :

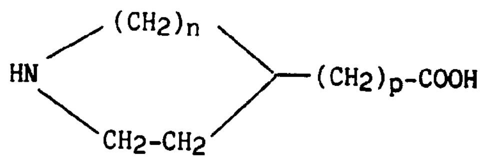

(III)

dans laquelle n et p ont les significations précédemment définies en présence de N,N-diéthyléthylène diamine dans la diméthylformamide ou un autre solvant organique équivalent,

pour former un composé de formule IV :

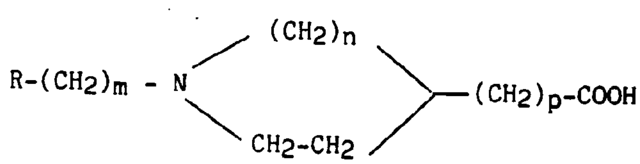

(IV)

dans laquelle R, m, n et p ont les significations précédemment définies,

que l'on soumet à l'action du chlorure de thionyle ou du chlorure d'oxalyle pour former un chlorure d'acide de formule V :

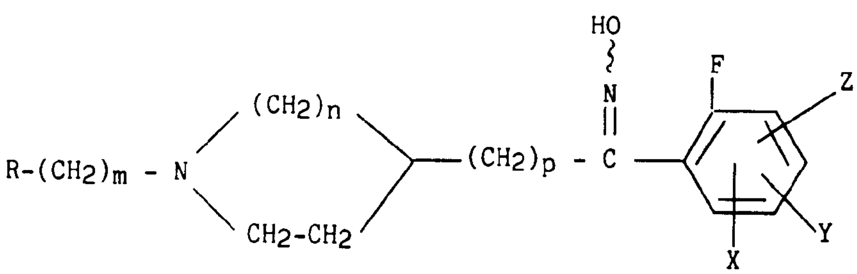

(VIII)

dans laquelle les significations de R, m, n et p reste identique à celle donnée précédemment,

que l'on condense en présence de chlorure d 'aluminium avec un composé de formule VI :

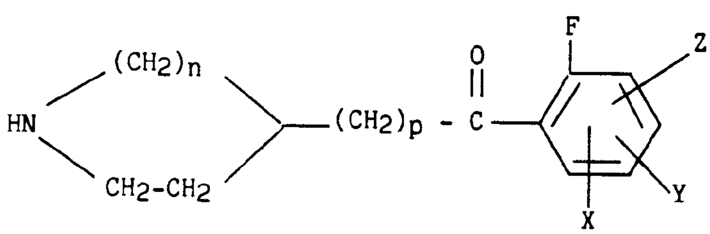

(IX)

dans laquelle X, Y et Z ont les significations précédemment définies,

pour former un composé de formule VII :

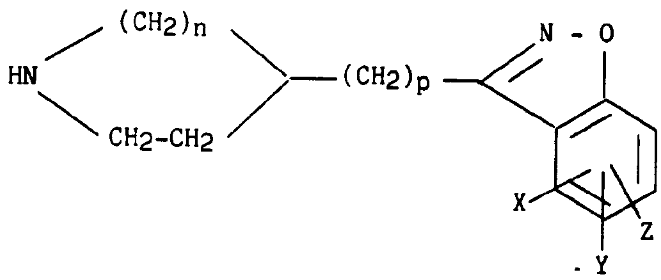

(X)

dans laquelle R, X, Y, Z, m, n et p ont les significations précédemment définies,

que l'on soumet à l'action du chlorhydrate d'hydroxylamine en présence de 2-diéthylamino éthylamine en milieu alcoolique,

pour obtenir un composé de formule VIII :

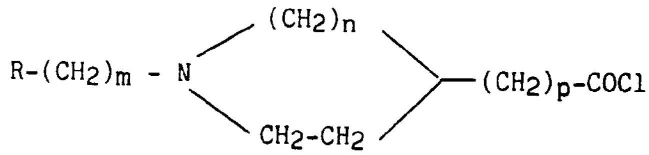

(V)

dans laquelle les significations de R, X, Y, Z, m, n et p sont telles que précédemment définies,

que l'on cyclise à l'aide d'une base minérale forte pour obtenir les composés de formule I,

b) soit avec un composé de formule IX :

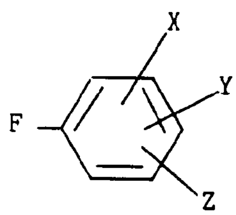

(VI)

dans laquelle X, Y, Z, n et p ont les significations précédemment définies, en présence de N,N-diéthyléthylène diamine dans la diméthylformamide ou un autre solvant organique équi-valent,

pour former les composés de formule VII, à partir desquels on obtient les composés de formule VIII, puis les composés de formule I, en utilisant le procédé indiqué ci-dessus,

c) soit avec un composé de formule X :

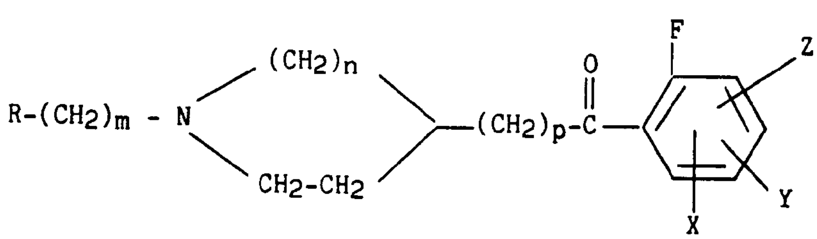

(VII)

dans laquelle X, Y, Z, n et p ont les significations précédemment définies, en présence de N,N-diéthyléthylène- diamine dans la diméthylformamide ou un autre solvant organique équivalent,

pour former les composés de formule I,

ou

**B) un composé de formule $XI_A$ou $XI_B$:**

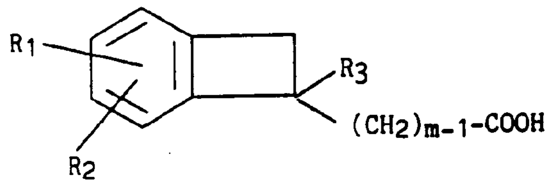

**(XIA)**

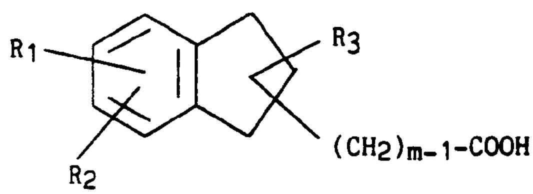

**(XIB)**

dans lesquelles $R_1$, $R_2$, $R_3$ et m ont la même signification que pour la formule I, sauf que dans ce cas, m, ne peut représenter le nombre 0, avec un composé de formule X,en présence de carbonyldiimidazole, pour former un composé de formule $XII_A$ ou $XII_B$ :

**(XIIA)**

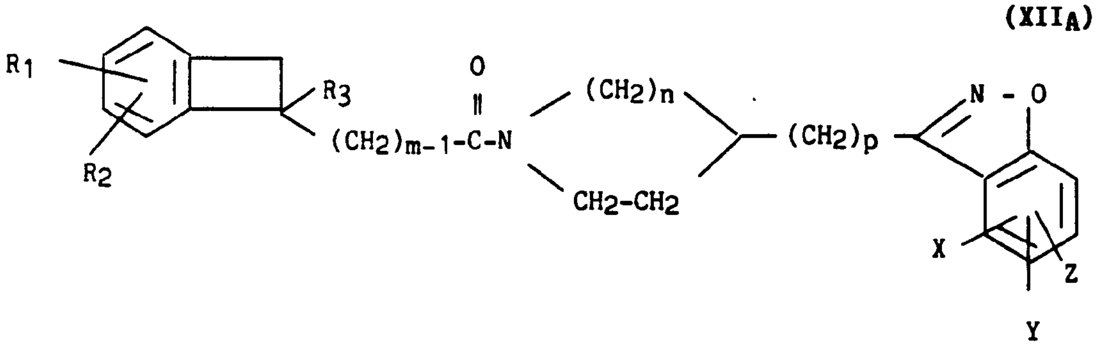

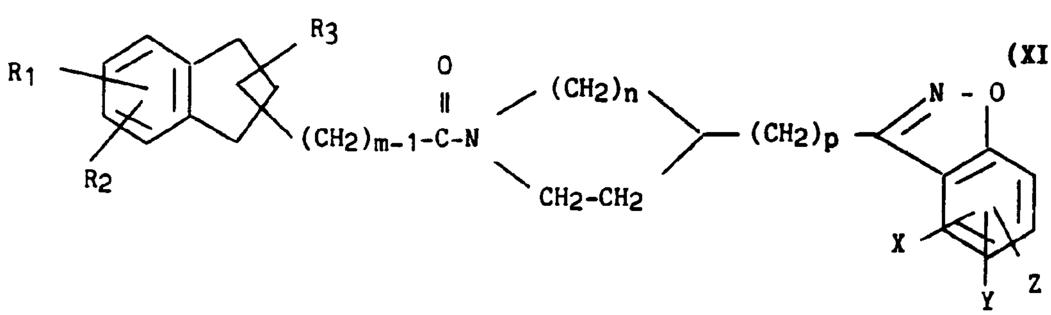

**(XIIB)**

dans lesquelles $R_1$, $R_2$, $R_3$, m, n, p, X, Y, et Z ont les significations donnée pour la formule I, sauf que dans ce cas, m, ne représente pas le nombre 0,

que l'on soumet à l'action d'hydrure de lithium et d'aluminium, pour former les composés de formule I,

ou

**C) un composé de formule XIII :**

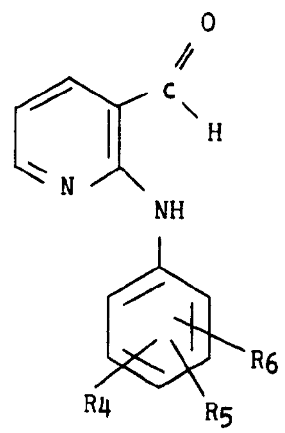

(XIII)

dans laquelle $R_4$, $R_5$, et $R_5$ ont les significations précédemment définies, avec un composé de formule XIV :

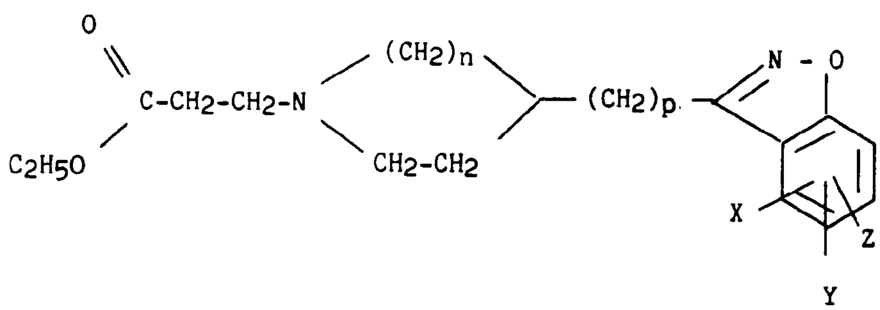

(XIV)

dans laquelle, la signification de n, p, X, Y, et Z reste identique à celle donnée pour la formule I précédemment définies, pour former les composés de formule I, dans laquelle R représente un radical de formule C et m est égal à 1,

lesquels composés de formule I

peuvent être salifiés avec un acide minéral ou organique pharmaceutiquement acceptable, pour former les sels correspondants,

ou

être séparés en leurs isomères optiques et ensuite salifiés.

## Patentansprüche

### Patentansprüche für folgende Vertragsstaaten : AT, BE, CH, DE, DK, FR, GB, IT, LI, LU, NL, SE

1. Verbindungen der Formel I:

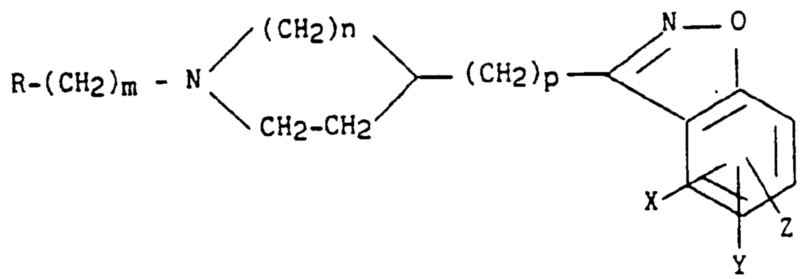

(I)

in der
- m eine ganze Zahl mit einem Wert von 0 bis 5,
- n eine ganze Zahl mit einem Wert von 1 bis 2,
- p 0, 1 oder 2,
- X, Y und Z, die gleichartig oder verschieden sein können, jeweils ein Wasserstoffatom. ein Halogenatom, eine geradkettige oder verzweigte Alkylgruppe mit 1 bis 6 Kohlenstoffatomen, eine Trifluormethylgruppe, eine Alkoxygruppe mit 1 bis 6 Kohlenstoffatomen, eine Alkylthiogruppe mit 1 bis 6 Kohlenstoffatomen oder eine Hydroxylgruppe,
- R eine Benzofuran-2-yl-gruppe oder eine 2,3,Dihydrobenzofuran-2-yl-gruppe (die jeweils am Benzolring durch ein oder mehrere Halogenatome. Alkoxygruppen mit 1 bis 6 Kohlenstoffatomen, Alkylthiogruppen mit 1 bis 6 Kohlenstoffatomen oder geradkettigen oder verzweigten Alkylgruppen mit 1 bis 6 Kohlenstoffatomen substituiert sein können). eine 2,3,6,7-Tetrahydro-benzo-[1,2-b:4,5b']difuran-2-yl-gruppe, eine 4-Oxo-4H-chromen-2-yl-gruppe (die gegebenenfalls am Benzolring durch ein oder mehrere Halogenatome, geradkettige oder verzweigte Alkylgruppen mit 1 bis 6 Kohlenstoffatomen oder Alkoxygruppen mit 1 bis 6 Kohlenstoffatomen substituiert sein kann), eine Benzocyclobutenyl-gruppe der Formel A oder eine Indanylgruppe der Formel B:

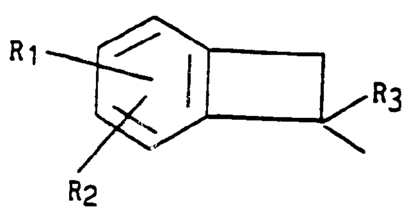

(A)

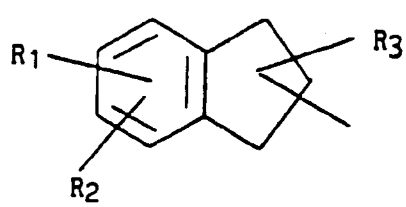

(B)

(worin:

$R_1$ und $R_2$, die gleichartig oder verschieden sein können, jeweils ein Wasserstoffatom, ein Halogenatom, eine Trifluormethylgruppe, eine geradkettige oder verzweigte Alkylgruppe mit 1 bis 6 Kohlenstoffatomen, eine Alkoxygruppe mit 1 bis 6 Kohlenstoffatomen, eine Hydroxylgruppe. eine Hydroxyalkylgruppe mit 1 bis 6 Kohlenstoffatomen, eine Alkylthiogruppe mit 1 bis 6 Kohlenstoffatomen

oder gemeinsam eine Methylendioxygruppe oder eine Ethylendioxygruppe und

$R_3$ ein Wasserstoffatom oder eine geradkettige oder verzweigte Alkylgruppe mit 1 bis 6 Kohlenstoffatomen darstellen),

oder eine Gruppe der Formel C:

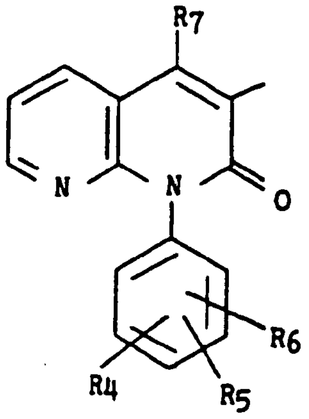

(C)

(in der

$R_4$, $R_5$ und $R_6$, die gleichartig oder verschieden sein können, jeweils ein Wasserstoffatom, ein Halogenatom, eine Trifluormethylgruppe, eine Hydroxylgruppe, eine geradkettige oder verzweigte Alkylgruppe mit 1 bis 6 Kohlenstoffatomen, eine Alkoxygruppe mit 1 bis 6 Kohlenstoffatomen oder eine Alkylthiogruppe mit 1 bis 6 Kohlenstoffatomen und

$R_7$ ein Wasserstoffatom oder eine Hydroxylgruppe darstellen) bedeuten. deren optische Isomere und deren Additionssalze mit einer pharmazeutisch annehmbaren organischen oder anorganischen Säure.

2. Verbindung der Formel I nach Anspruch 1, nämlich 3-[1-(Benzocylobuten-1-yl-methyl)-piperid-4-yl]-6-fluor-1,2-benzisoxazol (R,S) und dessen Additionssalze mit einer pharmazeutisch annehmbaren anorganischen oder organischen Säure.

3. Verbindung der Formel I nach Anspruch 1, nämlich (+)-3-[1-(Benzocylobuten-1-yl-methyl)-piperid-4-yl]-6-fluor-1,2-benzisoxazol und dessen Additionssalze mit einer pharmazeutisch annehmbaren anorganischen oder organischen Säure.

4. Verbindung der Formel I nach Anspruch 1, nämlich (-)-3-[1-(Benzocylobuten-1-yl-methyl)-piperid-4-yl]-6-fluor-1,2-benzisoxazol und dessen Additionssalze mit einer pharmazeutisch annehmbaren anorganischen oder organischen Säure.

5. Verbindung der Formel I nach Anspruch 1, nämlich 3-{1-{[1.2-Dihydro-2-oxo-1-(3-trifluormethyl-phenyl)-1,8-naphthyridin-3-yl]-ethyl}-piperid-4-yl}-6-fluor-1,2-benzisoxazol und dessen Additionssalze mit einer pharmazeutisch annehmbaren anorganischen oder organischen Säure.

6. Verbindung der Formel I nach Anspruch 1, nämlich 3-[1-(2-Benzocylobuten-1-yl-ethyl)-piperid-4-yl]-6-fluor-1,2-benzisoxazol (R,S), dessen optische Isomere und dessen Additionssalze mit einer pharmazeutisch annehmbaren anorganischen oder organischen Säure.

7. Verbindung der Formel I nach Anspruch 1, nämlich 6-Fluor-3-{1-[(1-(methyl-benzocylobuten-1-yl-methyl)-piperid-4-yl}-1,2-benzisoxazol (R,S), dessen optische Isomere und dessen Additionssalze mit einer pharmazeutisch annehmbaren anorganischen oder organischen Säure.

8. Verbindung der Formel I nach Anspruch 1, nämlich 3-{1-{(4,5-Dimethoxybenzocyclobuten-1-yl)-methyl]-piperid-4-yl}-6-fluor-1,2-benzisoxazol (R,S), dessen optische Isomere und dessen Additionssalze mit einer pharmazeutisch annehmbaren anorganischen oder organischen Säure.

9. Verbindung der Formel I nach Anspruch 1, nämlich 6-Fluor-3-[1-(indan-1-yl-methyl)-piperid-4-yl]-1,2-benzisoxazol (R,S), dessen optische Isomere und dessen Additionssalze mit einer pharmazeutisch annehmbaren anorganischen oder organischen Säure.

**10.** Verfahren zur Herstellung der Verbindungen der Formel I nach Anspruch 1, **dadurch gekennzeichnet,** daß man:

entweder

eine Verbindung der Formel II:

$$R - (CH_2)_m - W \qquad (II)$$

in der R und m die bezüglich der Formel I in Anspruch 1 angegebenen Bedeutungen besitzen und W ein Halogenatom, eine Tosyloxygruppe oder eine Mesyloxygruppe bedeutet,

entweder

mit einer Verbindung der Formel III:

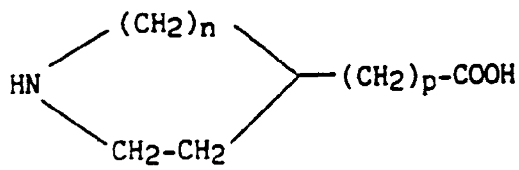

(III)

in der n und p die bezüglich der Formel I in Anspruch 1 angegebenen Bedeutungen besitzen, in Gegenwart von N,N-Diethylethylendiamin in Dimethylformamid oder einem anderen äquivalenten organischen Lösungsmittel umsetzt zur Bildung einer Verbindung der Formel IV:

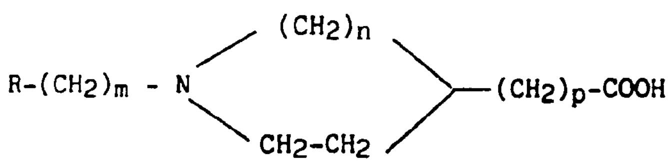

(IV)

in der R, m, n und p die bezüglich der Formel I in Anspruch 1 angegebenen Bedeutungen besitzen,

welche man der Einwirkung von Thionylchlorid oder Oxalylchlorid unterwirft zur Bildung eines Säurechlorids der Formel V:

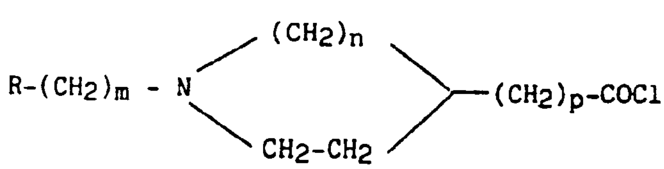

(V)

in der die Bedeutungen von R, m, n und p identisch sind zu den bezüglich der Formel I in Anspruch 1 angegebenen,

welches man in Gegenwart von Aluminiumchlorid mit einer Verbindung der Formel VI:

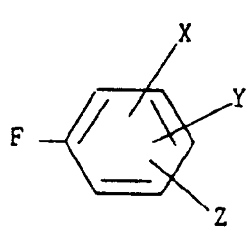

(VI)

in der X. Y, und Z die bezüglich der Formel I in Anspruch 1 angegebenen Bedeutungen besitzen, kondensiert

zur Bildung einer Verbindung der Formel VII:

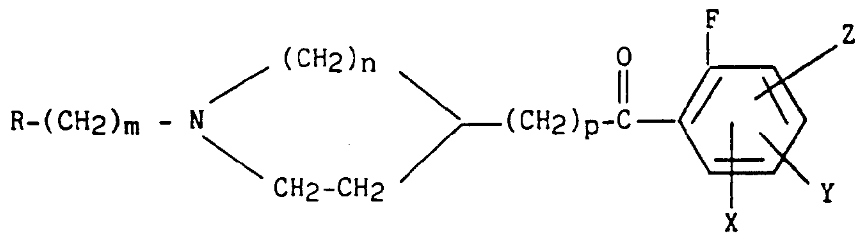

(VII)

in der R, X, Y, Z, m, n und p die bezüglich der Formel I in Anspruch 1 angegebenen Bedeutungen besitzen,

welche man der Einwirkung von Hydroxylamin-hydrochlorid in Gegenwart von 2-Diethylamino-ethylamin in alkoholischem Medium unterwirft

zur Bildung einer Verbindung der Formel VIII:

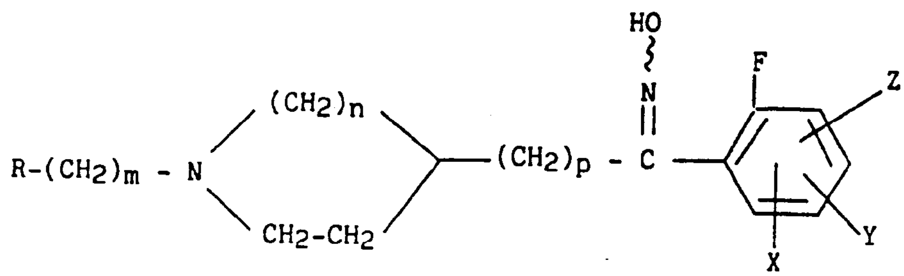

(VIII)

in der die Bedeutung von R, X, Y. Z, m, n und p identisch ist mit der bezüglich der Formel I in Anspruch 1 angegebenen,

welche man mit einer starken anorganischen Base cyclisiert zur Bildung der Verbindungen der Formel I gemäß Anspruch 1,

<u>oder</u>

mit einer Verbindung der Formel IX:

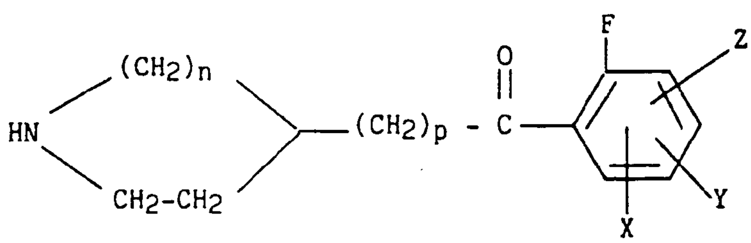

(IX)

in der X, Y, Z, n und p die bezüglich der Formel I in Anspruch 1 angegebenen Bedeutungen besitzen, in Gegenwart von N,N-Diethylethylendiamin in Dimethylformamid oder einem anderen äquivalenten organischen Lösungsmittel umsetzt zur Bildung der Verbindungen der Formel VII, ausgehend von welchen man unter Anwendung des oben angegebenen Verfahrens die Verbindungen der Formel VIII und dann die Verbindungen der Formel I gemäß Anspruch 1 erhält,

<u>oder</u>

mit einer Verbindung der Formel X:

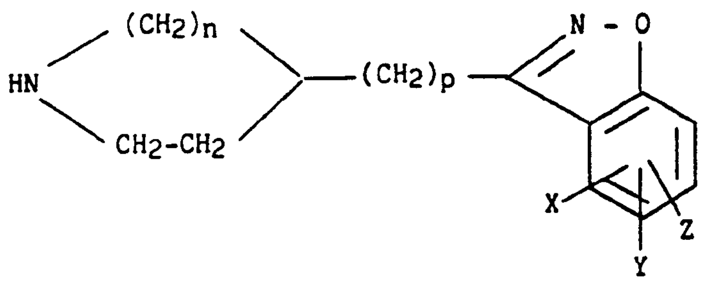

(X)

in der X, Y, Z, n und p die bezüglich der Formel I in Anspruch 1 angegebenen Bedeutungen besitzen, in Gegenwart von N,N-Diethylethylendiamin in Dimethylformamid oder einem anderen äquivalenten organischen Lösungsmittel umsetzt zur Bildung der Verbindungen der Formel I gemäß Anspruch 1,

oder

eine Verbindung der Formel $XI_A$ oder $XI_B$:

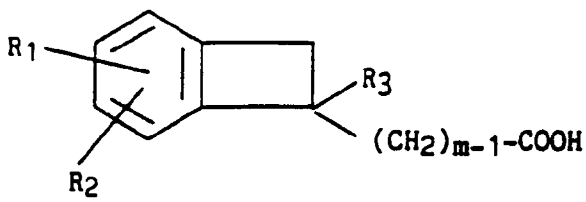

$(XI_A)$

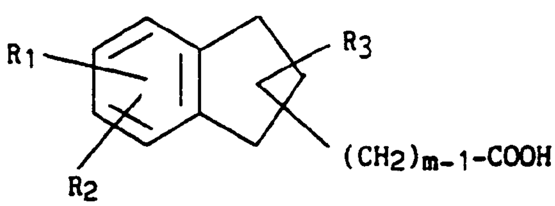

$(XI_B)$

worin $R_1$, $R_2$, $R_3$ und m die bezüglich der Formel I angegebenen Bedeutungen besitzen, mit der Maßgabe, daß m nicht den Wert 0 besitzen kann,

in Gegenwart von Carbonyldiimidazol mit einer Verbindung der Formel X umsetzt zur Bildung einer Verbindung der Formel $XII_A$ oder $XII_B$:

(XIIA)

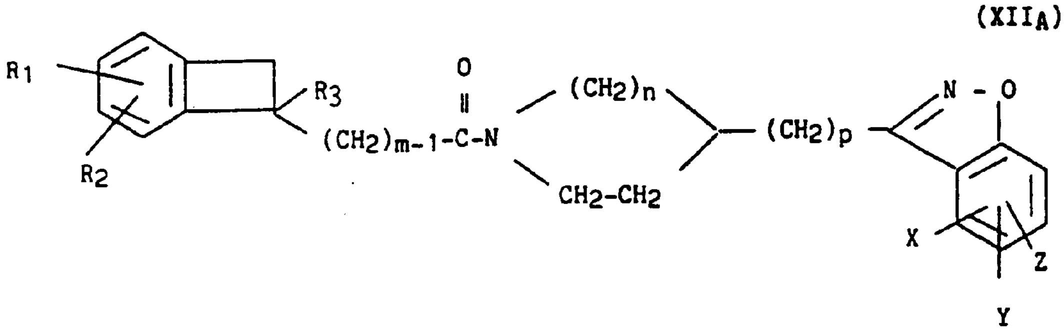

(XIIB)

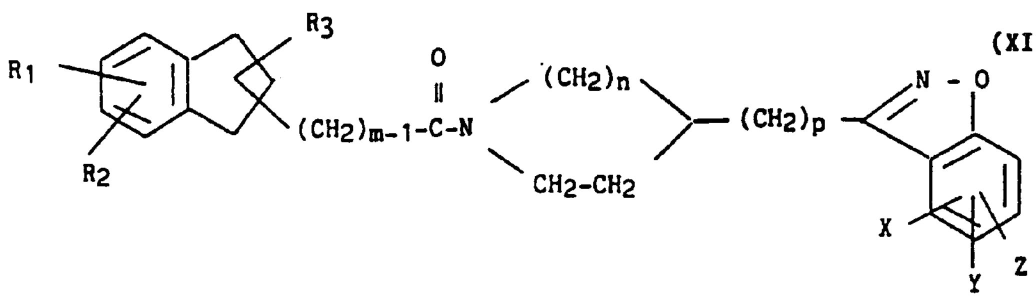

worin $R_1$, $R_2$, $R_3$, m, n, p, X, Y und Z die bezüglich der Formel I angegebenen Bedeutungen besitzen, mit der Maßgabe, daß m nicht die Zahl 0 bedeutet, welche man der Einwirkung von Lithiumaluminiumhydrid unterwirft zur Bildung der Verbindungen der Formel I,

oder

eine Verbindung der Formel XIII:

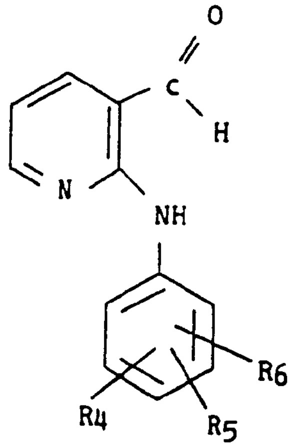

(XIII)

in der $R_4$, $R_5$ und $R_6$ die bezüglich der Formel I angegebenen Bedeutungen besitzen, mit einer Verbindung der Formel XIV:

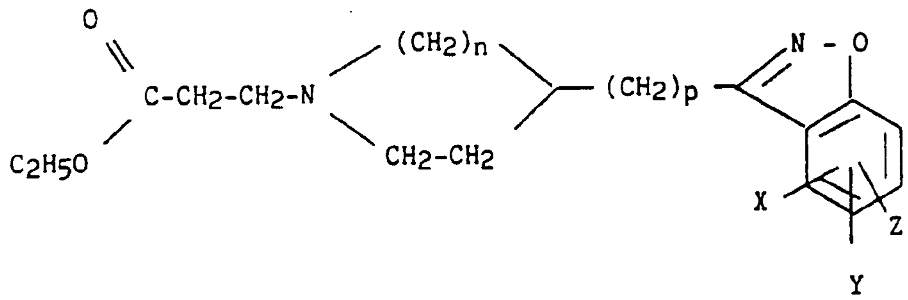

(XIV)

worin die Bedeutungen von n, p, X. Y und Z identisch sind mit den bezüglich der Formel I in Anspruch 1 angegebenen. umsetzt zur Bildung der Verbindungen der Formel I gemäß Anspruch 1, worin R eine Gruppe der Formel C darstellt und m den Wert 1 besitzt,

welche Verbindungen der Formel I, gemäß Anspruch 1

mit einer anorganischen oder organischen, pharmazeutisch annehmbaren Säure in die entsprechenden Salze überführt werden können,

oder

in ihre optischen Isomeren aufgetrennt und dann in ihre Salze umgewandelt werden können.

**11.** Pharmazeutische Zubereitung enthaltend als Wirkstoff eine Verbindung nach einem der Ansprüche 1 bis 9 in Kombination oder in Mischung mit einem inerten, nichttoxischen, pharmazeutisch annehmbaren Trägermaterial oder Bindemittel.

**12.** Pharmazeutische Zubereitung nach Anspruch 11, enthaltend den Wirkstoff in einer Dosis von 0,2 bis 100 mg.

**13.** Pharmazeutische Zubereitung nach den Ansprüchen 11 und 12 enthaltend als Wirkstoff mindestens eine Verbindung nach einem der Ansprüche 1 bis 9 zur Verwendung bei der Behandlung von Erkrankungen, welche die Anwendung von antipsychotischen Mitteln, Sedativa, anxiolytischen Mitteln, antiaggressiven Mitteln und analgetischen Mitteln erforderlich machen oder zur Behandlung der Schizophrenie und von Depressionen.

**Patentanspruch für folgenden Vertragsstaat : GR**

**1.** Verfahren zur Herstellung der Verbindungen der Formel I:

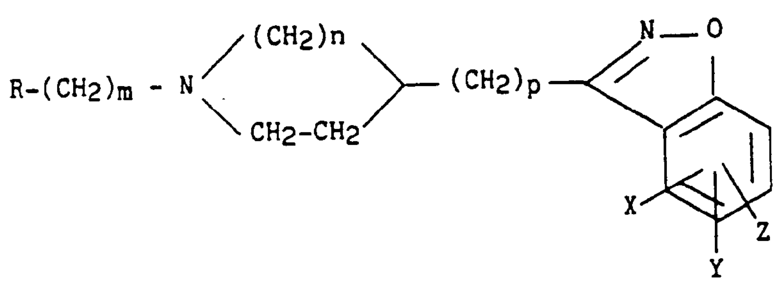

(I)

in der

- m eine ganze Zahl mit einem Wert von 0 bis 5,
- n eine ganze Zahl mit einem Wert von 1 bis 2,
- p 0, 1 oder 2,
- X, Y und Z, die gleichartig oder verschieden sein können, jeweils ein Wasserstoffatom, ein Halogenatom, eine geradkettige oder verzweigte Alkylgruppe mit 1 bis 6 Kohlenstoffatomen, eine Trifluormethylgruppe, eine Alkoxygruppe mit 1 bis 6 Kohlenstoffatomen, eine Alkylthiogruppe mit 1 bis 6

Kohlenstoffatomen oder eine Hydroxylgruppe,

- R eine Benzofuran-2-yl-gruppe oder eine 2,3-Dihydrobenzofuran-2-yl-gruppe (die jeweils am Benzolring durch ein oder mehrere Halogenatome, Alkoxygruppen mit 1 bis 6 Kohlenstoffatomen, Alkylthiogruppen mit 1 bis 6 Kohlenstoffatomen oder geradkettigen oder verzweigten Alkylgruppen mit 1 bis 6 Kohlenstoffatomen substituiert sein können), eine 2,3,6,7-Tetrahydro-benzo-[1,2-b:4,5b']difuran-2-yl-gruppe, eine 4-Oxo-4H-chromen-2-yl-gruppe (die gegebenenfalls am Benzolring durch ein oder mehrere Halogenatome, geradkettige oder verzweigte Alkylgruppen mit 1 bis 6 Kohlenstoffatomen oder Alkoxygruppen mit 1 bis 6 Kohlenstoffatomen substituiert sein kann), eine Benzocyclobutenyl-gruppe der Formel A oder eine Indanylgruppe der Formel B:

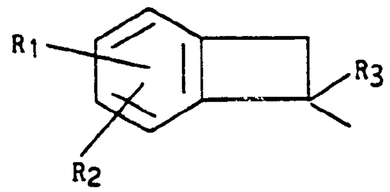

(A)

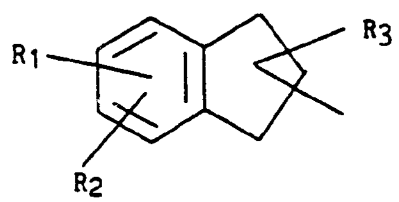

(B)

(worin:

$R_1$ und $R_2$, die gleichartig oder verschieden sein können, jeweils ein Wasserstoffatom, ein Halogenatom, eine Trifluormethylgruppe, eine geradkettige oder verzweigte Alkylgruppe mit 1 bis 6 Kohlenstoffatomen, eine Alkoxygruppe mit 1 bis 6 Kohlenstoffatomen, eine Hydroxylgruppe. eine Hydroxyalkylgruppe mit 1 bis 6 Kohlenstoffatomen, eine Alkylthiogruppe mit 1 bis 6 Kohlenstoffatomen oder gemeinsam eine Methylendioxygruppe oder eine Ethylendioxygruppe und

$R_3$ ein Wasserstoffatom oder eine geradkettige oder verzweigte Alkylgruppe mit 1 bis 6 Kohlenstoffatomen darstellen),

oder eine Gruppe der Formel C:

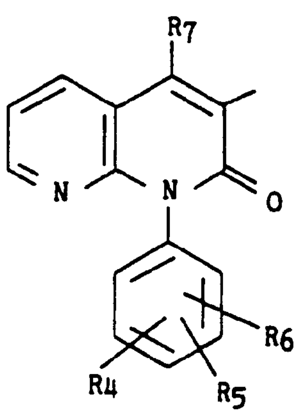

(C)

(in der

$R_4$, $R_5$ und $R_6$, die gleichartig oder verschieden sein können. jeweils ein Wasserstoffatom, ein Halogenatom. eine Trifluormethylgruppe, eine Hydroxylgruppe. eine geradkettige oder verzweigte Alkylgruppe mit 1 bis 6 Kohlenstoffatomen, eine Alkoxygruppe mit 1 bis 6 Kohlenstoffatomen oder

eine Alkylthiogruppe mit 1 bis 6 Kohlenstoffatomen und

$R_7$ ein Wasserstoffatom oder eine Hydroxylgruppe darstellen) bedeuten, von deren optischen Isomeren und von deren Additionssalzen mit einer pharmazeutisch annehmbaren organischen oder anorganischen Säure, **dadurch gekennzeichnet,** daß man

entweder

A) eine Verbindung der Formel II:

$$R - (CH_2)_m - W \qquad (II)$$

in der R und m die bezüglich der Formel I angegebenen Bedeutungen besitzen und W ein Halogenatom, eine Tosyloxygruppe oder eine Mesyloxygruppe bedeutet,

entweder

a) mit einer Verbindung der Formel III:

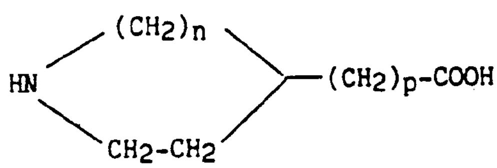

(III)

in der n und p die bezüglich der Formel I angegebenen Bedeutungen besitzen, in Gegenwart von N,N-Diethylethylendiamin in Dimethylformamid oder einem anderen äquivalenten organischen Lösungsmittel umsetzt zur Bildung einer Verbindung der Formel IV:

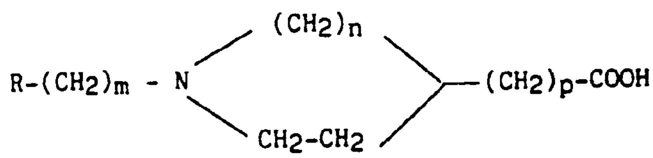

(IV)

in der R, m, n und p die bezüglich der Formel I angegebenen Bedeutungen besitzen,

welche man der Einwirkung von Thionylchlorid oder Oxalylchlorid unterwirft zur Bildung eines Säurechlorids der Formel V:

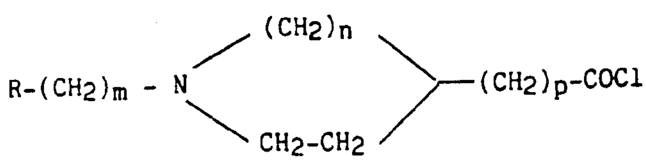

(V)

in der die Bedeutungen von R, m, n und p identisch sind zu denen bezüglich der Formel I angegebenen,

welches man in Gegenwart von Aluminiumchlorid mit einer Verbindung der Formel VI:

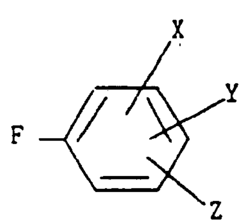

(VI)

in der X, Y, und Z die bezüglich der Formel I angegebenen Bedeutungen besitzen, kondensiert zur Bildung einer Verbindung der Formel VII:

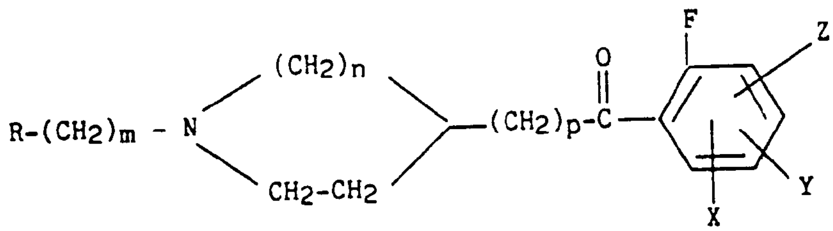

(VII)

in der R, X, Y, Z, m, n und p die bezüglich der Formel I angegebenen Bedeutungen besitzen, welche man der Einwirkung von Hydroxylamin-hydrochlorid in Gegenwart von 2-Diethylamino-ethylamin in alkoholischem Medium unterwirft zur Bildung einer Verbindung der Formel VIII:

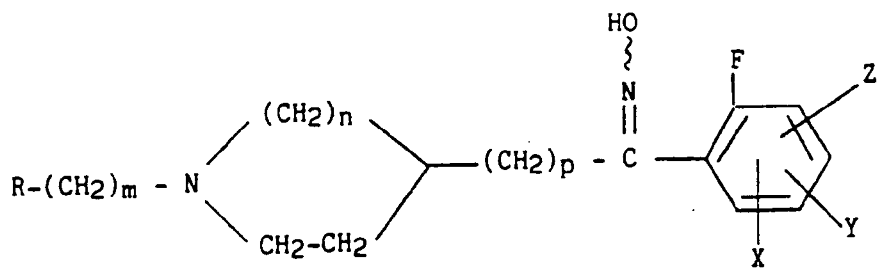

(VIII)

in der die Bedeutung von R, X, Y, Z, m, n und p identisch ist mit der bezüglich der Formel I angegebenen, welche man mit einer starken anorganischen Base cyclisiert zur Bildung der Verbindungen der Formel I,

oder

b) mit einer Verbindung der Formel IX:

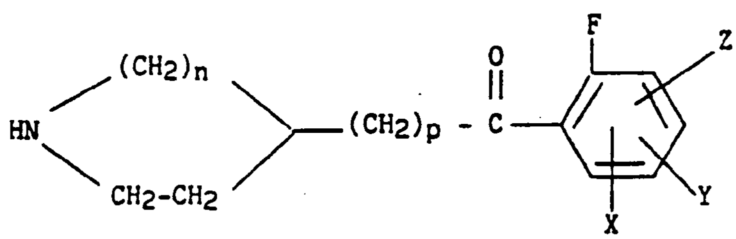

(IX)

in der X. Y, Z, n und p die bezüglich der Formel I angegebenen Bedeutungen besitzen, in Gegenwart von N,N-Diethylethylendiamin in Dimethylformamid oder einem anderen äquivalenten organischen Lösungsmittel umsetzt zur Bildung der Verbindungen der Formel VII, ausgehend von welchen man unter Anwendung des oben angegebenen Verfahrens die Verbindungen der Formel VIII und dann die Verbindungen der Formel I erhält,

oder

c) mit einer Verbindung der Formel X:

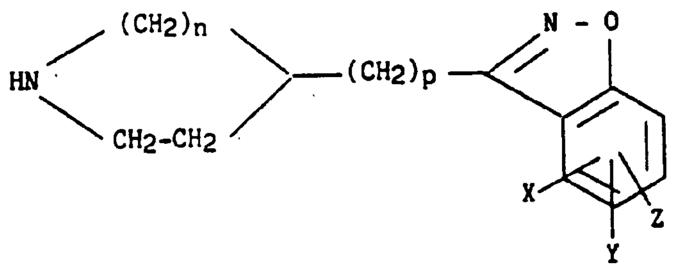

(X)

in der X, Y, Z, n und p die bezüglich der Formel I angegebenen Bedeutungen besitzen, in Gegenwart von N,N-Diethylethylendiamin in Dimethylformamid oder einem anderen äquivalenten organischen Lösungsmittel umsetzt zur Bildung der Verbindungen der Formel I,

**oder**

B) eine Verbindung der Formel $XI_A$ oder $XI_B$:

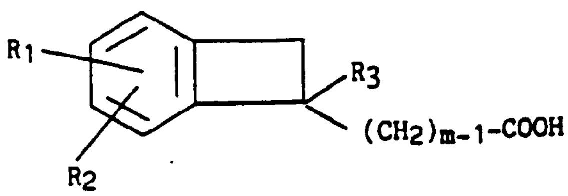

($XI_A$)

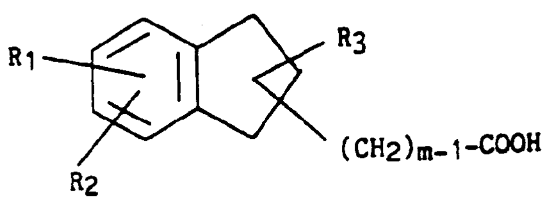

($XI_B$)

worin $R_1$, $R_2$, $R_3$ und m die bezüglich der Formel I angegebenen Bedeutungen besitzen, mit der Maßgabe, daß m nicht den Wert 0 besitzen kann,

in Gegenwart von Carbonyldiimidazol mit einer Verbindung der Formel X umsetzt zur Bildung einer Verbindung der Formel $XII_A$ oder $XII_B$:

($XII_A$)

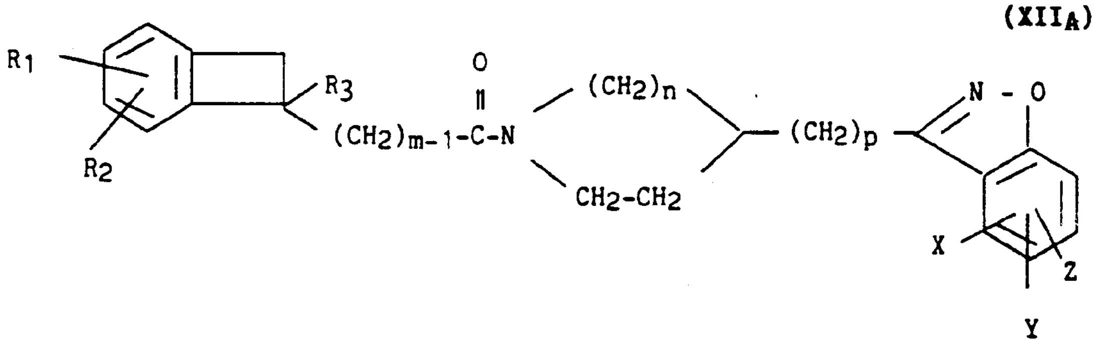

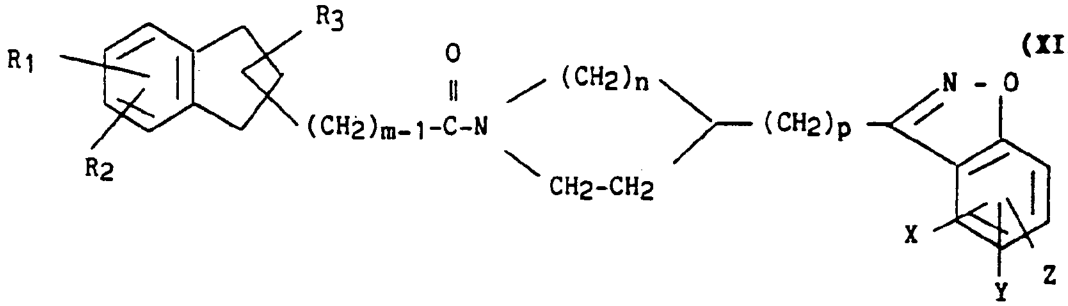

(XIIB)

worin $R_1$, $R_2$, $R_3$, m, n, p. X. Y und Z die bezüglich der Formel I angegebenen Bedeutungen besitzen, mit der Maßgabe, daß m nicht die Zahl 0 bedeutet, welche man der Einwirkung von Lithiumaluminiumhydrid unterwirft zur Bildung der Verbindungen der Formel I,

oder

C) eine Verbindung der Formel XIII:

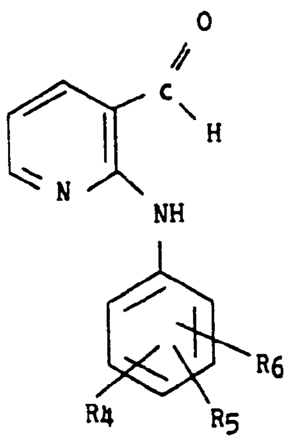

(XIII)

in der $R_4$, $R_5$ und $R_5$ die bezüglich der Formel I angegebenen Bedeutungen besitzen, mit einer Verbindung der Formel XIV:

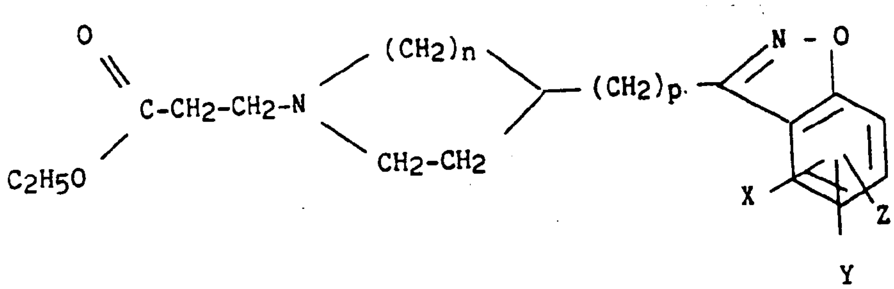

(XIV).

worin die Bedeutungen von n, p, X, Y und Z identisch sind mit den bezüglich der Formel I angegebenen. umsetzt zur Bildung der Verbindungen der Formel I, worin R eine Gruppe der Formel C darstellt und m den Wert 1 besitzt,

welche Verbindungen der Formel I

mit einer anorganischen oder organischen, pharmazeutisch annehmbaren Säure in die entsprechenden Salze überführt werden können,
oder
in ihre optischen Isomeren aufgetrennt und dann in ihre Salze umgewandelt werden können.

**Patentanspruch für folgenden Vertragsstaat : ES**

1. Verfahren zur Herstellung der Verbindungen der Formel I:

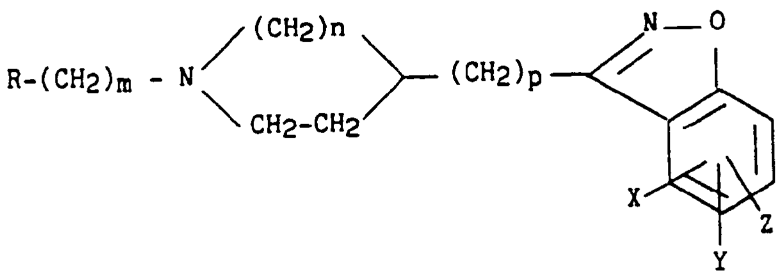

(I)

in der

- m eine ganze Zahl mit einem Wert von 0 bis 5,
- n eine ganze Zahl mit einem Wert von 1 bis 2,
- p 0, 1 oder 2,
- X, Y und Z, die gleichartig oder verschieden sein können, jeweils ein Wasserstoffatom, ein Halogenatom, eine geradkettige oder verzweigte Alkylgruppe mit 1 bis 6 Kohlenstoffatomen, eine Trifluormethylgruppe, eine Alkoxygruppe mit 1 bis 6 Kohlenstoffatomen, eine Alkylthiogruppe mit 1 bis 6 Kohlenstoffatomen oder eine Hydroxylgruppe.
- R eine Benzofuran-2-yl-gruppe oder eine 2,3-Dihydrobenzofuran-2-yl-gruppe (die jeweils am Benzolring durch ein oder mehrere Halogenatome, Alkoxygruppen mit 1 bis 6 Kohlenstoffatomen, Alkylthiogruppen mit 1 bis 6 Kohlenstoffatomen oder geradkettigen oder verzweigten Alkylgruppen mit 1 bis 6 Kohlenstoffatomen substituiert sein können), eine 2,3,6,7-Tetrahydro-benzo-[1,2-b:4,5b′]difuran-2-yl-gruppe, eine 4-Oxo-4H-chromen-2-yl-gruppe (die gegebenenfalls am Benzolring durch ein oder mehrere Halogenatome, geradkettige oder verzweigte Alkylgruppen mit 1 bis 6 Kohlenstoffatomen oder Alkoxygruppen mit 1 bis 6 Kohlenstoffatomen substituiert sein kann). eine Benzo-cyclobutenyl-gruppe der Formel A oder eine Indanylgruppe der Formel B:

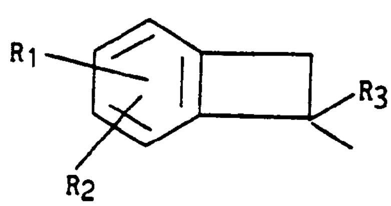

(A)

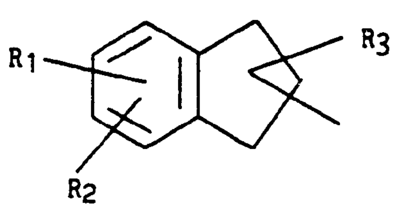

(B)

(worin:

$R_1$ und $R_2$, die gleichartig oder verschieden sein können, jeweils ein Wasserstoffatom, ein Halogenatom, eine Trifluormethylgruppe, eine geradkettige oder verzweigte Alkylgruppe mit 1 bis 6 Kohlenstoffatomen, eine Alkoxygruppe mit 1 bis 6 Kohlenstoffatomen, eine Hydroxylgruppe. eine Hydroxyalkylgruppe mit 1 bis 6 Kohlenstoffatomen, eine Alkylthiogruppe mit 1 bis 6 Kohlenstoffatomen oder gemeinsam eine Methylendioxygruppe oder eine Ethylendioxygruppe und

$R_3$ ein Wasserstoffatom oder eine geradkettige oder verzweigte Alkylgruppe mit 1 bis 6 Kohlenstoffatomen darstellen).

oder eine Gruppe der Formel C:

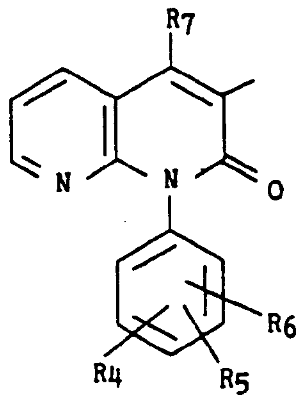

(C)

(in der

$R_4$, $R_5$ und $R_6$, die gleichartig oder verschieden sein können, jeweils ein Wasserstoffatom, ein Halogenatom, eine Trifluormethylgruppe, eine Hydroxylgruppe, eine geradkettige oder verzweigte Alkylgruppe mit 1 bis 6 Kohlenstoffatomen, eine Alkoxygruppe mit 1 bis 6 Kohlenstoffatomen oder eine Alkylthiogruppe mit 1 bis 6 Kohlenstoffatomen und

$R_7$ ein Wasserstoffatom oder eine Hydroxylgruppe darstellen) bedeuten, von deren optischen Isomeren und von deren Additionssalzen mit einer pharmazeutisch annehmbaren organischen oder anorganischen Säure, **dadurch gekennzeichnet,** daß man

entweder

A) eine Verbindung der Formel II:

$$R - (CH_2)_m - W \qquad (II)$$

in der R und m die bezüglich der Formel I angegebenen Bedeutungen besitzen und W ein Halogenatom, eine Tosyloxygruppe oder eine Mesyloxygruppe bedeutet,

entweder

a) mit einer Verbindung der Formel III:

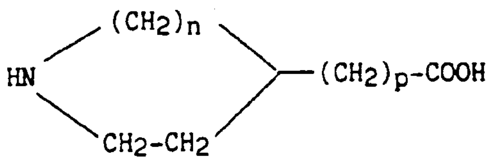

(III)

in der n und p die bezüglich der Formel I angegebenen Bedeutungen besitzen, in Gegenwart von N,N-Diethylethylendiamin in Dimethylformamid oder einem anderen äquivalenten organischen Lösungsmittel umsetzt zur Bildung einer Verbindung der Formel IV:

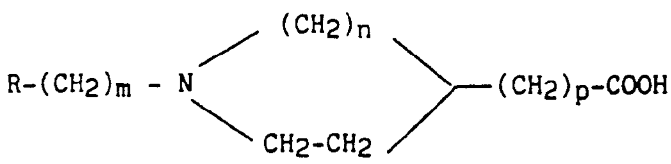

(IV)

in der R, m, n und p die bezüglich der Formel I angegebenen Bedeutungen besitzen,

welche man der Einwirkung von Thionylchlorid oder Oxalylchlorid unterwirft zur Bildung eines Säurechlorids der Formel V:

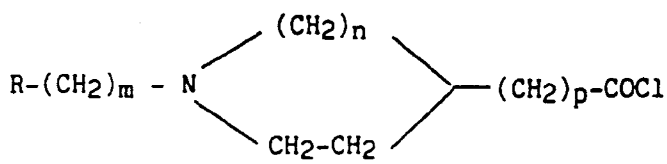

(V)

in der die Bedeutungen von R, m, n und p identisch sind zu denen bezüglich der Formel I angegebenen,

welches man in Gegenwart von Aluminiumchlorid mit einer Verbindung der Formel VI:

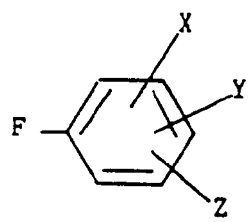

(VI)

in der X, Y, und Z die bezüglich der Formel I angegebenen Bedeutungen besitzen, kondensiert

zur Bildung einer Verbindung der Formel VII:

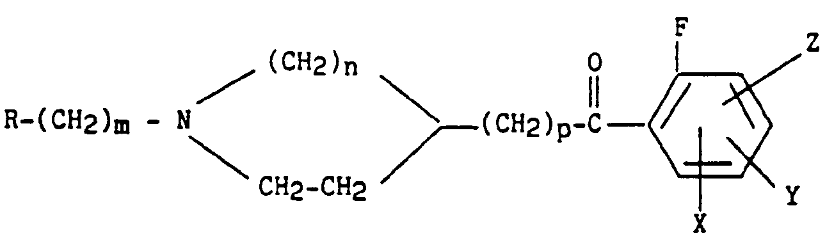

(VII)

in der R, X, Y, Z, m, n und p die bezüglich der Formel I angegebenen Bedeutungen besitzen,

welche man der Einwirkung von Hydroxylamin-hydrochlorid in Gegenwart von 2-Diethylamino-ethylamin in alkoholischem Medium unterwirft

zur Bildung einer Verbindung der Formel VIII:

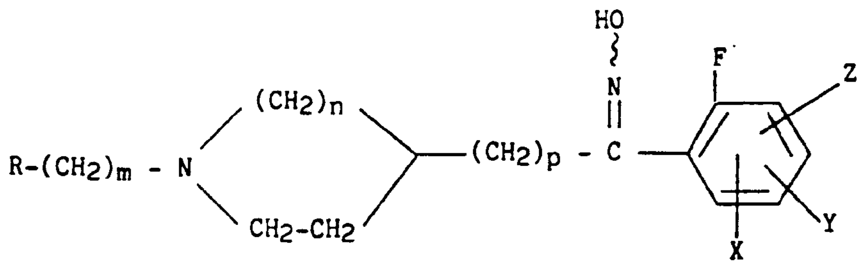

(VIII)

in der die Bedeutung von R, X, Y, Z, m, n und p identisch ist mit der bezüglich der Formel I angegebenen,

welche man mit einer starken anorganischen Base cycllsiert zur Bildung der Verbindungen der Formel I,

oder

b) mit einer Verbindung der Formel IX:

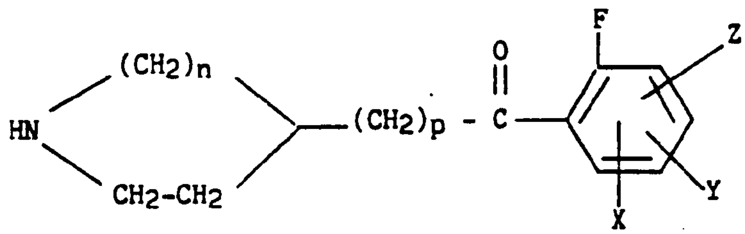

(IX)

in der X, Y, Z, n und p die bezüglich der Formel I angegebenen Bedeutungen besitzen, in Gegenwart von N,N-Diethylethylendiamin in Dimethylformamid oder einem anderen äquivalenten organischen Lösungsmittel umsetzt zur Bildung der Verbindungen der Formel VII. ausgehend von welchen man unter Anwendung des oben angegebenen Verfahrens die Verbindungen der Formel VIII und dann die Verbindungen der Formel I erhält,

oder

c) mit einer Verbindung der Formel X:

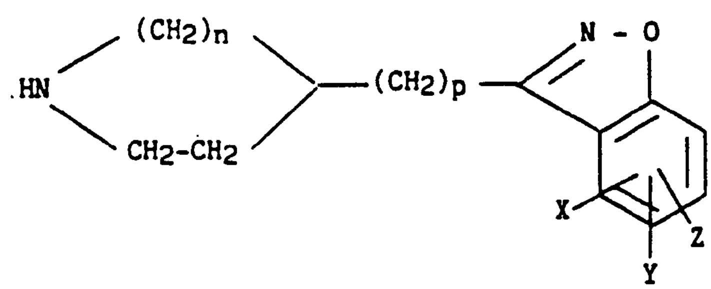

(X)

in der X, Y, Z, n und p die bezüglich der Formel I angegebenen Bedeutungen besitzen, in Gegenwart von N,N-Diethylethylendiamin in Dimethylformamid oder einem anderen äquivalenten organischen Lösungsmittel umsetzt zur Bildung der Verbindungen der Formel I,

oder

B) eine Verbindung der Formel $XI_A$ oder $XI_B$:

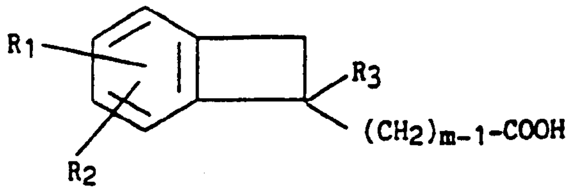

(XIA)

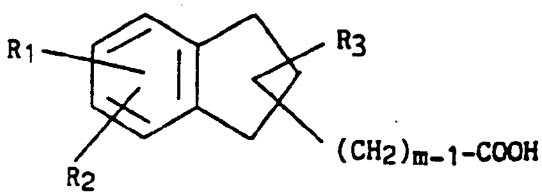

(XIB)

worin $R_1$, $R_2$, $R_3$ und m die bezüglich der Formel I angegebenen Bedeutungen besitzen, mit der Maßgabe, daß m nicht den Wert 0 besitzen kann,

in Gegenwart von Carbonyldiimidazol mit einer Verbindung der Formel X umsetzt zur Bildung einer Verbindung der Formel $XII_A$ oder $XII_B$:

(XIIA)

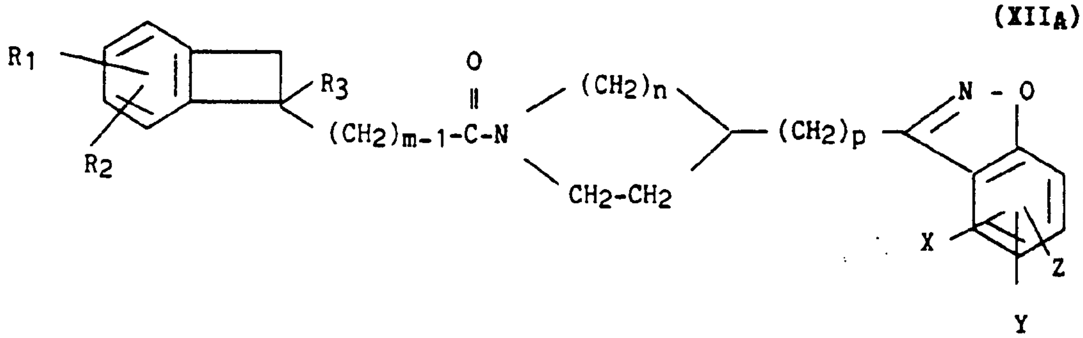

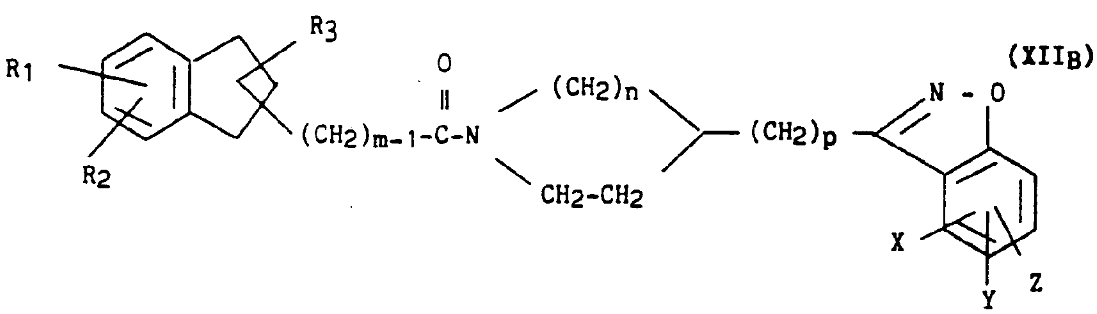

(XIIB)

worin $R_1$, $R_2$, $R_3$, m, n, p, X. Y und Z die bezüglich der Formel I angegebenen Bedeutungen besitzen, mit der Maßgabe, daß m nicht die Zahl 0 bedeutet, welche man der Einwirkung von Lithiumaluminiumhydrid unterwirft zur Bildung der Verbindungen der Formel I,

oder

C) eine Verbindung der Formel XIII:

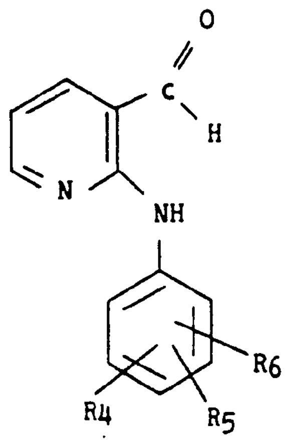

(XIII)

in der $R_4$, $R_5$ und $R_5$ die bezüglich der Formel I angegebenen Bedeutungen besitzen, mit einer Verbindung der Formel XIV:

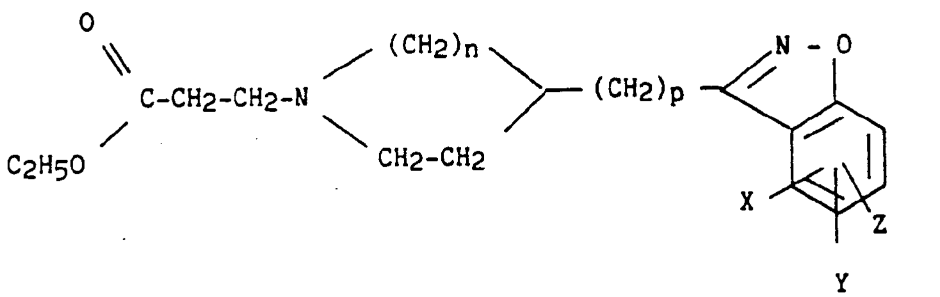

(XIV)

worin die Bedeutungen von n, p, X, Y und Z identisch sind mit den bezüglich der Formel I angegebenen. umsetzt zur Bildung der Verbindungen der Formel I, worin R eine Gruppe der Formel C darstellt und m den Wert 1 besitzt,

welche Verbindungen der Formel I

mit einer anorganischen oder organischen, pharmazeutisch annehmbaren Säure in die entsprechenden Salze überführt werden können,
oder
in ihre optischen Isomeren aufgetrennt und dann in ihre Salze umgewandelt werden können.

## Claims

**Claims for the following Contracting States : AT, BE, CH, DE, DK, FR, GB, IT, LI, LU, NL, SE**

**1.** Compounds of formula I:

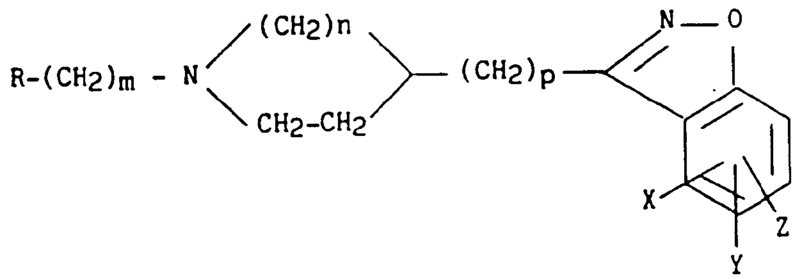

(I)

wherein :

- m represents an integer of from 0 to 5,
- n represents an integer 1 or 2,
- p is equal to 0, 1 or 2,
- X, Y and Z, which are the same or different, each represents a hydrogen atom, a halogen atom, a straight-chain or branched alkyl radical having from 1 to 6 carbon atoms, a trifluoromethyl radical, an alkoxy radical having from 1 to 6 carbon atoms, an alkylthio radical having from 1 to 6 carbon atoms or a hydroxy radical,
- R represents a benzofuran-2-yl or 2,3-dihydrobenzofuran-2-yl radical (each of which may be substituted in the benzene ring by one or more halogen atoms, by alkoxy radicals having from 1 to 6 carbon atoms, by alkylthio radicals having from 1 to 6 carbon atoms, or by straight-chain or branched alkyl radicals having from 1 to 6 carbon atoms), a 2,3,6,7-tetrahydrobenzo[1,2-b:4,5b']difuran-2-yl radical, a 4-oxo-4H-chromen-2-yl radical (optionally substituted in the benzene ring by one or more halogen atoms, by straight-chain or branched alkyl radicals having from 1 to 6 carbon atoms or by alkoxy radicals having from 1 to 6 carbon atoms), a benzocyclobutenyl radical of formula A or an indanyl radical of formula B :

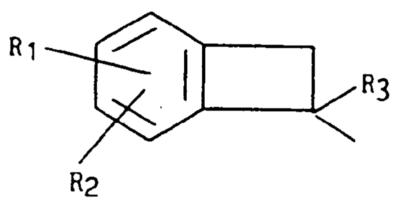

(A)

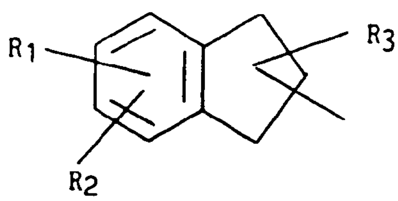

(B)

(wherein:

$R_1$ and $R_2$, which are the same or different, each represents a hydrogen atom, a halogen atom, a trifluoromethyl radical, a straight-chain or branched alkyl radical having from 1 to 6 carbon atoms, an alkoxy radical having from 1 to 6 carbon atoms, a hydroxy radical, a hydroxyalkyl radical having from 1 to 6 carbon atoms or an alkylthio radical having from 1 to 6 carbon atoms, or together form a methylenedioxy radical or an ethylenedioxy radical, and

$R_3$ represents a hydrogen atom or a straight-chain or branched alkyl radical having from 1 to 6 carbon atoms),

or a radical of formula C :

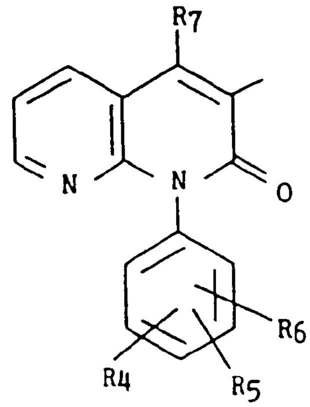

(C)

(wherein

$R_4$, $R_5$ and $R_6$, which are the same or different, each represents a hydrogen atom, a halogen atom, a trifluoromethyl radical, a hydroxy radical, a straight-chain or branched alkyl radical having from 1 to 6 carbon atoms, an alkoxy radical having from 1 to 6 carbon atoms, or an alkylthio radical having from 1 to 6 carbon atoms, and

$R_7$ represents a hydrogen atom or a hydroxy radical),

the optical isomers thereof and the addition salts thereof with a pharmaceutically acceptable organic or mineral acid.

2. (R,S)-3-[1-(benzocyclobuten-1-ylmethyl)-piperid-4-yl]-6-fluoro-1,2-benzisoxazole, a compound of formula I according to claim 1, and the addition salts thereof with a pharmaceutically acceptable mineral or organic acid.

3. (+)-3-[1-(benzocyclobuten-1-ylmethyl)-piperid-4-yl]-6-fluoro-1,2-benzisoxazole, a compound of formula I according to claim 1, and the addition salts thereof with a pharmaceutically acceptable mineral or organic acid.

4. (-)-3-[1-(benzocyclobuten-1-ylmethyl)-piperid-4-yl]-6-fluoro-1,2-benzisoxazole, a compound of formula I according to claim 1, and the addition salts thereof with a pharmaceutically acceptable mineral or organic acid.

5. 3-{1-{[1,2-dihydro-2-oxo-1-(3-trifluoromethylphenyl)-1,8-naphthyridin-3-yl]-ethyl}-piperid-4-yl}-6-fluoro-1,2-benzisoxazole, a compound of formula I according to claim 1, and the addition salts thereof with a pharmaceutically acceptable mineral or organic acid.

6. (R,S)-3-[1-(2-benzocyclobuten-1-ylethyl)-piperid-4-yl]-6-fluoro-1,2-benzisoxazole, a compound of formula I according to claim 1, the optical isomers thereof and the addition salts thereof with a pharmaceutically acceptable mineral or organic acid.

7. (R,S)-6-fluoro-3-{1-[(1-methylbenzocyclobuten-1-yl)-methyl]-piperid-4-yl}-1,2-benzisoxazole, a compound of formula I according to claim 1, the optical isomers thereof and the addition salts thereof with a pharmaceutically acceptable mineral or organic acid.

8. (R,S)-3-{1-[(4,5-dimethoxybenzocyclobuten-1-yl)methyl]-piperid-4-yl}-6-fluoro-1,2-benzisoxazole, a compound of formula I according to claim 1, the optical isomers thereof and the addition salts thereof with a pharmaceutically acceptable mineral or organic acid.

9. (R,S)-6-fluoro-3-[1-(indan-1-ylmethyl)-piperid-4-yl]-1,2-benzisoxazole, a compound of formula I according to claim 1, the optical isomers thereof and the addition salts thereof with a pharmaceutically acceptable mineral or organic acid.

10. A process for the preparation of compounds of formula I according to claim 1, characterised in that :

either

a compound of formula II :

$$R - (CH_2)_m - W \qquad (II)$$

wherein R and m are as defined for formula I according to claim 1 and W represents a halogen atom or a tosyloxy radical or a mesyloxy radical, is reacted

either

with a compound of formula III :

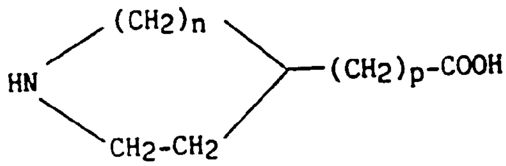

(III)

wherein n and p are as defined for formula I according to claim 1, in the presence of N,N-diethylethylenediamine in dimethylformamide or another equivalent organic solvent,

to form a compound of formula IV :

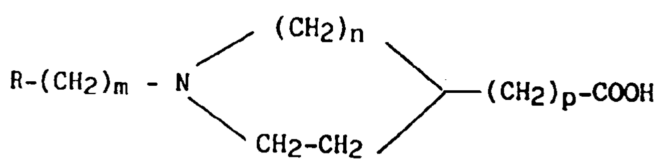

(IV)

wherein R, m, n and p are as defined for formula I according to claim 1,

which is subjected to the action of thionyl chloride or oxalyl chloride to form an acid chloride of formula V:

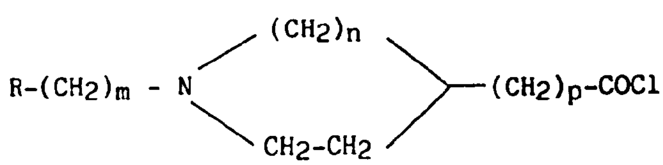

(V)

wherein the meanings of R, m, n and p remain identical to those given above for formula I according to claim 1,

which is condensed in the presence of aluminium chloride with a compound of formula VI :

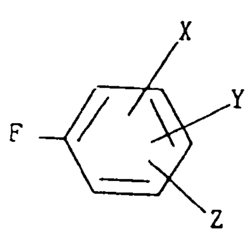

(VI)

wherein X, Y and Z are as defined for formula I according to claim 1,

to form a compound of formula VII :

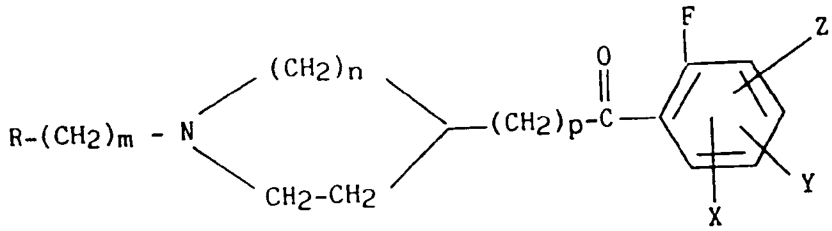

(VII)

wherein R, X, Y, Z, m, n and p are as defined for formula I according to claim 1,

which is subjected to the action of hydroxylamine hydrochloride in the presence of 2-diethylaminoethylamine in alcoholic medium,

to obtain a compound of formula VIII :

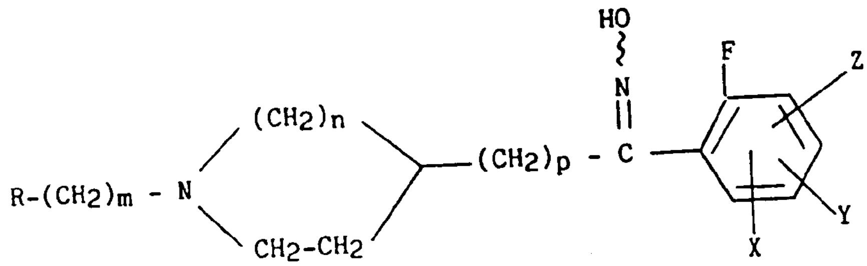

(VIII)

wherein the meanings of R, X, Y, Z, m, n and p remain identical to those given for formula I according to claim 1,

which is cyclised with the aid of a strong mineral base to obtain the compounds of formula I according to claim 1,

or

with a compound of formula IX :

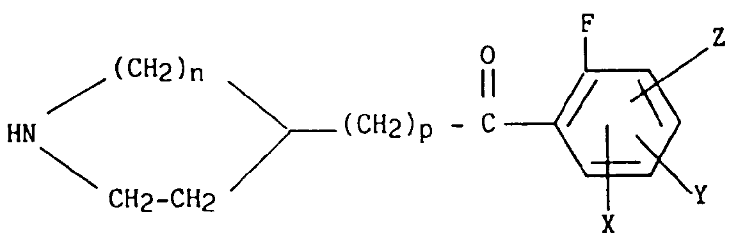

(IX)

wherein X, Y, Z, n and p are as defined for formula I according to claim 1, in the presence of N,N-diethylethylenediamine in dimethylformamide or another equivalent organic solvent,

to form the compounds of formula VII, from which the compounds of formula VIII and then the compounds of formula I according to claim 1 are obtained using the process indicated above,

or

with a compound of formula X :

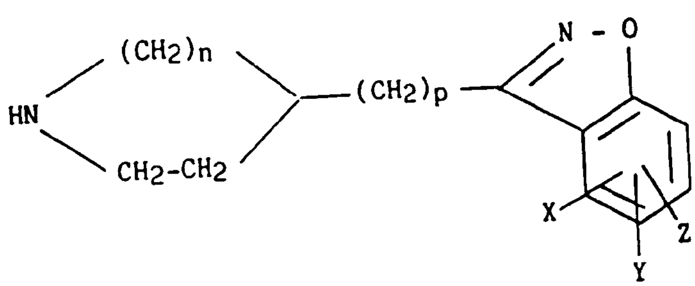

(X)

wherein X, Y, Z, n and p are as defined for formula I according to claim 1, in the presence of N,N-diethylethylenediamine in dimethylformamide or another equivalent organic solvent,

to form the compounds of formula I according to claim 1,

or

a compound of formula $XI_A$ or $XI_B$ :

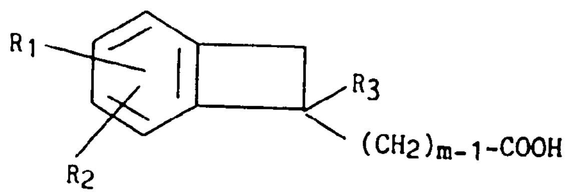

$(XI_A)$

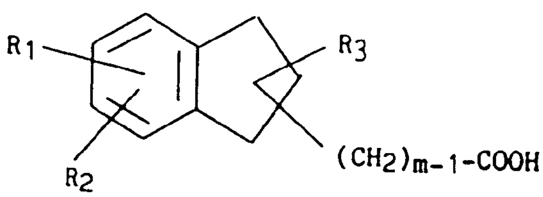

$(XI_B)$

wherein $R_1$, $R_2$, $R_3$ and m are as defined for formula I, except that in this case m cannot represent the number 0,

is reacted with a compound of formula X in the presence of carbonyldiimidazole to form a compound of formula XIIA or XIIB :

$(XII_A)$

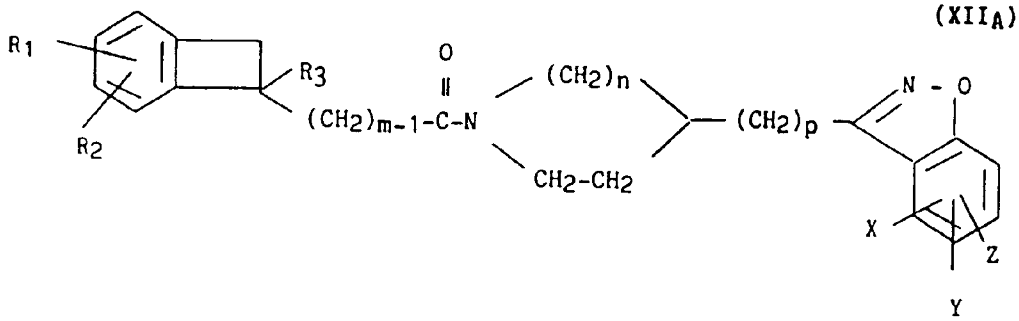

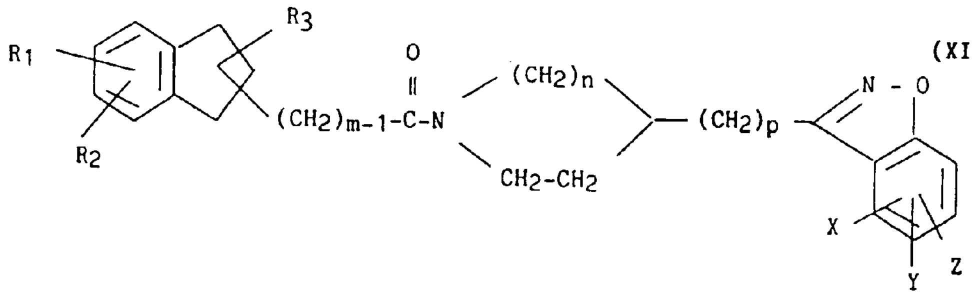

(XIIB)

wherein $R_1$, $R_2$, $R_3$, m, n, p, X, Y and Z are as defined for formula I except that in this case m does not represent the number 0,
which is subjected to the action of lithium aluminium hydride to form the compounds of formula I

| or |
|---|

a compound of formula XIII :

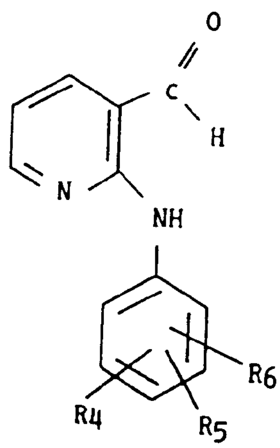

(XIII)

wherein $R_4$, $R_5$ and $R_6$ are as defined for formula I, is reacted with a compound of formula XIV :

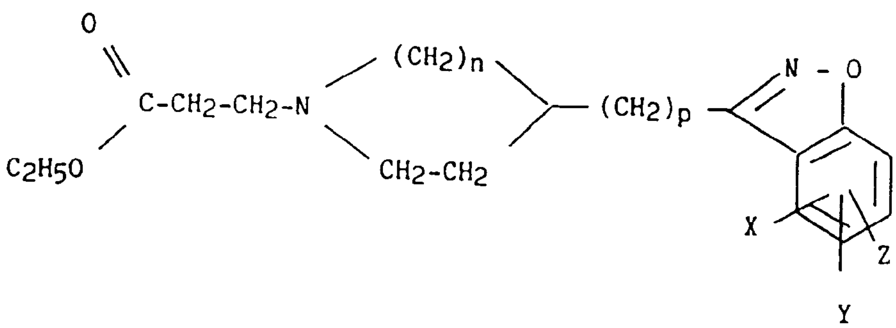

(XIV)

wherein the meanings of n, p, X, Y and Z remain identical to those given for formula I according to claim

1, to form the compounds of formula I according to claim 1 wherein R represents a radical of formula C and m is 1,

| which compounds of formula I according to claim 1 |
|---|

may be converted into salts with a pharmaceutically acceptable mineral or organic acid to form the corresponding salts or
may be separated into their optical isomers and then converted into salts.

**11.** Pharmaceutical composition comprising as active ingredient a compound according to any one of claims 1 to 9, in association or mixture with a pharmaceutically acceptable, inert, non-toxic excipient or carrier.

**12.** Pharmaceutical composition according to claim 11 containing the active ingredient in an amount of from 0.2 to 100 mg.

**13.** Pharmaceutical composition according to claims 11 and 12 containing as active ingredient at least one compound according to claims 1 to 9, which can be used in the treatment of disorders requiring antipsychotic, sedative, anxiolytic, antiagressive and analgesic agents, or in the treatment of schizophrenia and depression.

**Claim for the following Contracting State : GR**

**1.** A process for the preparation of compounds of formula I:

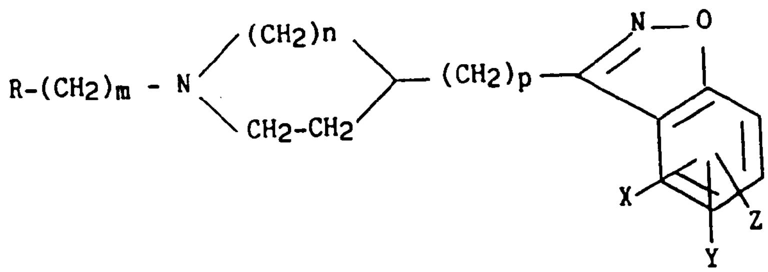

(I)

wherein :
- m represents an integer of from 0 to 5,
- n represents an integer 1 or 2,
- p is equal to 0, 1 or 2,
- X, Y and Z, which are the same or different, each represents a hydrogen atom, a halogen atom, a straight-chain or branched alkyl radical having from 1 to 6 carbon atoms, a trifluoromethyl radical, an alkoxy radical having from 1 to 6 carbon atoms, an alkylthio radical having from 1 to 6 carbon atoms or a hydroxy radical,
- R represents a benzofuran-2-yl or 2,3-dihydrobenzofuran-2-yl radical (each of which may be substituted in the benzene ring by one or more halogen atoms, by alkoxy radicals having from 1 to 6 carbon atoms, by alkylthio radicals having from 1 to 6 carbon atoms, or by straight-chain or branched alkyl radicals having from 1 to 6 carbon atoms), a 2,3,6,7-tetrahydrobenzo[1,2-b:4,5b']difuran-2-yl radical, a 4-oxo-4H-chromen-2-yl radical (optionally substituted in the benzene ring by one or more halogen atoms, by straight-chain or branched alkyl radicals having from 1 to 6 carbon atoms or by alkoxy radicals having from 1 to 6 carbon atoms), a benzocyclobutenyl radical of formula A or an indanyl radical of formula B :

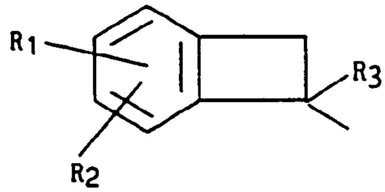

(A)

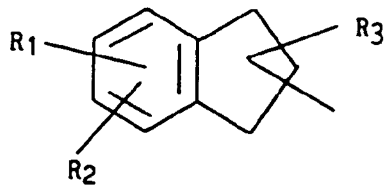

(B)

(wherein:

$R_1$ and $R_2$, which are the same or different, each represents a hydrogen atom, a halogen atom, a trifluoromethyl radical, a straight-chain or branched alkyl radical having from 1 to 6 carbon atoms, an alkoxy radical having from 1 to 6 carbon atoms, a hydroxy radical, a hydroxyalkyl radical having from 1 to 6 carbon atoms or an alkylthio radical having from 1 to 6 carbon atoms, or together form a methylenedioxy radical or an ethylenedioxy radical, and

$R_3$ represents a hydrogen atom or a straight-chain or branched alkyl radical having from 1 to 6 carbon atoms),

or a radical of formula C :

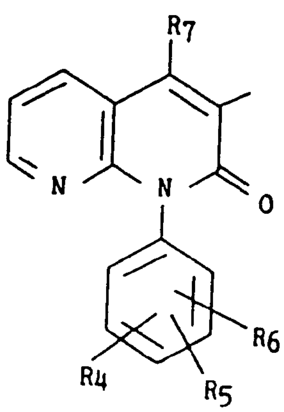

(C)

(wherein

$R_4$, $R_5$ and $R_6$, which are the same or different, each represents a hydrogen atom, a halogen atom, a trifluoromethyl radical, a hydroxy radical, a straight-chain or branched alkyl radical having from 1 to 6 carbon atoms, an alkoxy radical having from 1 to 6 carbon atoms, or an alkylthio radical having from 1 to 6 carbon atoms, and

$R_7$ represents a hydrogen atom or a hydroxy radical),

the optical isomers thereof and the addition salts thereof with a pharmaceutically acceptable organic or mineral acid,

characterised in that :

either

A) a compound of formula II :

$$R - (CH_2)_m W \qquad (II)$$

wherein R and m are as defined above and W represents a halogen atom or a tosyloxy radical or a mesyloxy radical, is reacted

a) with a compound of formula III :

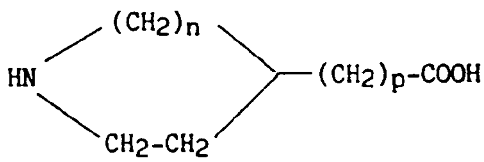

(III)

wherein n and p are as defined above, in the presence of N,N-diethylethylenediamine in dimethylformamide or another equivalent organic solvent,

to form a compound of formula IV :

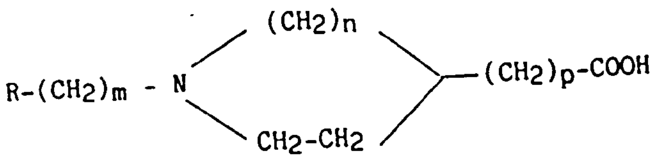

(IV)

wherein R, m, n and p are as defined above,

which is subjected to the action of thionyl chloride or oxalyl chloride to form an acid chloride of formula V:

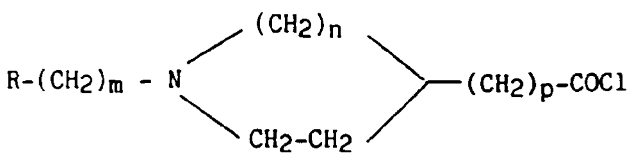

(V)

wherein the meanings of R, m, n and p remain identical to those given above,

which is condensed in the presence of aluminium chloride with a compound of formula VI :

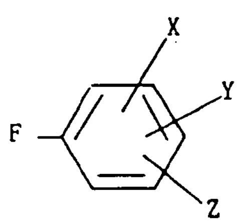

(VI)

wherein X, Y and Z are as defined above,

to form a compound of formula VII :

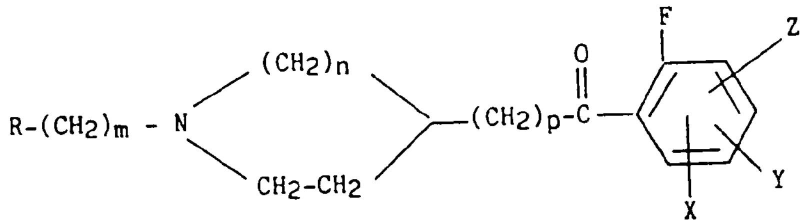

(VII)

wherein R, X, Y, Z, m, n and p are as defined above,

which is subjected to the action of hydroxylamine hydrochloride in the presence of 2-diethylaminoethylamine in alcoholic medium,

to obtain a compound of formula VIII :

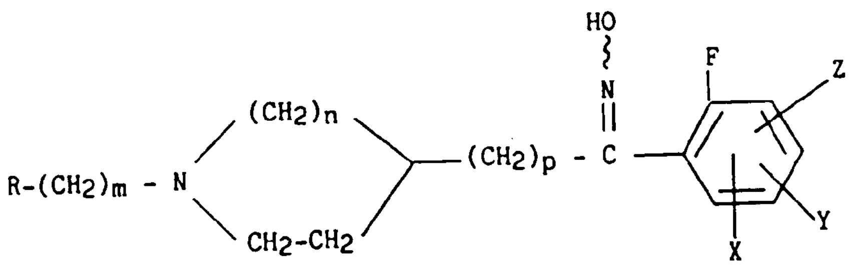

(VIII)

wherein R, X, Y, Z, m, n and p are as defined above,

which is cyclised with the aid of a strong mineral base to obtain the compounds of formula I,

b) or with a compound of formula IX :

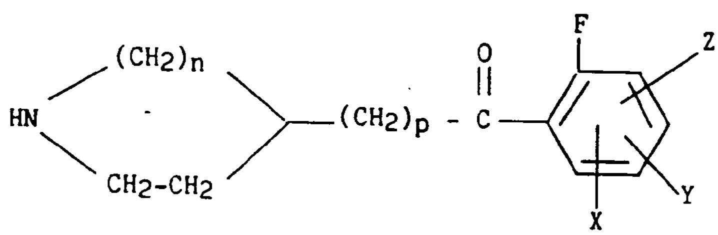

(IX)

wherein X, Y, Z, n and p are as defined above, in the presence of N,N-diethylethylenediamine in dimethylformamide or another equivalent organic solvent,

to form the compounds of formula VII, from which the compounds of formula VIII and then the compounds of formula I are obtained using the process indicated above,

c) or with a compound of formula X :

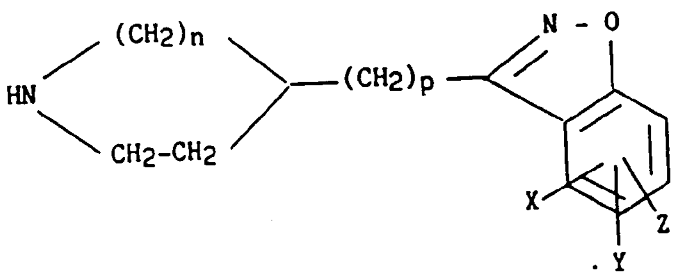

(X)

wherein X, Y, Z, n and p are as defined above, in the presence of N,N-diethylethylenediamine in dimethylformamide or another equivalent organic solvent,

to form the compounds of formula I,

or

B) a compound of formula $XI_A$ or $XI_B$ :

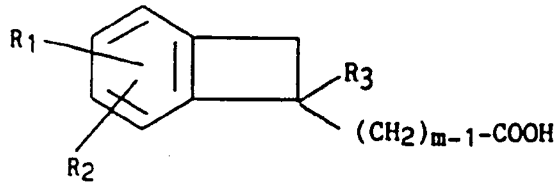

($XI_A$)

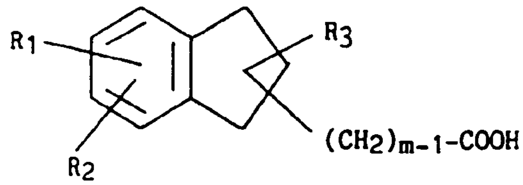

($XI_B$)

wherein $R_1$, $R_2$, $R_3$ and m are as defined for formula I, except that in this case m cannot represent the number 0, is reacted with a compound of formula X in the presence of carbonyldiimidazole to form a compound of formula $XII_A$ or $XII_B$ :

($XII_A$)

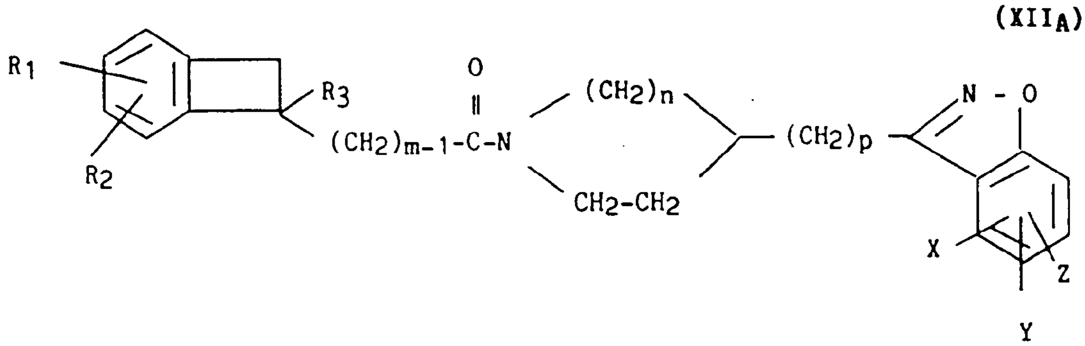

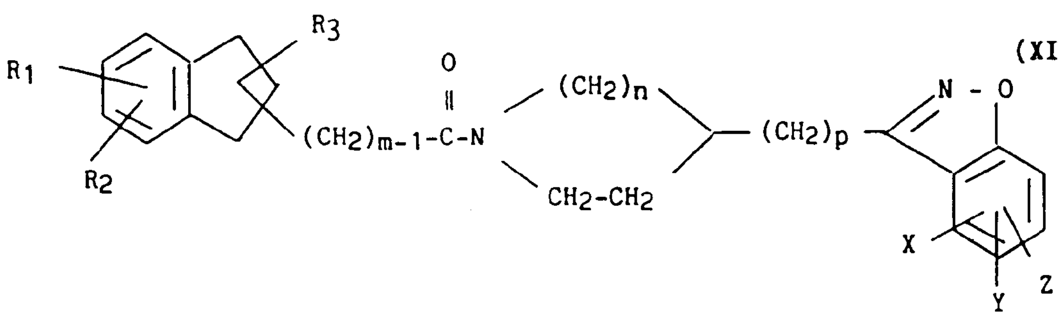

($XII_B$)

wherein $R_1$, $R_2$, $R_3$, m, n, p, X, Y and Z are as defined for formula I except that in this case m does not represent the number 0,
which is subjected to the action of lithium aluminium hydride to form the compounds of formula I

or

C) a compound of formula XIII :

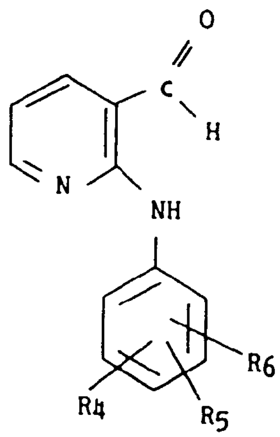

(XIII)

wherein $R_4$, $R_5$ and $R_5$ are as defined above, is reacted with a compound of formula XIV :

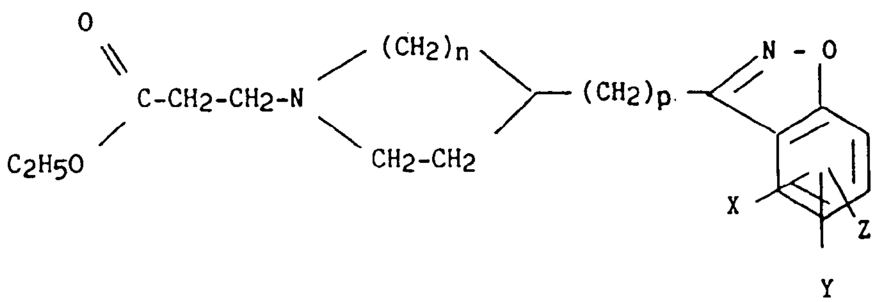

(XIV)

wherein the meanings of n, p, X, Y and Z remain identical to those given for formula I defined above, to form the compounds of formula I wherein R represents a radical of formula C and m is 1,

which compounds of formula I

may be converted into salts with a pharmaceutically acceptable mineral or organic acid to form the corresponding salts or

may be separated into their optical isomers and then converted into salts.

**Claim for the following Contracting State : ES**

**1.** A process for the preparation of compounds of formula I:

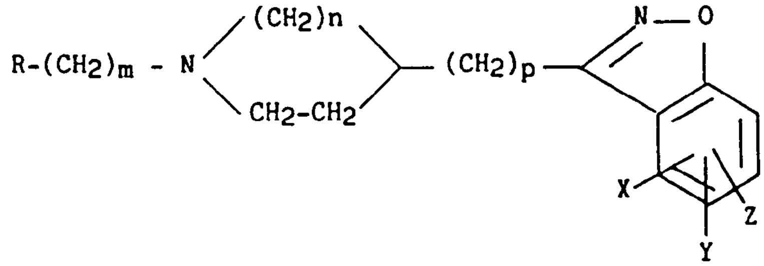

(I)

wherein :

- m represents an integer of from 0 to 5,
- n represents an integer 1 or 2,
- p is equal to 0, 1 or 2,
- X, Y and Z, which are the same or different, each represents a hydrogen atom, a halogen atom, a straight-chain or branched alkyl radical having from 1 to 6 carbon atoms, a trifluoromethyl radical, an alkoxy radical having from 1 to 6 carbon atoms, an alkylthio radical having from 1 to 6 carbon atoms or a hydroxy radical,
- R represents a benzofuran-2-yl or 2,3-dihydrobenzofuran-2-yl radical (each of which may be substituted in the benzene ring by one or more halogen atoms, by alkoxy radicals having from 1 to 6 carbon atoms, by alkylthio radicals having from 1 to 6 carbon atoms, or by straight-chain or branched alkyl radicals having from 1 to 6 carbon atoms), a 2,3,6,7-tetrahydrobenzo[1,2-b:4,5b′]difuran-2-yl radical, a 4-oxo-4H-chromen-2-yl radical (optionally substituted in the benzene ring by one or more halogen atoms, by straight-chain or branched alkyl radicals having from 1 to 6 carbon atoms or by alkoxy radicals having from 1 to 6 carbon atoms), a benzocyclobutenyl radical of formula A or an indanyl radical of formula B :

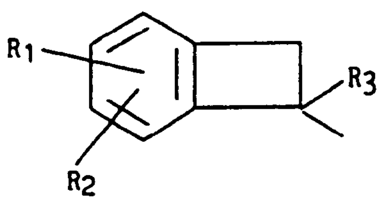

(A)

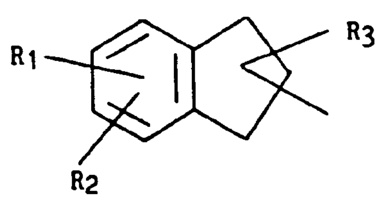

(B)

(wherein:

$R_1$ and $R_2$, which are the same or different, each represents a hydrogen atom, a halogen atom, a trifluoromethyl radical, a straight-chain or branched alkyl radical having from 1 to 6 carbon atoms, an alkoxy radical having from 1 to 6 carbon atoms, a hydroxy radical, a hydroxyalkyl radical having from 1 to 6 carbon atoms or an alkylthio radical having from 1 to 6 carbon atoms, or together form a methylenedioxy radical or an ethylenedioxy radical, and

$R_3$ represents a hydrogen atom or a straight-chain or branched alkyl radical having from 1 to 6 carbon atoms),

or a radical of formula C :

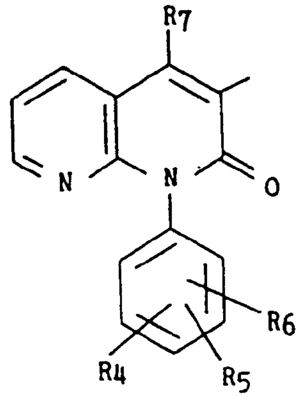

(C)

(wherein

$R_4$, $R_5$ and $R_6$, which are the same or different, each represents a hydrogen atom, a halogen atom, a trifluoromethyl radical, a hydroxy radical, a straight-chain or branched alkyl radical having from 1 to 6 carbon atoms, an alkoxy radical having from 1 to 6 carbon atoms, or an alkylthio radical having from 1 to 6 carbon atoms, and

$R_7$ represents a hydrogen atom or a hydroxy radical),

the optical isomers thereof and the addition salts thereof with a pharmaceutically acceptable organic or mineral acid,

characterised in that :

either

A) a compound of formula II :

$$R - (CH_2)_m - W \qquad (II)$$

wherein R and m are as defined above and W represents a halogen atom or a tosyloxy radical or a mesyloxy radical, is reacted

a) with a compound of formula III :

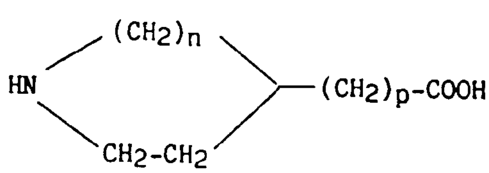

(III)

wherein n and p are as defined above, in the presence of N,N-diethylethylenediamine in dimethylformamide or another equivalent organic solvent,

to form a compound of formula IV :

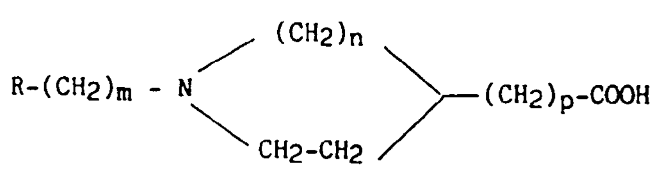

(IV)

wherein R, m, n and p are as defined above,

which is subjected to the action of thionyl chloride or oxalyl chloride to form an acid chloride of formula V:

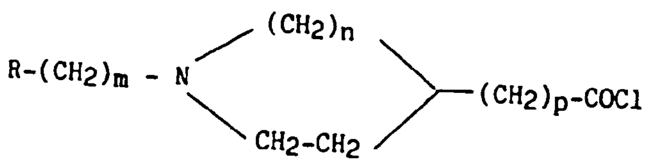

(V)

wherein the meanings of R, m, n and p remain identical to those given above,

which is condensed in the presence of aluminium chloride with a compound of formula VI :

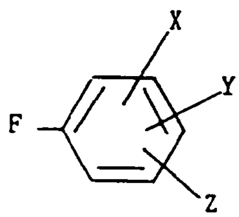

(VI)

wherein X, Y and Z are as defined above,

to form a compound of formula VII :

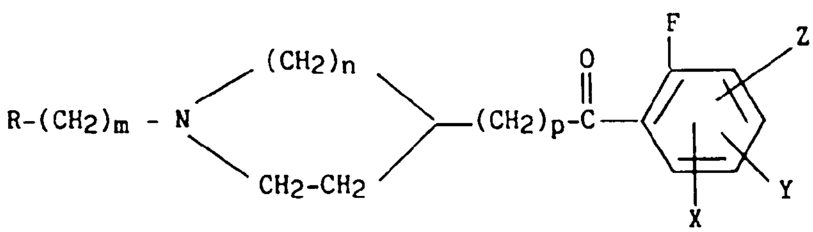

(VII)

wherein R, X, Y, Z, m, n and p are as defined above,

which is subjected to the action of hydroxylamine hydrochloride in the presence of 2-diethylaminoethylamine in alcoholic medium,

to obtain a compound of formula VIII :

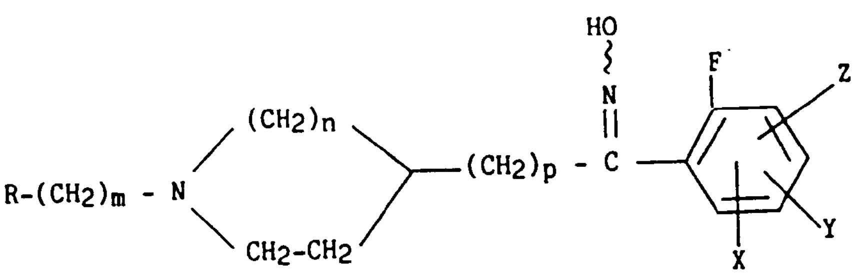

(VIII)

wherein R, X, Y, Z, m, n and p are as defined above,

which is cyclised with the aid of a strong mineral base to obtain the compounds of formula I,

b) or with a compound of formula IX :

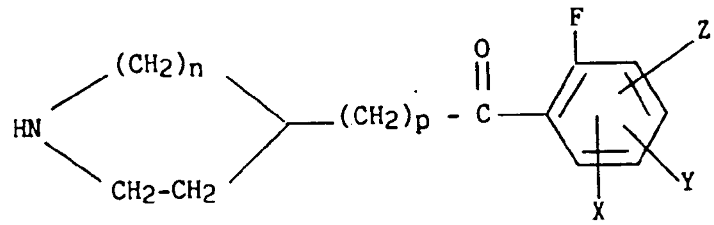

(IX)

wherein X, Y, Z, n and p are as defined above, in the presence of N,N-diethylethylenediamine in dimethylformamide or another equivalent organic solvent,

to form the compounds of formula VII, from which the compounds of formula VIII and then the compounds of formula I are obtained using the process indicated above,

c) or with a compound of formula X :

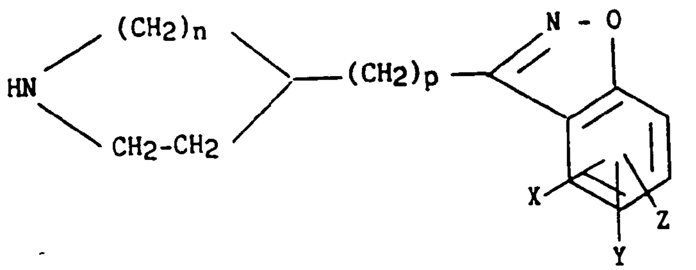

(X)

wherein X, Y, Z, n and p are as defined above, in the presence of N,N-diethylethylenediamine in dimethylformamide or another equivalent organic solvent,

to form the compounds of formula I,

or

B) a compound of formula $XI_A$ or $XI_B$ :

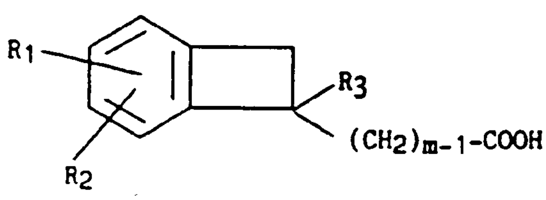

**($XI_A$)**

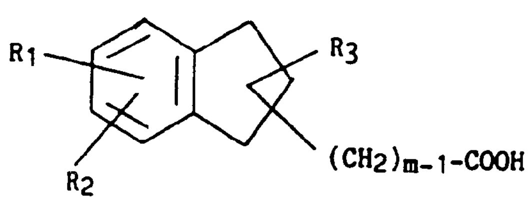

**($XI_B$)**

wherein $R_1$, $R_2$, $R_3$ and m are as defined for formula I, except that in this case m cannot represent the number 0, is reacted with a compound of formula X in the presence of carbonyldiimidazole to form a compound of formula $XII_A$ or $XII_B$ :

(XIIA)

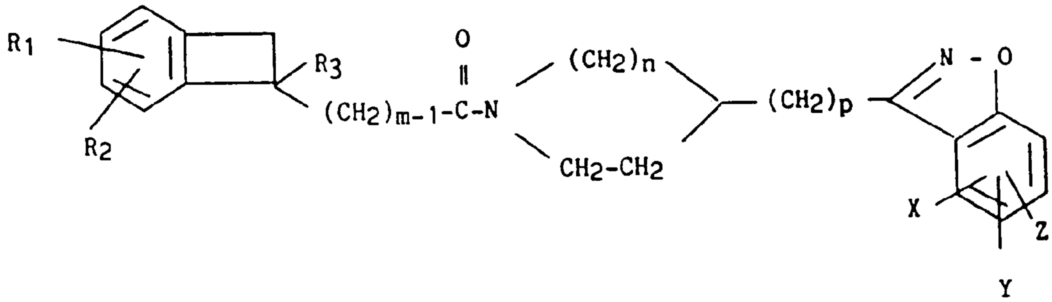

(XIIB)

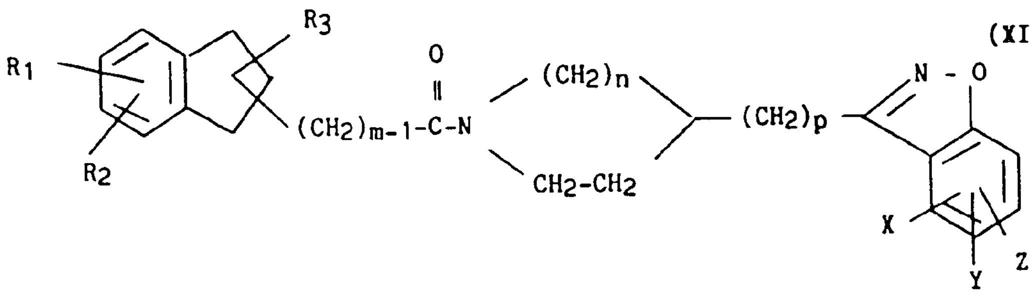

wherein $R_1$, $R_2$, $R_3$, m, n, p, X, Y and Z are as defined for formula I except that in this case m does not represent the number 0,
which is subjected to the action of lithium aluminium hydride to form the compounds of formula I

or

C) a compound of formula XIII :

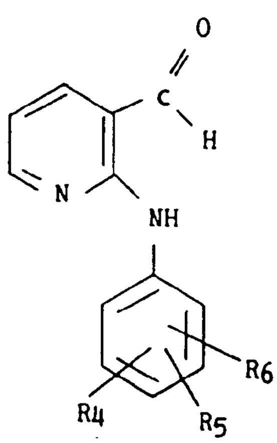

(XIII)

wherein $R_4$, $R_5$ and $R_5$ are as defined above, is reacted with a compound of formula XIV :

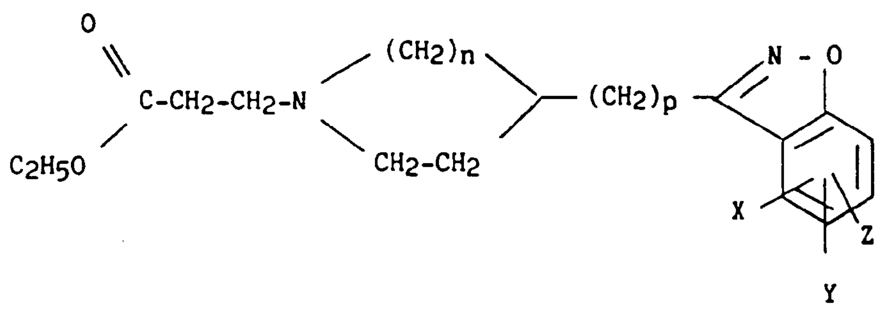

(XIV)

wherein the meanings of n, p, X, Y and Z remain identical to those given for formula I defined above, to form the compounds of formula I wherein R represents a radical of formula C and m is 1,

which compounds of formula I

may be converted into salts with a pharmaceutically acceptable mineral or organic acid to form the corresponding salts or

may be separated into their optical isomers and then converted into salts.